(19) Europäisches Patentamt
European Patent Office
Office européen des brevets

(11) **EP 2 649 454 B1**

(12) **EUROPEAN PATENT SPECIFICATION**

(45) Date of publication and mention
of the grant of the patent:
**20.06.2018 Bulletin 2018/25**

(51) Int Cl.:
***G01N 33/68*** [(2006.01)]

(21) Application number: **11799153.9**

(22) Date of filing: **06.12.2011**

(86) International application number:
**PCT/EP2011/071978**

(87) International publication number:
**WO 2012/076553 (14.06.2012 Gazette 2012/24)**

(54) **BIOMARKERS AND PARAMETERS FOR HYPERTENSIVE DISORDERS OF PREGNANCY**

BIOMARKER UND PARAMETER FÜR BLUTHOCHDRUCKLEIDEN WÄHREND DER SCHWANGERSCHAFT

BIOMARQUEURS ET PARAMÈTRES DES TROUBLES D'HYPERTENSION DE LA GROSSESSE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **06.12.2010 EP 10193834**
**06.12.2010 US 420171 P**

(43) Date of publication of application:
**16.10.2013 Bulletin 2013/42**

(73) Proprietor: **Mycartis N.V.**
**9052 Gent (BE)**

(72) Inventors:
• **TUYTTEN, Robin**
**Glanmire**
**Co Cork (IE)**
• **THOMAS, Gregoire**
**B-9160 Lokeren (BE)**
• **MOERMAN, Piet**
**B-9831 Deurle (BE)**

(74) Representative: **Cabinet Laurent & Charras**
**Le Contemporain**
**50 Chemin de la Bruyère**
**69574 Dardilly Cedex (FR)**

(56) References cited:
**WO-A2-2011/128357    KR-A- 20100 088 217**

• **JISOOK PARK ET AL: "Discovery of the serum biomarker proteins in severe preeclampsia by proteomic analysis", EXPERIMENTAL AND MOLECULAR MEDICINE, vol. 43, no. 7, 1 January 2011 (2011-01-01), page 427, XP55019907, ISSN: 1226-3613, DOI: 10.3858/emm.2011.43.7.047**
• **LEWITT ET AL: "Developmental regulation of circulating insulin-like growth factor-binding proteins in normal pregnancies and in pre-eclampsia.", PROGRESS IN GROWTH FACTOR RESEARCH, vol. 6, no. 2-4, 1 January 1995 (1995-01-01), pages 475-80, XP55001957, ISSN: 0955-2235**
• **KAAJA R: "Predictors and risk factors of pre-eclampsia", MINERVA GINECOLOGICA, TORINO, IT, vol. 60, no. 5, 1 October 2008 (2008-10-01), pages 421-429, XP009156780, ISSN: 0026-4784**
• **QIN LIU ET AL: "Pre-eclampsia is associated with the failure of melanoma cell adhesion molecule (MCAM/CD146) expression by intermediate trophoblast", LABORATORY INVESTIGATION, vol. 84, no. 2, 1 February 2004 (2004-02-01), pages 221-228, XP055049281, ISSN: 0023-6837, DOI: 10.1038/labinvest.3700033**
• **RAYMAN ET AL: "Selenoproteins and human health: Insights from epidemiological data", BIOCHIMICA ET BIOPHYSICA ACTA - GENERAL SUBJECTS, ELSEVIER SCIENCE PUBLISHERS, NL, vol. 1790, no. 11, 1 November 2009 (2009-11-01), pages 1533-1540, XP026688184, ISSN: 0304-4165, DOI: 10.1016/J.BBAGEN.2009.03.014 [retrieved on 2009-03-25]**

**(Cont. next page)**

EP 2 649 454 B1

• **Robert E. Gerszten ET AL: "The search for new cardiovascular biomarkers", Nature, vol. 451, no. 7181, 21 February 2008 (2008-02-21), pages 949-952, XP055033236, ISSN: 0028-0836, DOI: 10.1038/nature06802**

Remarks:
   The file contains technical information submitted after the application was filed and not included in this specification

## Description

### FIELD OF THE INVENTION

The invention relates to biomarkers and parameters useful for the diagnosis, prediction, prognosis and/or monitoring of diseases and conditions in subjects, in particular hypertensive disorders of pregnancy, more in particular preeclampsia; and to related methods, uses, kits and devices.

### BACKGROUND OF THE INVENTION

In many diseases and conditions, a favourable outcome of prophylactic and/or therapeutic treatments is strongly correlated with early and/or accurate prediction, diagnosis, prognosis and/or monitoring of a disease or condition. Therefore, there exists a continuous need for additional and preferably improved manners for early and/or accurate prediction, diagnosis, prognosis and/or monitoring of diseases and conditions to guide the treatment choices.

Hypertensive disorders occurring during pregnancy represent a major cause of maternal morbidity and mortality worldwide, and are also associated with increased perinatal mortality.

A prominent place among hypertensive disorders of pregnancy belongs to preeclampsia (PE), which develops in about 5% to 10% of pregnant females (Solomon & Seely 2006, Endocrinol Metab Clin North Am 35(1): 157-71, vii).

PE may be described as new onset hypertension and proteinuria past 20 weeks gestation in a previously normotensive pregnant female, which may be mild or severe. Patients with mild disease display blood pressures > 140/90 and proteinuria with >300mg protein noted on a 24 hour urine sample after 20 weeks gestation, and usually deliver near term without significant co-morbidities. However, about 25% of PE tends to be severe, involving symptoms and signs of central nervous system dysfunction, hepatocellular injury, reduced urine output and markedly elevated blood pressure (systolic >160 mmHg or diastolic >110 mmHg). Severe PE typically occurs in late 2nd and early 3rd trimester and is associated with increased maternal and perinatal morbidity and mortality.

Severe complications of PE include 1) HELLP syndrome characterised by haemolysis, elevated liver enzymes and low platelets, and 2) eclampsia characterised by the development of seizures. Whereas both these conditions are rare, they are associated with poor prognosis (Solomon & Seely 2006, supra).

Preeclampsia is also associated with foetal complications such as intrauterine growth retardation (IUGR) and small for gestational age (SGA).

The only cure for PE is delivery of the baby and placenta. Beyond 37 weeks of gestation, delivery is warranted. At gestational ages of less than 34 weeks, treatment of hypertension and close foetal surveillance may prevent cerebral vascular accidents and prolong the pregnancy, without curing the underlying disease process. Delivery is also warranted for development of severe PE or eclampsia (Sibai & Barton 2007, Am J Obstet Gynecol 196(6):514.e1-9).

The aetiology and pathophysiology of PE remains largely unresolved and its diagnosis is currently based entirely on clinical criteria once the disease unfolds (Roberts et al. 2003, Hypertension 41(3): 37-45). However, recent data suggests that events leading to PE may begin and progress insidiously as early as 1st trimester.

Dependable and early prediction and/or diagnosis is therefore crucial for successful treatment interventions in hypertensive disorders of pregnancy including inter alia PE. Consequently, provision of further, alternative and preferably improved methods and means for diagnosis, prediction, prognosis and/or monitoring of hypertensive disorders of pregnancy continues to be of prime importance.

However, clinically useful screening tests to predict the development of PE are sparse (Conde-Agudelo et al. 2004, Obstet Gynecol 104: 1367-91). Reliance on risk factors is also substandard, since (although several risk factors for PE have been identified) over 50% of cases occur among otherwise young, low risk, nulliparous females. Hence, hypertensive disorders of pregnancy and particularly PE remain largely unpredictable in their onset and disease progression.

Kaajar R. reports that family history of PE has been described as a known risk factor of PE (Kaajar R., Minerva Ginecologica, 60(5): 475-480, 2008).

Lewitt et al. 1998 (Journal of Endocrinology 159: 265) mentioned that the insulin-like growth factor (IGF) system is believed to be important in pregnancy and implicated in the pathophysiology of pre-eclampsia.

Mistry et al. 2008 (Hypertension 52: 881) reported reduced selenium concentrations and glutathione peroxidase activity in preeclamptic pregnancies.

Rayman et al. 2003 (Am J Obstet Gynecol 189: 1343) observed that median toenail selenium concentrations in preeclamptic subjects were significantly lower than in their matched controls.

WO 2009/094665 to PerkinElmer Health Sciences Inc. concerns methods for determining the risk of pre-eclampsia in a pregnant individual using a test panel comprising the level of placental growth factor (PlGF) and the level of pregnancy-associated plasma protein A (PAPP-A) in a blood sample from a subject and the measurement of blood pressure in the subject.

**SUMMARY OF THE INVENTION**

Having conducted extensive experiments and tests, the inventors identified panels comprising biomarker(s) and clinical parameter(s), said panels being closely predictive and/or indicative of hypertensive disorders of pregnancy (henceforth "HDP"), more specifically preeclampsia (henceforth "PE").

As herein disclosed, additional and markedly improved methods and means for diagnosis, prediction, prognosis and/or monitoring of HDP and particularly PE are realised through provision of a test panel comprising:

- measurement of the level of insulin-like growth factor-binding protein complex acid labile subunit (IGFALS),

- a score for the maternal history parameter 'mother or sister with previous PE and/or father with ischemic heart disease' (henceforth "*fh_petxcardio*"), and

- measurement of blood pressure.

As shall be understood the parameter *fh_petxcardio* as disclosed herein includes within it information on the maternal history parameter 'mother or sister of subject has/had preeclampsia', i.e., 'mother or sister with previous PE' (also denoted herein "*fh_pet*") and/or information on the maternal history parameter 'father of subject has/had ischemic heart disease', i.e., 'father with ischemic heart disease' (also denoted herein "*father_any_ihd*"). Preferably, the parameter *father_any_ihd* may encompass or refer to the fact that father of woman has had a heart attack, coronary heart disease, coronary bypass, angioplasty or angina (see, e.g., BMJ 2011, vol. 342, d1875, supplementary info).

Accordingly, a positive score for the parameter *fh_petxcardio* may mean an underlying positive score for the parameter *fh_pet,* or may mean an underlying positive score for the parameter *father_any_ihd,* or may mean an underlying positive score for both parameters *fh_pet* and *father_any_ihd.*

The inventors have realised that satisfactory and even more accurate evaluation of HDP and particularly PE may be achieved when only the maternal history parameter *father_any_ihd* is scored instead of scoring the parameter *fh_petxcardio.* Hence, it is herein disclosed a test panel comprising:

- measurement of the level of insulin-like growth factor-binding protein complex acid labile subunit (IGFALS),

- a score for the maternal history parameter 'father with ischemic heart disease' (*father_any_ihd*), and

- measurement of blood pressure.

The disclosed test panel can provide even more dependable and early prediction and/or diagnosis of HDP or PE when further comprising at least one, more preferably at least two or even at least three of (i.e., $\geq 1$, $\geq 2$ or $\geq 3$) biomarkers and/or parameters selected from the group consisting of): measurement of the level of selenoprotein P (SEPP1), measurement of the level of s-Endoglin (ENG), measurement of the level of quiescin Q6 (QSOX1), measurement of the level of peroxiredoxin-2 (PRDX2), measurement of blood glucose level, measurement of body mass index (BMI), a score for the maternal history parameter 'father of subject has/had ischemic heart disease' ("*father_any_ihd*"), a score for the maternal history parameter 'mother or sister of subject has/had preeclampsia' ("*fh_pet*"), measurement of the level of vascular endothelial growth factor receptor 3 (FLT4), measurement of the level of lysosomal Pro-X carboxypeptidase (PRCP), measurement of the level of peroxiredoxin-1 (PRDX1), measurement of the level of leucyl-cystinyl aminopeptidase (LNPEP, OTASE), measurement of the level of tenascin-X (TNXB), measurement of the level of basement membrane-specific heparan sulfate proteoglycan core protein (HSPG2), measurement of the level of cell surface glycoprotein (CD146, MUC18, MCAM), measurement of the level of phosphatidylinositol-glycan-specific phospholipase D (GPLD1), measurement of the level of collagen alpha-3(VI) chain (COL6A3), measurement of the level of Kunitz-type protease inhibitor 1 (SPINT1), measurement of the level of hepatocyte growth factor-like protein (MST1), measurement of the level of probable G-protein coupled receptor 126 (GPR126), measurement of the level of intercellular adhesion molecule 3 (ICAM3), and measurement of the level of C-reactive protein (CRP); particularly preferably selected from the group consisting of measurement of the level of SEPP1, measurement of the level of ENG, measurement of the level of QSOX1, measurement of the level of PRDX2, and measurement of blood glucose level. The measurement of the level of ENG may be excluded.

The disclosed test panel can provide even more dependable and early prediction and/or diagnosis of HDP or PE when, in addition to the measurement of the level of IGFALS, the score for *fh_petxcardio* or preferably the score of *father_any_ihd,* and measurement of blood pressure, they further comprise at least one, more preferably at least two or even at least three of (i.e., $\geq 1$, $\geq 2$ or $\geq 3$) biomarkers and/or parameters selected from the group consisting of): measurement of the level of SEPP1, measurement of the level of s-Endoglin (ENG or s-ENG), measurement of the level of quiescin Q6 (QSOX1), measurement of the level of PRDX2, measurement of blood glucose level, measurement of BMI, a score for *father_any_ihd,* a score for *fh_pet,* a value for the parameter *bb_hdl* parameter (i.e., the high density lipoprotein level; for example, in the experimental section this parameter may denote HDL level as obtained from the subject and stored in the SCOPE biobank), a value for the parameter *bb_total_hdl_ratio* (i.e., the ratio of total cholesterol to high density lipoprotein; for example, in the experimental section this parameter may denote the ratio of total cholesterol to HDL as obtained from the subject and stored in the SCOPE biobank), a score for the parameter *metabolic syndrome* {the condition metabolic syndrome is known *per se* (see, e.g., Alberti et al. Diabetic Medicine, 2006, vol. 23, 469-480) and any subject diagnosed as having metabolic syndrome according to art-established definitions and methods would be scored as, e.g., "1" or "yes" or "positive" for the parameter metabolic syndrome as intended herein; preferably, a subject can be qualified as being metabolic syndrome positive (e.g., score = "1" or "yes" or "positive") when she fulfilled at least 2 of the following 4 conditions: 1) BMI >=30, 2) *bb_trig* >1.7 (mmol/L)(the parameter *"bb_trig"* denotes the triglycerides level, for example, in the experimental section this parameter may denote the triglycerides level as obtained from the subject and stored in the SCOPE biobank), 3) *bb_hdl* <1.29 (mmol/L) and 4) 1st_vst_sbp_2nd > 130 (mm Hg) or 1st_vst_dbp_2nd > 85 (mm Hg)}, a value for the parameter 'triglycerides level' ("*bb_trig*"), measurement of the level of FLT4, measurement of the level of PRCP, measurement of the level of PRDX1, measurement of the level of LNPEP, measurement of the level of TNXB, measurement of the level of HSPG2, measurement of the level of MUC18, measurement of the level of GPLD1, measurement of the level of COL6A3, measurement of the level of SPINT1, measurement of the level of MST1, measurement of the level of GPR126, measurement of the level of ICAM3, measurement of the level of CRP, measurement of the level of disintegrin and metalloproteinase domain-containing protein 12 (ADAM12), measurement of the level of phosphatidylcholine-sterol acyltransferase (LCAT), measurement of the level of roundabout homolog 4 (ROBO4), measurement of the level of ectonucleotide pyrophosphatase/phosphodiesterase family member 2 (ENPP2), and measurement of the level of protein S100-A9 (S100A9).

The disclosed test panel can provide even more dependable and early prediction and/or diagnosis of HDP or PE when, in addition to the measurement of the level of IGFALS, the score of *father_any_ihd,* and measurement of blood pressure, they further comprise at least one, more preferably at least two or even at least three of (i.e., ≥1, ≥2 or ≥3) biomarkers and/or parameters selected from the group consisting of): measurement of the level of SEPP1, measurement of the level of s-Endoglin (ENG or s-ENG), measurement of BMI, a score for *fh_pet,* a value for *bb_hdl,* a value for *bb_total_hdl_ratio,* a score for *metabolic syndrome,* measurement of the level of HSPG2, measurement of the level of MUC18, measurement of the level of SPINT1, measurement of the level of ADAM12, measurement of the level of LCAT, measurement of the level of ROBO4, measurement of the level of ENPP2, and measurement of the level of S100A9.

For the sake of conciseness, the phrase "measurement of the level of [a biomarker]" may be used herein synonymously with phrases such as "measurement of [a biomarker] level" or simply "[a biomarker] level".

Furthermore, in any one panel disclosed throughout this specification, the measurement of the level of selenoprotein P (SEPP1) may be supplemented or substituted by the measurement of the level of selenium. Hence, for any one panel specified herein as comprising the measurement of the level of selenoprotein P (SEPP1), the present specification also discloses an otherwise identical panel comprising the measurement of the level of selenium instead of the measurement of the level of SEPP1, as well as another otherwise identical panel comprising the measurement of the level of selenium in addition to the measurement of the level of SEPP1. The measurement of the level of at least or only SEPP1 may, however, be preferred.

A particularly preferred test panel as herein disclosed (henceforth "panel A") comprises measurement of IGFALS level, a score for *fh_petxcardio,* and measurement of blood pressure; and:

- further comprises any one or more of, particularly any one, any two or any three of, measurement of the level of SEPP1, measurement of the level of ENG, measurement of the level of QSOX1, measurement of the level of PRDX2, and measurement of blood glucose level; or
- further comprises any one or more of, particularly any one, any two or any three of, measurement of the level of SEPP1, measurement of the level of QSOX1, measurement of the level of PRDX2, and measurement of blood glucose level.

Preferably, panel A may comprise a score for *father_any_ihd* instead of the score for *fh_petxcardio.*

Another particularly preferred test panel as herein disclosed comprises (henceforth "panel B") measurement of IGFALS level, a score for *fh_petxcardio,* measurement of blood pressure, and measurement of the level of SEPP1; and:

- optionally and preferably further comprises any one or more of, particularly any one, any two or any three of, measurement of the level of ENG, measurement of the level of QSOX1, measurement of the level of PRDX2, and measurement of blood glucose level; or

- optionally and preferably further comprises any one or more of, particularly any one, any two or all three of, measurement of the level of QSOX1, measurement of the level of PRDX2, and measurement of blood glucose level.

Preferably, panel B may comprise a score for *father_any_ihd* instead of the score for *fh_petxcardio.*

A further particularly preferred test panel as herein disclosed (henceforth "panel C") comprises measurement of IGFALS level, a score for *fh_petxcardio,* measurement of blood pressure, and measurement of blood glucose level; and:

- optionally and preferably further comprises any one or both of measurement of the level of SEPP1, and measurement of the level of ENG; or

- optionally and preferably further comprises measurement of the level of SEPP1.

Preferably, panel C may comprise a score for *father_any_ihd* instead of the score for *fh_petxcardio.*

A yet further particularly preferred test panel as herein disclosed (henceforth "panel D") comprises measurement of IGFALS level, a score for *fh_petxcardio,* measurement of blood pressure, and measurement of the level of QSOX1; and:

- optionally and preferably further comprises any one or more of, particularly any one, any two or all three of, measurement of the level of ENG, measurement of the level of SEPP1, and measurement of the level of PRDX2; or

- optionally and preferably further comprises any one or both of measurement of the level of SEPP1, and measurement of the level of PRDX2; or

- optionally and preferably further comprises measurement of the level of ENG.

Preferably, panel D may comprise a score for *father_any_ihd* instead of the score for *fh_petxcardio.*

The disclosed test panel have been realised which display unexpectedly advantageous, even synergistic degree of specificity and selectivity in diagnosis, prediction, prognosis and/or monitoring of HDP and particularly PE.

Thus, preferably, a panel as herein disclosed (henceforth "panel E") comprises or consists of measurement of IGFALS level, a score for *fh_petxcardio,* measurement of blood pressure, measurement of blood glucose level, and measurement of SEPP1 level. Preferably, panel E may comprise a score for *father_any_ihd* instead of the score for *fh_petxcardio.*

The panel E may advantageously also comprise measurement of ENG level. Hence, preferably a panel (henceforth "panel F") comprises or consists of measurement of IGFALS level, a score for *fh_petxcardio,* measurement of blood pressure, measurement of blood glucose level, measurement of SEPP1 level, and measurement of ENG level. Preferably, panel F may comprise a score for *father_any_ihd* instead of the score for *fh_petxcardio.*

Preferably, a panel as herein disclosed (henceforth "panel G") comprises or consists of measurement of IGFALS level, a score for *fh_petxcardio,* measurement of blood pressure, measurement of SEPP1 level, measurement of PRDX2 level, and measurement of QSOX1 level. Preferably, panel G may comprise a score for *father_any_ihd* instead of the score for *fh_petxcardio.*

Preferably, a panel as herein disclosed (henceforth "panel H") comprises or consists of measurement of IGFALS level, a score for *fh_petxcardio,* measurement of blood pressure, measurement of ENG level, and measurement of QSOX1 level. Preferably, panel H may comprise a score for *father_any_ihd* instead of the score for *fh_petxcardio.*

The disclosed panels may display their diagnostic, predictive, prognostic and/or monitoring value for HDP or PE substantially throughout pregnancy and/or postpartum, or when evaluated within one or more sections of pregnancy (e.g., within 1 st, 2nd and/or 3rd trimesters) or postpartum, or only when evaluated within one or more comparably short periods (e.g., about 10, 8, 6, 4 or 2 weeks) within pregnancy or postpartum. All such panels are useful and suitable herein.

The disclosed panels can particularly advantageously allow the prediction of a subsequent / later incidence of HDP or PE in a subject which is considered healthy at the time of testing, i.e., in a subject not having clinically manifest (active) HDP or PE at the time of testing. Advantageously, the present panels can thus be particularly evaluated in subjects between about 10 and about 24 weeks of gestation, preferably between about 13 and about 22 weeks of gestation, more preferably between 14 and 21 weeks of gestation, more preferably between 15 and 20 weeks of gestation; such as between about 12 and about 18 weeks (i.e., 15 +/- about 3 weeks), preferably between about 13 and about 17 weeks (i.e., 15 +/- about 2 weeks), preferably between about 14 and about 16 weeks (i.e., 15 +/- about 1 week) or more preferably at about 15 weeks of gestation; or such as between about 17 and about 23 weeks (i.e., 20 +/- about 3 weeks), preferably between about 18 and about 22 weeks (i.e., 20 +/- about 2 weeks), preferably between about 19 and about 21 weeks (i.e., 20 +/- about 1 week) or more preferably at about 20 weeks of gestation (with reference to human female gestation). Such prediction may preferably indicate a probability, chance or risk that a tested subject will develop clinically manifest HDP or PE, optionally also allowing to predict onset within a certain time period or onset at a given age of gestation or postpartum, such as, for example, early onset preeclampsia (i.e., clinical manifestation <34 weeks of gestation) vs. preterm PE (i.e., clinical manifestation <37 weeks of gestation) vs. term PE (i.e., clinical manifestation ≥37 weeks of gestation).

Particularly preferably, the disclosed panels can allow the prediction of a subsequent / later incidence of HDP or PE in a subject tested at between about 17 and about 23 weeks (i.e., 20 +/- about 3 weeks), preferably between about 18 and about 22 weeks (i.e., 20 +/about 2 weeks), preferably between about 19 and about 21 weeks (i.e., 20 +/- about 1 week) or more preferably at about 20 weeks of gestation (with reference to human female gestation).

Preferably, a test panel to be evaluated at 15 +/- about 3 weeks or preferably 15 +/- about 2 weeks or preferably 15 +/- about 1 week or more preferably about 15 weeks of gestation may - in addition to measurement of IGFALS level, a score for *fh_petxcardio* and measurement of blood pressure - further comprise at least one, more preferably at least two or even at least three of PRDX2 level, QSOX1 level, SEPP1 level, ENG level, FLT4 level, TNXB level, HSPG2 level, LNPEP level, MST1 level, GPR126 level, ICAM3 level, CRP level, measurement of BMI, a score for the maternal history parameter *father_any_ihd,* and a score for the maternal history parameter *fh_pet*; particularly preferably selected from the group consisting of PRDX2 level, QSOX1 level, SEPP1 level and ENG level (henceforth "panel I"). The measurement of the level of ENG may be excluded. Preferably, panels as detailed in this paragraph, such as particularly panel I, may comprise a score for *father_any_ihd* instead of the score for *fh_petxcardio.*

Preferably, very dependable and early prediction and/or diagnosis of HDP or PE at 15 +/about 3 weeks, preferably 15 +/- about 2 weeks or preferably 15 +/- about 1 week or more preferably about 15 weeks of gestation can be obtained using any one of the panels D, G or H as set forth above, more preferably wherein said panels D, G or H comprise a score for *father_any_ihd* instead of the score for *fh_petxcardio.*

Preferably, a test panel to be evaluated at 20 +/- about 3 weeks, preferably 20 +/- about 2 weeks or preferably 20 +/- about 1 week or more preferably about 20 weeks of gestation may - in addition to measurement of IGFALS level, a score for *fh_petxcardio* and measurement of blood pressure - further comprise at least one, more preferably at least two or even at least three of ENG level, SEPP1 level, FLT4 level, PRCP level, PRDX1 level, LNPEP level (LNPEP level may be particularly useful in albeit not limited to panels excluding ENG level), TNXB level, HSPG2 level, MUC18 / MCAM level, GPLD1 level, COL6A3 level and SPINT1 level, measurement of blood glucose level, measurement of BMI, a score for the maternal history parameter *father_any_ihd*" and a score for the maternal history parameter *fh_pet*;particularly preferably selected from the group consisting of measurement of blood glucose level and measurement of the level of any one of SEPP1 and ENG (henceforth "panel J"). The measurement of the level of ENG may be excluded. Preferably, panels as detailed in this paragraph, such as particularly panel J, may comprise a score for *father_any_ihd* instead of the score for *fh_petxcardio.*

Preferably, very dependable and early prediction and/or diagnosis of HDP or PE at 20 +/about 3 weeks, preferably 20 +/- about 2 weeks or preferably 20 +/- about 1 week or more preferably about 20 weeks of gestation can be obtained using any one of the panels C, E or F as set forth above, more preferably wherein said panels C, E or F comprise a score for *father_any_ihd* instead of the score for *fh_petxcardio.*

It is also herein disclosed test panels, suitable particularly but without limitation for prediction of HDP or preferably PE, preferably at 20 +/- about 3 weeks of gestation (preferably at 20 +/- about 2 weeks or more preferably at 20 +/- about 1 week or more preferably at about 20 weeks of gestation), said panels comprising measurement of IGFALS level, a score for *father_any_ihd* and measurement of blood pressure, and further optionally and preferably comprising at least one, more preferably at least two or even more preferably at least three markers and/or parameters selected from the group consisting of: SEPP1 level, ENG level, measurement of BMI, a value for *bb_hdl,* a value for *bb_total_hdl_ratio,* a score for *metabolic syndrome,* HSPG2 level, MUC18 level, SPINT1 level, ADAM12 level, LCAT level, ROBO4 level, ENPP2 level and S100A9 level. Preferred ≥4-member panels of this type may comprise or consist of measurement of IGFALS level, a score for *father_any_ihd* and measurement of blood pressure, and one marker or parameter selected from the group consisting of ENG level, a value for *bb_hdl,* a score for *metabolic syndrome,* MUC18 level and ADAM12 level; particularly preferably selected from the group consisting of ENG level, a value for *bb_hdl,* a score for *metabolic syndrome* and ADAM12 level. Preferred ≥5-member panels of this type may comprise or consist of measurement of IGFALS level, a score for *father_any_ihd* and measurement of blood pressure, and two markers and/or parameters selected from the group consisting of SEPP1 level, ENG level, measurement of BMI, a value for *bb_hdl,* a value for *bb_total_hdl_ratio,* a score for *metabolic syndrome,* MUC18 level, SPINT1 level and ADAM12 level; particularly preferably wherein at least one and more preferably at least two of said markers and/or parameters are selected from ENG level, a value for *bb_hdl,* a score for *metabolic syndrome,* MUC18 level and ADAM12 level. Preferred ≥6-member panels of this type may comprise or consist of measurement of IGFALS level, a score for *father_any_ihd* and measurement of blood pressure, and three markers and/or parameters selected from the group consisting of SEPP1 level, ENG level, measurement of BMI, a value for *bb_hdl,* a value for *bb_total_hdl_ratio,* a score for *metabolic syndrome,* HSPG2 level, MUC18 level, SPINT1 level, ADAM12 level, LCAT level, ROBO4 level, ENPP2 level and S100A9 level; particularly preferably wherein at least one and more preferably at least two and even more preferably at least three of said markers and/or parameters are selected from ENG level, measurement of BMI, a value for *bb_hdl,* a score for *metabolic syndrome,* MUC18 level and ADAM12 level. Particularly preferred panels of this type may comprise or consist of markers and parameters as included in any one of the exemplary panels T4.1 to T4.4, as demonstrated in Example 5.

It is also herein disclosed test panels, suitable particularly but without limitation for prediction of HDP or preferably PE using a "rule-in" test, preferably at 20 +/- about 3 weeks of gestation (preferably at 20 +/- about 2 weeks or more preferably at 20 +/- about 1 week or more preferably at about 20 weeks of gestation), said panels comprising measurement of IGFALS level, a score for *father_any_ihd* and measurement of blood pressure, and further optionally and preferably comprising at least one, more preferably at least two or even more preferably at least three markers and/or parameters selected from the group consisting of: SEPP1 level, ENG level, measurement of BMI, a value for *bb_hdl,* a score for *metabolic syndrome,* HSPG2 level, MUC18 level, SPINT1 level, ADAM12 level, ROBO4 level and ENPP2 level. Preferred ≥4-member panels of this type may comprise or consist of measurement of IGFALS level, a score for *father_any_ihd* and measurement of blood pressure, and one marker or parameter selected from the group consisting of ENG level and ADAM12 level. Preferred ≥5-member panels of this type may comprise or consist of measurement of IGFALS level, a score for *father_any_ihd* and measurement of blood pressure, and two markers and/or parameters selected from the group consisting of SEPP1 level, ENG level, measurement of BMI, a score for *metabolic syndrome,* MUC18 level and ADAM12 level. Preferred ≥6-member panels of this type may comprise or consist of measurement of IGFALS level, a score for *father_any_ihd* and measurement of blood pressure, and three markers and/or parameters selected from the group consisting of SEPP1 level, ENG level, measurement of BMI, a value for *bb_hdl,* a score for *metabolic syndrome,* HSPG2 level, MUC18 level, SPINT1 level, ADAM12 level, ROBO4 level and ENPP2 level; particularly preferably wherein at least one and more preferably at least two and even more preferably at least three of said markers and/or parameters are selected from ENG level, measurement of BMI, a score for *metabolic syndrome,* MUC18 level and ADAM12 level. Particularly preferred panels of this type may comprise or consist of markers and parameters as included in any one of the exemplary panels T5.1 to T5.9, as demonstrated in Example 5.

It is also herein disclosed test panels, suitable particularly but without limitation for prediction of HDP or preferably pre-term PE, preferably at 20 +/- about 3 weeks of gestation (preferably at 20 +/- about 2 weeks or more preferably at 20 +/- about 1 week or more preferably at about 20 weeks of gestation), said panels comprising measurement of IGFALS level, a score for *father_any_ihd* and measurement of blood pressure, and further optionally and preferably comprising at least one, more preferably at least two or even more preferably at least three markers and/or parameters selected from the group consisting of: SEPP1 level, ENG level, a value for *bb_total_hdl_ratio,* a score for *metabolic syndrome,* MUC18 level, SPINT1 level, ADAM12 level and ROBO4 level. Preferred ≥5-member panels of this type may comprise or consist of measurement of IGFALS level, a score for *father_any_ihd* and measurement of blood pressure, and two markers and/or parameters selected from the group consisting of SEPP1 level, ENG level, a value for *bb_total_hdl_ratio,* a score for *metabolic syndrome,* MUC18 level, SPINT1 level and ADAM12 level; particularly preferably wherein at least one and more preferably at least two of said markers and/or parameters are selected from ENG level, a score for *metabolic syndrome,* SPINT1 level and ADAM12 level. Preferred ≥6-member panels of this type may comprise or consist of measurement of IGFALS level, a score for *father_any_ihd* and measurement of blood pressure, and three markers and/or parameters selected from the group consisting of SEPP1 level, ENG level, a score for *metabolic syndrome,* MUC18 level, SPINT1 level, ADAM12 level and ROBO4 level; particularly preferably wherein at least one and more preferably at least two and even more preferably at least three of said markers and/or parameters are selected from ENG level, a score for *metabolic syndrome,* SPINT1 level and ADAM12 level. Particularly preferred panels of this type may comprise or consist of markers and parameters as included in any one of the exemplary panels T6.1 to T6.2, as demonstrated in Example 5.

It is also herein disclosed test panels, suitable particularly but without limitation for prediction of HDP or preferably pre-term PE using a "rule-in" test, preferably at 20 +/about 3 weeks of gestation (preferably at 20 +/- about 2 weeks or more preferably at 20 +/- about 1 week or more preferably at about 20 weeks of gestation), said panels comprising measurement of IGFALS level, a score for *father_any_ihd* and measurement of blood pressure, and further optionally and preferably comprising at least one, more preferably at least two or even more preferably at least three markers and/or parameters selected from the group consisting of: SEPP1 level, ENG level, a score for *metabolic syndrome,* MUC18 level, SPINT1 level, ADAM12 level and ROBO4 level. Preferred ≥5-member panels of this type may comprise or consist of measurement of IGFALS level, a score for *father_any_ihd* and measurement of blood pressure, and two markers and/or parameters selected from the group consisting of ENG level, a score for *metabolic syndrome* and ADAM12 level. Preferred ≥6-member panels of this type may comprise or consist of measurement of IGFALS level, a score for *father_any_ihd* and measurement of blood pressure, and three markers and/or parameters selected from the group consisting of SEPP1 level, ENG level, a score for *metabolic syndrome,* MUC18 level, SPINT1 level, ADAM12 level and ROBO4 level; particularly preferably wherein at least one and more preferably at least two and even more preferably at least three of said markers and/or parameters are selected from ENG level, a score for *metabolic syndrome,* SPINT1 level and ADAM12 level. Particularly preferred panels of this type may comprise or consist of markers and parameters as included in any one of the exemplary panels T7.1 to T7.4, as demonstrated in Example 5.

It is also herein disclosed test panels, suitable particularly but without limitation for prediction of HDP or preferably PE in non-obese subjects, preferably at 20 +/- about 3 weeks of gestation (preferably at 20 +/- about 2 weeks or more preferably at 20 +/- about 1 week or more preferably at about 20 weeks of gestation), said panels comprising measurement of IGFALS level, a score for *father_any_ihd* and measurement of blood pressure, and further optionally and preferably comprising at least one, more preferably at least two or even more preferably at least three markers and/or parameters selected from the group consisting of: SEPP1 level, ENG level, a score for *metabolic syndrome,* HSPG2 level, MUC18 level, ADAM12 level and ROBO4 level. Preferred ≥4-member panels of this type may comprise or consist of measurement of IGFALS level, a score for *father_any_ihd* and measurement of blood pressure, and one marker or parameter selected from the group consisting of ENG level, ADAM12 level and ROBO4 level; particularly preferably selected from the group consisting of ENG level and ADAM12 level. Preferred ≥5-member panels of this type may comprise or consist of measurement of IGFALS level, a score for *father_any_ihd* and measurement of blood pressure, and two markers and/or parameters selected from the group consisting of SEPP1 level, ENG level, a score for *metabolic syndrome,* MUC18 level, ADAM12 level and ROBO4 level; particularly preferably wherein at least one and more preferably at least two of said markers and/or parameters are selected from SEPP1 level, ENG level, MUC18 level, ADAM12 level and ROBO4 level. Preferred ≥6-member panels of this type may comprise or consist of measurement of IGFALS level, a score for *father_any_ihd* and measurement of blood pressure, and three markers and/or parameters selected from the group consisting of SEPP1 level, ENG level, a score for *metabolic syndrome,* HSPG2 level, MUC18 level, ADAM12 level and ROBO4 level; particularly preferably wherein at least one and more preferably at least two and even more preferably at least three of said markers and/or parameters are selected from SEPP1 level, ENG level, MUC18 level, ADAM12 level and ROBO4 level. Particularly preferred panels of this type may comprise or consist of markers and parameters as included in any one of the exemplary panels T8.1 to T8.4, as demonstrated in Example 5.

It is also herein disclosed test panels, suitable particularly but without limitation for prediction of HDP or preferably PE in non-obese subjects using a "rule-in" test, preferably at 20 +/- about 3 weeks of gestation (preferably at 20 +/- about 2 weeks or more preferably at 20 +/- about 1 week or more preferably at about 20 weeks of gestation), said panels comprising measurement of IGFALS level, a score for *father_any_ihd* and measurement of blood pressure, and further optionally and preferably comprising at least one, more preferably at least two or even more preferably at least three markers and/or parameters selected from the group consisting of: SEPP1 level, ENG level, a score for *metabolic syndrome,* HSPG2 level, MUC18 level, ADAM12 level and ROBO4 level. Preferred ≥4-member panels of this type may comprise or consist of measurement of IGFALS level, a score for *father_any_ihd* and measurement of blood pressure, and one marker or parameter selected from the group consisting of ENG level and ADAM12 level. Preferred ≥5-member panels of this type may comprise or consist of measurement of IGFALS level, a score for *father_any_ihd* and measurement of blood pressure, and two markers and/or parameters selected from the group consisting of SEPP1 level, ENG level, a score for *metabolic syndrome,* MUC18 level, ADAM12 level and ROBO4 level; particularly preferably wherein at least one and more preferably at least two of said markers and/or parameters are selected from SEPP1 level, ENG level, MUC18 level, ADAM12 level and ROBO4 level. Preferred ≥6-member panels of this type may comprise or consist of measurement of IGFALS level, a score for *father_any_ihd* and measurement of blood pressure, and three markers and/or parameters selected from the group consisting of SEPP1 level, ENG level, a score for *metabolic syndrome,* HSPG2 level, MUC18 level, ADAM12 level and ROBO4 level; particularly preferably wherein at least one and more preferably at least two and even more preferably at least three of said markers and/or parameters are selected from SEPP1 level, ENG level, MUC18 level, ADAM12 level and ROBO4 level. Particularly preferred panels of this type may comprise or consist of markers and parameters as included in any one of the exemplary panels T9.1 to T9.10, as demonstrated in Example 5.

It is also herein disclosed test panels, suitable particularly but without limitation for prediction of HDP or preferably pre-term PE in non-obese subjects, preferably at 20 +/about 3 weeks of gestation (preferably at 20 +/- about 2 weeks or more preferably at 20 +/- about 1 week or more preferably at about 20 weeks of gestation), said panels comprising measurement of IGFALS level, a score for *father_any_ihd* and measurement of blood pressure, and further optionally and preferably comprising at least one, more preferably at least two or even more preferably at least three markers and/or parameters selected from the group consisting of: SEPP1 level, ENG level, a score for *metabolic syndrome,* MUC18 level, ADAM12 level and ROBO4 level. Preferred ≥4-member panels of this type may comprise or consist of measurement of IGFALS level, a score for *father_any_ihd* and measurement of blood pressure, and one marker or parameter selected from the group consisting of ENG level and ADAM12 level. Preferred ≥5-member panels of this type may comprise or consist of measurement of IGFALS level, a score for *father_any_ihd* and measurement of blood pressure, and two markers and/or parameters selected from the group consisting of SEPP1 level, ENG level, a score for *metabolic syndrome,* MUC18 level, ADAM12 level and ROBO4 level; particularly preferably wherein at least one and more preferably at least two of said markers and/or parameters are selected from ENG level, MUC18 level, ADAM12 level and ROBO4 level. Preferred ≥6-member panels of this type may comprise or consist of measurement of IGFALS level, a score for *father_any_ihd* and measurement of blood pressure, and three markers and/or parameters selected from the group consisting of SEPP1 level, ENG level, a score for *metabolic syndrome,* MUC18 level, ADAM12 level and ROBO4 level; particularly preferably wherein at least one and more preferably at least two and even more preferably at least three of said markers and/or parameters are selected from ENG level, a score for *metabolic syndrome,* MUC18 level, ADAM12 level and ROBO4 level. Particularly preferred panels of this type may comprise or consist of markers and parameters as included in any one of the exemplary panels T10.1 to T10.4, as demonstrated in Example 5.

It is also herein disclosed test panels, suitable particularly but without limitation for prediction of HDP or preferably pre-term PE in non-obese subjects using a "rule-in" test, preferably at 20 +/- about 3 weeks of gestation (preferably at 20 +/- about 2 weeks or more preferably at 20 +/- about 1 week or more preferably at about 20 weeks of gestation), said panels comprising measurement of IGFALS level, a score for *father_any_ihd* and measurement of blood pressure, and further optionally and preferably comprising at least one, more preferably at least two or even more preferably at least three markers and/or parameters selected from the group consisting of: SEPP1 level, ENG level, a score for *metabolic syndrome,* MUC18 level, ADAM12 level and ROBO4 level. Preferred ≥4-member panels of this type may comprise or consist of measurement of IGFALS level, a score for *father_any_ihd* and measurement of blood pressure, and one marker or parameter selected from the group consisting of ENG level and ADAM12 level. Preferred ≥5-member panels of this type may comprise or consist of measurement of IGFALS level, a score for *father_any_ihd* and measurement of blood pressure, and two markers and/or parameters selected from the group consisting of SEPP1 level, ENG level, a score for *metabolic syndrome,* MUC18 level, ADAM12 level and ROBO4 level; particularly preferably wherein at least one and more preferably at least two of said markers and/or parameters are selected from ENG level, MUC18 level, ADAM12 level and ROBO4 level. Preferred ≥6-member panels of this type may comprise or consist of measurement of IGFALS level, a score for *father_any_ihd* and measurement of blood pressure, and three markers and/or parameters selected from the group consisting of SEPP1 level, ENG level, a score for *metabolic syndrome,* MUC18 level, ADAM12 level and ROBO4 level; particularly preferably wherein at least one and more preferably at least two and even more preferably at least three of said markers and/or parameters are selected from ENG level, a score for *metabolic syndrome,* MUC18 level, ADAM12 level and ROBO4 level. Particularly preferred panels of this type may comprise or consist of markers and parameters as included in any one of the exemplary panels T11.1 to T11.7, as demonstrated in Example 5.

Having conducted additional experiments and tests, the inventors also realised that panels not necessarily comprising the score for either the parameter *fh_petxcardio* or the score for the parameter *father_any_ihd* may also be closely predictive and/or indicative of HDP, more specifically PE.

The invention pertains to a method for the prediction of preeclampsia (PE) in a subject comprising testing and evaluating in a sample from said subject a test panel, wherein the sample is whole blood, plasma or serum, the test panel comprising:

- measurement of the level of insulin-like growth factor-binding protein complex acid labile subunit (IGFALS),
- measurement of blood pressure,
- measurement of the level of Kunitz-type protease inhibitor 1 (SPINT1), and
- measurement of the level of either i) disintegrin and metalloproteinase domain containing protein 12 (ADAM12) or ii) s-Endoglin (ENG or s-ENG),

wherein the measurements of the biomarkers and parameter of the test panel is used to establish a biomarker-and-parameter profile, which is compared with a corresponding multi-parameter reference value for prediction of PE.

Preferably, in the method of the invention, the test panel comprises measurement of the level of s-Endoglin and measurement of the level of ADAM12.

Advantageously, in the method of the invention, the test panel further comprises at least one, more preferably at least two or even at least three of measurement of the level of selenoprotein P (SEPP1), measurement of the level of quiescin Q6 (QSOX1), measurement of the level of peroxiredoxin-2 (PRDX2), measurement of blood glucose level, measurement of body mass index (BMI), a score for the maternal history parameter 'mother or sister of subject has/had preeclampsia' ("fh pet"), a value for the parameter 'high density lipoprotein level' ("bb hdr"), a value for the parameter 'ratio of total cholesterol to high density lipoprotein' ("bb total hdt ratio"), a score for the parameter metabolic syndrome, a value for the parameter triglycerides level ("bb trip"), measurement of the level of vascular endothelial growth factor receptor 3 (FLT4), measurement of the level of lysosomal Pro-X carboxypeptidase (PRCP), measurement of the level of peroxiredoxin-1 (PRDX1), measurement of the level of leucyl-cystinyl aminopeptidase (LNPEP), measurement of the level of tenascin-X (TNXB), measurement of the level of basement membrane-specific heparan sulfate proteoglycan core protein (HSPG2), measurement of the level of cell surface glycoprotein (CD146, MUC18, MCAM), measurement of the level of phosphatidylinositol-glycan-specific phospholipase D (GPLD1), measurement of the level of collagen alpha-3(VI) chain (COL6A3), measurement of the level of hepatocyte growth factor-like protein (MST1), measurement of the level of probable G-protein coupled receptor 126 (GPR126), measurement of the level of intercellular adhesion molecule 3 (ICAM3), measurement of the level of C-reactive protein (CRP), measurement of the level of phosphatidylcholine-sterol acyltransferase (LCAT), measurement of the level of roundabout homolog 4 (ROBO4), measurement of the level of ectonucleotide pyrophosphatase/phosphodiesterase family member 2 (ENPP2), and measurement of the level of protein S100-A9 (S100A9). Preferably, the measurement of the level of SEPP1 is supplemented or substituted by the measurement of the level of selenium.

It is also herein disclosed additional and markedly improved methods and means for diagnosis, prediction, prognosis and/or monitoring (preferably prediction) of HDP and particularly PE are realised through provision of a test panel comprising:

- measurement of the level of insulin-like growth factor-binding protein complex acid labile subunit (IGFALS), and

- measurement of blood pressure.

Preferably, in the disclosed methods and means, the test panel may comprise:

- measurement of the level of insulin-like growth factor-binding protein complex acid labile subunit (IGFALS),

- measurement of blood pressure, and

at least one, more preferably at least two or even at least three of (i.e., $\geq 1$, $\geq 2$ or $\geq 3$) biomarkers and/or parameters selected from the group consisting of): measurement of the level of SEPP1, measurement of the level of s-Endoglin, measurement of the level of QSOX1, measurement of the level of PRDX2, measurement of blood glucose level, measurement of BMI, a score for *fh_pet,* a value for *bb_hdl,* a value for *bb_total_hdl_ratio,* a score for *metabolic syndrome,* a value for *bb_trig* (the parameter *"bb_trig"* denotes the triglycerides level, for example, in the experimental section this parameter may denote the triglycerides level as obtained from the subject and stored in the SCOPE biobank), measurement of the level of FLT4, measurement of the level of PRCP, measurement of the level of PRDX1, measurement of the level of LNPEP, measurement of the level of TNXB, measurement of the level of HSPG2, measurement of the level of MUC18, measurement of the level of GPLD1, measurement of the level of COL6A3, measurement of the level of SPINT1, measurement of the level of MST1, measurement of the level of GPR126, measurement of the level of ICAM3, measurement of the level of CRP, measurement of the level of ADAM12, measurement of the level of LCAT, measurement of the level of ROBO4, measurement of the level of ENPP2, and measurement of the level of S100A9.
Preferably, in the disclosed methods and means, the test panel may comprise:

- measurement of the level of insulin-like growth factor-binding protein complex acid labile subunit (IGFALS),

- measurement of blood pressure, and

- at least one, more preferably at least two or even at least three of (i.e., $\geq 1$, $\geq 2$ or $\geq 3$) biomarkers and/or parameters selected from the group consisting of): measurement of the level of SEPP1, measurement of the level of ENG, measurement of BMI, a score for *father_any_ihd,* a score for *fh_pet,* a value for *bb_hdl,* a value for *bb_total_hdl_ratio,* a score for *metabolic syndrome,* a value for *bb_trig,* measurement of the level of HSPG2, measurement of the level of MUC18, measurement of the level of SPINT1, measurement of the level of ADAM12, measurement of the level of LCAT, measurement of the level of ROBO4, measurement of the level of ENPP2 and measurement of the level of S100A9 level.

It is also herein disclosed test panels, suitable particularly but without limitation for prediction of HDP or preferably PE, preferably at 20 +/- about 3 weeks of gestation (preferably at 20 +/- about 2 weeks or more preferably at 20 +/- about 1 week or more preferably at about 20 weeks of gestation), said panels comprising measurement of IGFALS level and measurement of blood pressure, and further optionally and preferably comprising at least one, more preferably at least two, even more preferably at least three, and still more preferably at least four markers and/or parameters selected from the group consisting of those markers and parameters listed in Table 12 of Example 6 for which the number set forth in at least one column of the columns no.2, 3, 4 and 5 of Table 12 is not 0 (i.e., the marker or parameter is present in at least one panel considered successful). Preferred $\geq 3$-member panels of this type may comprise or consist of measurement of IGFALS level and measurement of blood pressure, and one marker or parameter selected from the group consisting of those markers and parameters listed in Table 12 for which the number set forth in column no. 2 of Table 12 is not 0 (i.e., the marker or parameter is present in at least one 3-member panel considered successful); particularly preferably selected from the group consisting of those markers and parameters listed in Table 12 for which the product of dividing the number set forth in column no. 2 of Table 12 by the number in the field "number of panels" in column no. 2 of Table 12 is 0.20 (20%) or greater (i.e., the marker or parameter is present in at least 20% of 3-member panels considered successful). Preferred $\geq 4$-member panels of this type may comprise or consist of measurement of IGFALS level and measurement of blood pressure, and two markers and/or parameters selected from the group consisting of those markers and parameters listed in Table 12 for which the number set forth in column no. 3 of Table 12 is not 0 (i.e., the marker or parameter is present in at least one 4-member panel considered successful); particularly preferably wherein at least one and more preferably at least two of said markers and/or parameters are selected from the group consisting of those markers and parameters listed in Table 12 for which the product of dividing the number set forth in column no. 3 of Table 12 by the number in the field "number of panels" in column no. 3 of Table 12 is 0.20 (20%) or greater (i.e., the marker or parameter is present in at least 20% of 4-member panels considered successful). Preferred ≥5-member panels of this type may comprise or consist of measurement of IGFALS level and measurement of blood pressure, and three markers and/or parameters selected from the group consisting of those markers and parameters listed in Table 12 for which the number set forth in column no. 4 of Table 12 is not 0 (i.e., the marker or parameter is present in at least one 5-member panel considered successful); particularly preferably wherein at least one and preferably at least two and more preferably at least three of said markers and/or parameters are selected from the group consisting of those markers and parameters listed in Table 12 for which the product of dividing the number set forth in column no. 4 of Table 12 by the number in the field "number of panels" in column no. 4 of Table 12 is 0.20 (20%) or greater (i.e., the marker or parameter is present in at least 20% of 5-member panels considered successful). Preferred ≥6-member panels of this type may comprise or consist of measurement of IGFALS level and measurement of blood pressure, and four markers and/or parameters selected from the group consisting of those markers and parameters listed in Table 12 for which the number set forth in column no. 5 of Table 12 is not 0 (i.e., the marker or parameter is present in at least one 6-member panel considered successful); particularly preferably wherein at least one and preferably at least two and more preferably at least three and even more preferably at least four of said markers and/or parameters are selected from the group consisting of those markers and parameters listed in Table 12 for which the product of dividing the number set forth in column no. 5 of Table 12 by the number in the field "number of panels" in column no. 5 of Table 12 is 0.20 (20%) or greater (i.e., the marker or parameter is present in at least 20% of 6-member panels considered successful). Particularly preferred panels of this type may comprise or consist of markers and parameters as included in any one of the exemplary panels T12.1 to T12.4, as demonstrated in Example 6.

It is also herein disclosed test panels, suitable particularly but without limitation for prediction of HDP or preferably PE using a "rule-in" test, preferably at 20 +/- about 3 weeks of gestation (preferably at 20 +/- about 2 weeks or more preferably at 20 +/- about 1 week or more preferably at about 20 weeks of gestation), said panels comprising measurement of IGFALS level and measurement of blood pressure, and further optionally and preferably comprising at least one, more preferably at least two, even more preferably at least three, and still more preferably at least four markers and/or parameters selected from the group consisting of those markers and parameters listed in Table 13 of Example 6 for which the number set forth in at least one column of the columns no.2, 3, 4 and 5 of Table 13 is not 0 (i.e., the marker or parameter is present in at least one panel considered successful). Preferred ≥4-member panels of this type may comprise or consist of measurement of IGFALS level and measurement of blood pressure, and two markers and/or parameters selected from the group consisting of those markers and parameters listed in Table 13 for which the number set forth in column no. 3 of Table 13 is not 0 (i.e., the marker or parameter is present in at least one 4-member panel considered successful); particularly preferably wherein at least one and more preferably at least two of said markers and/or parameters are selected from the group consisting of those markers and parameters listed in Table 13 for which the product of dividing the number set forth in column no. 3 of Table 13 by the number in the field "number of panels" in column no. 3 of Table 13 is 0.20 (20%) or greater (i.e., the marker or parameter is present in at least 20% of 4-member panels considered successful). Preferred ≥5-member panels of this type may comprise or consist of measurement of IGFALS level and measurement of blood pressure, and three markers and/or parameters selected from the group consisting of those markers and parameters listed in Table 13 for which the number set forth in column no. 4 of Table 13 is not 0 (i.e., the marker or parameter is present in at least one 5-member panel considered successful); particularly preferably wherein at least one and preferably at least two and more preferably at least three of said markers and/or parameters are selected from the group consisting of those markers and parameters listed in Table 13 for which the product of dividing the number set forth in column no. 4 of Table 13 by the number in the field "number of panels" in column no. 4 of Table 13 is 0.20 (20%) or greater (i.e., the marker or parameter is present in at least 20% of 5-member panels considered successful). Preferred ≥6-member panels of this type may comprise or consist of measurement of IGFALS level and measurement of blood pressure, and four markers and/or parameters selected from the group consisting of those markers and parameters listed in Table 13 for which the number set forth in column no. 5 of Table 13 is not 0 (i.e., the marker or parameter is present in at least one 6-member panel considered successful); particularly preferably wherein at least one and preferably at least two and more preferably at least three and even more preferably at least four of said markers and/or parameters are selected from the group consisting of those markers and parameters listed in Table 13 for which the product of dividing the number set forth in column no. 5 of Table 13 by the number in the field "number of panels" in column no. 5 of Table 13 is 0.20 (20%) or greater (i.e., the marker or parameter is present in at least 20% of 6-member panels considered successful). Particularly preferred panels of this type may comprise or consist of markers and parameters as included in any one of the exemplary panels T13.1 to T13.8, as demonstrated in Example 6.

It is also herein disclosed test panels, suitable particularly but without limitation for prediction of HDP or preferably pre-term PE, preferably at 20 +/- about 3 weeks of gestation (preferably at 20 +/- about 2 weeks or more preferably at 20 +/- about 1 week or more preferably at about 20 weeks of gestation), said panels comprising measurement of IGFALS level and measurement of blood pressure, and further optionally and preferably comprising at least one, more preferably at least two, even more preferably at least three, and still more preferably at least four markers and/or parameters selected from the group consisting of those markers and parameters listed in Table 14 of Example 6 for which the number set forth in at least one column of the columns no.2, 3, 4 and 5 of Table 14 is not 0 (i.e., the marker or parameter is present in at least one panel considered successful). Preferred ≥4-member panels of this type may comprise or consist of measurement of IGFALS level and measurement of blood pressure, and two markers and/or parameters selected from the group consisting of those markers and parameters listed in Table 14 for which the number set forth in column no. 3 of Table 14 is not 0 (i.e., the marker or parameter is present in at least one 4-member panel considered successful); particularly preferably wherein at least one and more preferably at least two of said markers and/or parameters are selected from the group consisting of those markers and parameters listed in Table 14 for which the product of dividing the number set forth in column no. 3 of Table 14 by the number in the field "number of panels" in column no. 3 of Table 14 is 0.20 (20%) or greater (i.e., the marker or parameter is present in at least 20% of 4-member panels considered successful). Preferred ≥5-member panels of this type may comprise or consist of measurement of IGFALS level and measurement of blood pressure, and three markers and/or parameters selected from the group consisting of those markers and parameters listed in Table 14 for which the number set forth in column no. 4 of Table 14 is not 0 (i.e., the marker or parameter is present in at least one 5-member panel considered successful); particularly preferably wherein at least one and preferably at least two and more preferably at least three of said markers and/or parameters are selected from the group consisting of those markers and parameters listed in Table 14 for which the product of dividing the number set forth in column no. 4 of Table 14 by the number in the field "number of panels" in column no. 4 of Table 14 is 0.20 (20%) or greater (i.e., the marker or parameter is present in at least 20% of 5-member panels considered successful). Preferred ≥6-member panels of this type may comprise or consist of measurement of IGFALS level and measurement of blood pressure, and four markers and/or parameters selected from the group consisting of those markers and parameters listed in Table 14 for which the number set forth in column no. 5 of Table 14 is not 0 (i.e., the marker or parameter is present in at least one 6-member panel considered successful); particularly preferably wherein at least one and preferably at least two and more preferably at least three and even more preferably at least four of said markers and/or parameters are selected from the group consisting of those markers and parameters listed in Table 14 for which the product of dividing the number set forth in column no. 5 of Table 14 by the number in the field "number of panels" in column no. 5 of Table 14 is 0.20 (20%) or greater (i.e., the marker or parameter is present in at least 20% of 6-member panels considered successful). Particularly preferred panels of this type may comprise or consist of markers and parameters as included in any one of the exemplary panels T14.1 to T14.3, as demonstrated in Example 6.

It is also herein disclosed test panels, suitable particularly but without limitation for prediction of HDP or preferably pre-term PE using a "rule-in" test, preferably at 20 +/about 3 weeks of gestation (preferably at 20 +/- about 2 weeks or more preferably at 20 +/- about 1 week or more preferably at about 20 weeks of gestation), said panels comprising measurement of IGFALS level and measurement of blood pressure, and further optionally and preferably comprising at least one, more preferably at least two, even more preferably at least three, and still more preferably at least four markers and/or parameters selected from the group consisting of those markers and parameters listed in Table 15 of Example 6 for which the number set forth in at least one column of the columns no.2, 3, 4 and 5 of Table 15 is not 0 (i.e., the marker or parameter is present in at least one panel considered successful). Preferred ≥4-member panels of this type may comprise or consist of measurement of IGFALS level and measurement of blood pressure, and two markers and/or parameters selected from the group consisting of those markers and parameters listed in Table 15 for which the number set forth in column no. 3 of Table 15 is not 0 (i.e., the marker or parameter is present in at least one 4-member panel considered successful); particularly preferably wherein at least one and more preferably at least two of said markers and/or parameters are selected from the group consisting of those markers and parameters listed in Table 15 for which the product of dividing the number set forth in column no. 3 of Table 15 by the number in the field "number of panels" in column no. 3 of Table 15 is 0.20 (20%) or greater (i.e., the marker or parameter is present in at least 20% of 4-member panels considered successful). Preferred ≥5-member panels of this type may comprise or consist of measurement of IGFALS level and measurement of blood pressure, and three markers and/or parameters selected from the group consisting of those markers and parameters listed in Table 15 for which the number set forth in column no. 4 of Table 15 is not 0 (i.e., the marker or parameter is present in at least one 5-member panel considered successful); particularly preferably wherein at least one and preferably at least two and more preferably at least three of said markers and/or parameters are selected from the group consisting of those markers and parameters listed in Table 15 for which the product of dividing the number set forth in column no. 4 of Table 15 by the number in the field "number of panels" in column no. 4 of Table 15 is 0.20 (20%) or greater (i.e., the marker or parameter is present in at least 20% of 5-member panels considered successful). Preferred ≥6-member panels of this type may comprise or consist of measurement of IGFALS level and measurement of blood pressure, and four markers and/or parameters selected from the group consisting of those markers and parameters listed in Table 15 for which the number set forth in column no. 5 of Table 15 is not 0 (i.e., the marker or parameter is present in at least one 6-member panel considered successful); particularly preferably wherein at least one and preferably at least two and more preferably at least three and even more preferably at least four of said markers and/or parameters are selected from the group consisting of those markers and parameters listed in Table 15 for which the product of dividing the number set forth in column no. 5 of Table 15 by the number in the field "number of panels" in column no. 5 of Table 15 is 0.20 (20%) or greater (i.e., the marker or parameter is present in at least 20% of 6-member panels considered successful). Particularly preferred panels of this type may comprise or consist of markers and parameters as included in any one of the exemplary panels T15.1 to T15.7, as demonstrated in Example 6.

It is also herein disclosed test panels, suitable particularly but without limitation for prediction of HDP or preferably PE in non-obese subjects, preferably at 20 +/- about 3 weeks of gestation (preferably at 20 +/- about 2 weeks or more preferably at 20 +/- about 1 week or more preferably at about 20 weeks of gestation), said panels comprising measurement of IGFALS level and measurement of blood pressure, and further optionally and preferably comprising at least one, more preferably at least two, even more preferably at least three, and still more preferably at least four markers and/or parameters selected from the group consisting of those markers and parameters listed in Table 16 of Example 6 for which the number set forth in at least one column of the columns no.2, 3, 4 and 5 of Table 16 is not 0 (i.e., the marker or parameter is present in at least one panel considered successful). Preferred ≥3-member panels of this type may comprise or consist of measurement of IGFALS level and measurement of blood pressure, and one marker or parameter selected from the group consisting of those markers and parameters listed in Table 16 for which the number set forth in column no. 2 of Table 16 is not 0 (i.e., the marker or parameter is present in at least one 3-member panel considered successful); particularly preferably selected from the group consisting of those markers and parameters listed in Table 16 for which the product of dividing the number set forth in column no. 2 of Table 16 by the number in the field "number of panels" in column no. 2 of Table 16 is 0.20 (20%) or greater (i.e., the marker or parameter is present in at least 20% of 3-member panels considered successful). Preferred ≥4-member panels of this type may comprise or consist of measurement of IGFALS level and measurement of blood pressure, and two markers and/or parameters selected from the group consisting of those markers and parameters listed in Table 16 for which the number set forth in column no. 3 of Table 16 is not 0 (i.e., the marker or parameter is present in at least one 4-member panel considered successful); particularly preferably wherein at least one and more preferably at least two of said markers and/or parameters are selected from the group consisting of those markers and parameters listed in Table 16 for which the product of dividing the number set forth in column no. 3 of Table 16 by the number in the field "number of panels" in column no. 3 of Table 16 is 0.20 (20%) or greater (i.e., the marker or parameter is present in at least 20% of 4-member panels considered successful). Preferred ≥5-member panels of this type may comprise or consist of measurement of IGFALS level and measurement of blood pressure, and three markers and/or parameters selected from the group consisting of those markers and parameters listed in Table 16 for which the number set forth in column no. 4 of Table 16 is not 0 (i.e., the marker or parameter is present in at least one 5-member panel considered successful); particularly preferably wherein at least one and preferably at least two and more preferably at least three of said markers and/or parameters are selected from the group consisting of those markers and parameters listed in Table 16 for which the product of dividing the number set forth in column no. 4 of Table 16 by the number in the field "number of panels" in column no. 4 of Table 16 is 0.20 (20%) or greater (i.e., the marker or parameter is present in at least 20% of 5-member panels considered successful). Preferred ≥6-member panels of this type may comprise or consist of measurement of IGFALS level and measurement of blood pressure, and four markers and/or parameters selected from the group consisting of those markers and parameters listed in Table 16 for which the number set forth in column no. 5 of Table 16 is not 0 (i.e., the marker or parameter is present in at least one 6-member panel considered successful); particularly preferably wherein at least one and preferably at least two and more preferably at least three and even more preferably at least four of said markers and/or parameters are selected from the group consisting of those markers and parameters listed in Table 16 for which the product of dividing the number set forth in column no. 5 of Table 16 by the number in the field "number of panels" in column no. 5 of Table 16 is 0.20 (20%) or greater (i.e., the marker or parameter is present in at least 20% of 6-member panels considered successful). Particularly preferred panels of this type may comprise or consist of markers and parameters as included in any one of the exemplary panels T16.1 to T16.4, as demonstrated in Example 6.

It is also herein disclosed test panels, suitable particularly but without limitation for prediction of HDP or preferably PE in non-obese subjects using a "rule-in" test, preferably at 20 +/- about 3 weeks of gestation (preferably at 20 +/- about 2 weeks or more preferably at 20 +/- about 1 week or more preferably at about 20 weeks of gestation), said panels comprising measurement of IGFALS level and measurement of blood pressure, and further optionally and preferably comprising at least one, more preferably at least two, even more preferably at least three, and still more preferably at least four markers and/or parameters selected from the group consisting of those markers and parameters listed in Table 17 of Example 6 for which the number set forth in at least one column of the columns no.2, 3, 4 and 5 of Table 17 is not 0 (i.e., the marker or parameter is present in at least one panel considered successful). Preferred ≥4-member panels of this type may comprise or consist of measurement of IGFALS level and measurement of blood pressure, and two markers and/or parameters selected from the group consisting of those markers and parameters listed in Table 17 for which the number set forth in column no. 3 of Table 17 is not 0 (i.e., the marker or parameter is present in at least one 4-member panel considered successful); particularly preferably wherein at least one and more preferably at least two of said markers and/or parameters are selected from the group consisting of those markers and parameters listed in Table 17 for which the product of dividing the number set forth in column no. 3 of Table 17 by the number in the field "number of panels" in column no. 3 of Table 17 is 0.20 (20%) or greater (i.e., the marker or parameter is present in at least 20% of 4-member panels considered successful). Preferred ≥5-member panels of this type may comprise or consist of measurement of IGFALS level and measurement of blood pressure, and three markers and/or parameters selected from the group consisting of those markers and parameters listed in Table 17 for which the number set forth in column no. 4 of Table 17 is not 0 (i.e., the marker or parameter is present in at least one 5-member panel considered successful); particularly preferably wherein at least one and preferably at least two and more preferably at least three of said markers and/or parameters are selected from the group consisting of those markers and parameters listed in Table 17 for which the product of dividing the number set forth in column no. 4 of Table 17 by the number in the field "number of panels" in column no. 4 of Table 17 is 0.20 (20%) or greater (i.e., the marker or parameter is present in at least 20% of 5-member panels considered successful). Preferred ≥6-member panels of this type may comprise or consist of measurement of IGFALS level and measurement of blood pressure, and four markers and/or parameters selected from the group consisting of those markers and parameters listed in Table 17 for which the number set forth in column no. 5 of Table 17 is not 0 (i.e., the marker or parameter is present in at least one 6-member panel considered successful); particularly preferably wherein at least one and preferably at least two and more preferably at least three and even more preferably at least four of said markers and/or parameters are selected from the group consisting of those markers and parameters listed in Table 17 for which the product of dividing the number set forth in column no. 5 of Table 17 by the number in the field "number of panels" in column no. 5 of Table 17 is 0.20 (20%) or greater (i.e., the marker or parameter is present in at least 20% of 6-member panels considered successful). Particularly preferred panels of this type may comprise or consist of markers and parameters as included in any one of the exemplary panels T17.1 to T17.8, as demonstrated in Example 6.

It is also herein disclosed test panels, suitable particularly but without limitation for prediction of HDP or preferably pre-term PE in non-obese subjects, preferably at 20 +/about 3 weeks of gestation (preferably at 20 +/- about 2 weeks or more preferably at 20 +/- about 1 week or more preferably at about 20 weeks of gestation), said panels comprising measurement of IGFALS level and measurement of blood pressure, and further optionally and preferably comprising at least one, more preferably at least two, even more preferably at least three, and still more preferably at least four markers and/or parameters selected from the group consisting of those markers and parameters listed in Table 18 of Example 6 for which the number set forth in at least one column of the columns no.2, 3, 4 and 5 of Table 18 is not 0 (i.e., the marker or parameter is present in at least one panel considered successful). Preferred ≥3-member panels of this type may comprise or consist of measurement of IGFALS level and measurement of blood pressure, and one marker or parameter selected from the group consisting of those markers and parameters listed in Table 18 for which the number set forth in column no. 2 of Table 18 is not 0 (i.e., the marker or parameter is present in at least one 3-member panel considered successful); particularly preferably selected from the group consisting of those markers and parameters listed in Table 18 for which the product of dividing the number set forth in column no. 2 of Table 18 by the number in the field "number of panels" in column no. 2 of Table 18 is 0.20 (20%) or greater (i.e., the marker or parameter is present in at least 20% of 3-member panels considered successful). Preferred ≥4-member panels of this type may comprise or consist of measurement of IGFALS level and measurement of blood pressure, and two markers and/or parameters selected from the group consisting of those markers and parameters listed in Table 18 for which the number set forth in column no. 3 of Table 18 is not 0 (i.e., the marker or parameter is present in at least one 4-member panel considered successful); particularly preferably wherein at least one and more preferably at least two of said markers and/or parameters are selected from the group consisting of those markers and parameters listed in Table 18 for which the product of dividing the number set forth in column no. 3 of Table 18 by the number in the field "number of panels" in column no. 3 of Table 18 is 0.20 (20%) or greater (i.e., the marker or parameter is present in at least 20% of 4-member panels considered successful). Preferred ≥5-member panels of this type may comprise or consist of measurement of IGFALS level and measurement of blood pressure, and three markers and/or parameters selected from the group consisting of those markers and parameters listed in Table 18 for which the number set forth in column no. 4 of Table 18 is not 0 (i.e., the marker or parameter is present in at least one 5-member panel considered successful); particularly preferably wherein at least one and preferably at least two and more preferably at least three of said markers and/or parameters are selected from the group consisting of those markers and parameters listed in Table 18 for which the product of dividing the number set forth in column no. 4 of Table 18 by the number in the field "number of panels" in column no. 4 of Table 18 is 0.20 (20%) or greater (i.e., the marker or parameter is present in at least 20% of 5-member panels considered successful). Preferred ≥6-member panels of this type may comprise or consist of measurement of IGFALS level and measurement of blood pressure, and four markers and/or parameters selected from the group consisting of those markers and parameters listed in Table 18 for which the number set forth in column no. 5 of Table 18 is not 0 (i.e., the marker or parameter is present in at least one 6-member panel considered successful); particularly preferably wherein at least one and preferably at least two and more preferably at least three and even more preferably at least four of said markers and/or parameters are selected from the group consisting of those markers and parameters listed in Table 18 for which the product of dividing the number set forth in column no. 5 of Table 18 by the number in the field "number of panels" in column no. 5 of Table 18 is 0.20 (20%) or greater (i.e., the marker or parameter is present in at least 20% of 6-member panels considered successful). Particularly preferred panels of this type may comprise or consist of markers and parameters as included in any one of the exemplary panels T18.1 to T18.4, as demonstrated in Example 6.

It is also herein disclosed test panels, suitable particularly but without limitation for prediction of HDP or preferably pre-term PE in non-obese subjects using a "rule-in" test, preferably at 20 +/- about 3 weeks of gestation (preferably at 20 +/- about 2 weeks or more preferably at 20 +/- about 1 week or more preferably at about 20 weeks of gestation), said panels comprising measurement of IGFALS level and measurement of blood pressure, and further optionally and preferably comprising at least one, more preferably at least two, even more preferably at least three, and still more preferably at least four markers and/or parameters selected from the group consisting of those markers and parameters listed in Table 19 of Example 6 for which the number set forth in at least one column of the columns no.2, 3, 4 and 5 of Table 19 is not 0 (i.e., the marker or parameter is present in at least one panel considered successful). Preferred ≥4-member panels of this type may comprise or consist of measurement of IGFALS level and measurement of blood pressure, and two markers and/or parameters selected from the group consisting of those markers and parameters listed in Table 19 for which the number set forth in column no. 3 of Table 19 is not 0 (i.e., the marker or parameter is present in at least one 4-member panel considered successful); particularly preferably wherein at least one and more preferably at least two of said markers and/or parameters are selected from the group consisting of those markers and parameters listed in Table 19 for which the product of dividing the number set forth in column no. 3 of Table 19 by the number in the field "number of panels" in column no. 3 of Table 19 is 0.20 (20%) or greater (i.e., the marker or parameter is present in at least 20% of 4-member panels considered successful). Preferred ≥5-member panels of this type may comprise or consist of measurement of IGFALS level and measurement of blood pressure, and three markers and/or parameters selected from the group consisting of those markers and parameters listed in Table 19 for which the number set forth in column no. 4 of Table 19 is not 0 (i.e., the marker or parameter is present in at least one 5-member panel considered successful); particularly preferably wherein at least one and preferably at least two and more preferably at least three of said markers and/or parameters are selected from the group consisting of those markers and parameters listed in Table 19 for which the product of dividing the number set forth in column no. 4 of Table 19 by the number in the field "number of panels" in column no. 4 of Table 19 is 0.20 (20%) or greater (i.e., the marker or parameter is present in at least 20% of 5-member panels considered successful). Preferred ≥6-member panels of this type may comprise or consist of measurement of IGFALS level and measurement of blood pressure, and four markers and/or parameters selected from the group consisting of those markers and parameters listed in Table 19 for which the number set forth in column no. 5 of Table 19 is not 0 (i.e., the marker or parameter is present in at least one 6-member panel considered successful); particularly preferably wherein at least one and preferably at least two and more preferably at least three and even more preferably at least four of said markers and/or parameters are selected from the group consisting of those markers and parameters listed in Table 19 for which the product of dividing the number set forth in column no. 5 of Table 19 by the number in the field "number of panels" in column no. 5 of Table 19 is 0.20 (20%) or greater (i.e., the marker or parameter is present in at least 20% of 6-member panels considered successful). Particularly preferred panels of this type may comprise or consist of markers and parameters as included in any one of the exemplary panels T19.1 to T19.7, as demonstrated in Example 6.

Disclosed herein is also the use of a test panel comprising measurement of IGFALS level, a score for the maternal history parameter *fh_petxcardio,* and measurement of blood pressure, and optionally and preferably further comprising at least one, more preferably at least two or even at least three of measurement of the level of SEPP1, measurement of the level of s-Endoglin, measurement of the level of QSOX1, measurement of the level of PRDX2, measurement of blood glucose level, measurement of BMI, a score for *father_any_ihd,* a score for *fh_pet,* a value for *bb_hdl,* a value for *bb_total_hdl_ratio,* a score for *metabolic syndrome,* a value for *bb_trig,* measurement of the level of FLT4, measurement of the level of PRCP, measurement of the level of PRDX1, measurement of the level of LNPEP, measurement of the level of TNXB, measurement of the level of HSPG2, measurement of the level of MUC18, measurement of the level of GPLD1, measurement of the level of COL6A3, measurement of the level of SPINT1, measurement of the level of MST1, measurement of the level of GPR126, measurement of the level of ICAM3, measurement of the level of CRP, measurement of the level of ADAM12, measurement of the level of LCAT, measurement of the level of ROBO4, measurement of the level of ENPP2, and measurement of the level of S100A9; or, preferably, further comprising at least one, more preferably at least two or even at least three of SEPP1 level, ENG level, QSOX1 level, PRDX2 level, FLT4 level, PRCP level, PRDX1 level, LNPEP level, TNXB level, HSPG2 level, MUC18 / MCAM level, GPLD1 level, COL6A3 level, SPINT1 level, MST1 level, GPR126 level, ICAM3 level, CRP level, measurement of blood glucose level, measurement of BMI, a score for the maternal history parameter *father_any_ihd,* and a score for the maternal history parameter *fh_pet,* for the diagnosis, prediction, prognosis and/or monitoring HDP or PE, preferably for the prediction of HDP or PE, more preferably for the prediction of PE. Preferably, in such uses as detailed in this paragraph, the test panel may comprise a score for *father_any_ihd* instead of the score for *fh_petxcardio.*

Disclosed herein is also the use of a test panel comprising IGFALS level, the score for *father_any_ihd,* and measurement of blood pressure, and optionally and preferably further comprising at least one, more preferably at least two or even at least three of SEPP1 level, ENG level, measurement of BMI, a score for *fh_pet,* a value for *bb_hdl,* a value for *bb_total_hdl_ratio,* a score for *metabolic syndrome,* HSPG2 level, MUC18 level, SPINT1 level, ADAM12 level, LCAT level, ROBO4 level, ENPP2 level and S100A9 level, for the diagnosis, prediction, prognosis and/or monitoring HDP or PE, preferably for the prediction of HDP or PE, more preferably for the prediction of PE.

Disclosed herein is also the use of a test panel comprising IGFALS level and measurement of blood pressure, and optionally and preferably further comprising at least one, more preferably at least two or even at least three of the level of SEPP1, measurement of the level of s-Endoglin, measurement of the level of QSOX1, measurement of the level of PRDX2, measurement of blood glucose level, measurement of BMI, a score for *father_any_ihd,* a score for *fh_pet,* a value for *bb_hdl,* a value for *bb_total_hdl_ratio,* a score for *metabolic syndrome,* a value for *bb_trig,* measurement of the level of FLT4, measurement of the level of PRCP, measurement of the level of PRDX1, measurement of the level of LNPEP, measurement of the level of TNXB, measurement of the level of HSPG2, measurement of the level of MUC18, measurement of the level of GPLD1, measurement of the level of COL6A3, measurement of the level of SPINT1, measurement of the level of MST1, measurement of the level of GPR126, measurement of the level of ICAM3, measurement of the level of CRP, measurement of the level of ADAM12, measurement of the level of LCAT, measurement of the level of ROBO4, measurement of the level of ENPP2, and measurement of the level of S100A9; or, preferably, further comprising at least one, more preferably at least two or even at least three of SEPP1 level, ENG level, measurement of BMI, a score for *father_any_ihd,* a score for *fh_pet,* a value for *bb_hdl,* a value for *bb_total_hdl_ratio,* a score for *metabolic syndrome,* a value for *bb_trig,* HSPG2 level, MUC18 level, SPINT1 level, ADAM12 level, LCAT level, ROBO4 level, ENPP2 level and S100A9 level, for the diagnosis, prediction, prognosis and/or monitoring HDP or PE, preferably for the prediction of HDP or PE, more preferably for the prediction of PE.

Also disclosed herein is the use of any one of the test panels described above, such as in particular but without limitation any one of the test panels denoted herein as A, B, C, D, E, F G, H, I or J, for the diagnosis, prediction, prognosis and/or monitoring HDP or PE, preferably for the prediction of HDP or PE, more preferably for the prediction of PE, more preferably wherein said panels A, B, C, D, E, F G, H, I or J comprise a score for *father_any_ihd* instead of the score for *fh_petxcardio.* Where applicable and desired, a given test panel may be so used or evaluated at its respective preferred age of gestation, as explained hereinbefore.

Also disclosed herein is a method for the diagnosis, prediction, prognosis and/or monitoring of HDP or PE, preferably for the prediction of HDP or PE, more preferably for the prediction of PE, in a subject comprising testing or evaluating in said subject a test panel comprising measurement of IGFALS level, a score for the maternal history parameter *fh_petxcardio,* and measurement of blood pressure, and optionally and preferably further comprising at least one, more preferably at least two or even at least three of the level of SEPP1, measurement of the level of s-Endoglin, measurement of the level of QSOX1, measurement of the level of PRDX2, measurement of blood glucose level, measurement of BMI, a score for *father_any_ihd,* a score for *fh_pet,* a value for *bb_hdl,* a value for *bb_total_hdl_ratio,* a score for *metabolic syndrome,* a value for *bb_trig,* measurement of the level of FLT4, measurement of the level of PRCP, measurement of the level of PRDX1, measurement of the level of LNPEP, measurement of the level of TNXB, measurement of the level of HSPG2, measurement of the level of MUC18, measurement of the level of GPLD1, measurement of the level of COL6A3, measurement of the level of SPINT1, measurement of the level of MST1, measurement of the level of GPR126, measurement of the level of ICAM3, measurement of the level of CRP, measurement of the level of ADAM12, measurement of the level of LCAT, measurement of the level of ROBO4, measurement of the level of ENPP2, and measurement of the level of S100A9; or, preferably, further comprising at least one, more preferably at least two or even at least three of SEPP1 level, ENG level, QSOX1 level, PRDX2 level, FLT4 level, PRCP level, PRDX1 level, LNPEP level, TNXB level, HSPG2 level, MUC18 / MCAM level, GPLD1 level, COL6A3 level, SPINT1 level, MST1 level, GPR126 level, ICAM3 level, CRP level, measurement of blood glucose level, measurement of BMI, a score for the maternal history parameter *father_any_ihd,* and a score for the maternal history parameter *fh_pet.* Preferably, in such methods as detailed in this paragraph, the test panel may comprise a score for *father_any_ihd* instead of the score for *fh_petxcardio.*

Also disclosed herein is a method for the diagnosis, prediction, prognosis and/or monitoring, preferably prediction, of HDP or preferably PE in a subject comprising testing or evaluating in said subject a test panel comprising IGFALS level, the score for *father_any_ihd,* and measurement of blood pressure, and optionally and preferably further comprising at least one, more preferably at least two or even at least three of SEPP1 level, ENG level, measurement of BMI, a score for *fh_pet,* a value for *bb_hdl,* a value for *bb_total_hdl_ratio,* a score for *metabolic syndrome,* HSPG2 level, MUC18 level, SPINT1 level, ADAM12 level, LCAT level, ROBO4 level, ENPP2 level and S100A9 level.

Also disclosed herein is a method for the diagnosis, prediction, prognosis and/or monitoring, preferably prediction, of HDP or preferably PE in a subject comprising testing or evaluating in said subject a test panel comprising IGFALS level and measurement of blood pressure, and optionally and preferably further comprising at least one, more preferably at least two or even at least three of the level of SEPP1, measurement of the level of s-Endoglin, measurement of the level of QSOX1, measurement of the level of PRDX2, measurement of blood glucose level, measurement of BMI, a score for *father_any_ihd,* a score for *fh_pet,* a value for *bb_hdl,* a value for *bb_total_hdl_ratio,* a score for *metabolic syndrome,* a value for *bb_trig,* measurement of the level of FLT4, measurement of the level of PRCP, measurement of the level of PRDX1, measurement of the level of LNPEP, measurement of the level of TNXB, measurement of the level of HSPG2, measurement of the level of MUC18, measurement of the level of GPLD1, measurement of the level of COL6A3, measurement of the level of SPINT1, measurement of the level of MST1, measurement of the level of GPR126, measurement of the level of ICAM3, measurement of the level of CRP, measurement of the level of ADAM12, measurement of the level of LCAT, measurement of the level of ROBO4, measurement of the level of ENPP2, and measurement of the level of S100A9; or, preferably, further comprising at least one, more preferably at least two or even at least three of SEPP1 level, ENG level, measurement of BMI, a score for *father_any_ihd,* a score for *fh_pet,* a value for *bb_hdl,* a value for *bb_total_hdl_ratio,* a score for *metabolic syndrome,* a value for *bb_trig,* HSPG2 level, MUC18 level, SPINT1 level, ADAM12 level, LCAT level, ROBO4 level, ENPP2 level and S100A9 level.

Also disclosed is a method for the diagnosis, prediction, prognosis and/or monitoring of HDP or PE, preferably for the prediction of HDP or PE, more preferably for the prediction of PE, in a subject comprising testing or evaluating in said subject any one of the test panels described above, such as in particular but without limitation any one of the test panels denoted herein as A, B, C, D, E, F G, H, I or J, more preferably wherein said panels A, B, C, D, E, F G, H, I or J comprise a score for *father_any_ihd* instead of the score for *fh_petxcardio.* Where applicable and desired, the method may be performed for a given test panel at its respective preferred age of gestation, as explained hereinbefore.

To test or evaluate a test panel in a subject, the present methods, and particularly the examination phase of such methods in which data is collected from and/or about the subject, comprise measuring the level (i.e., quantity, amount) of the biomarker(s) comprised in said test panel in a sample from the subject and measuring or scoring the parameter(s) comprised in said test panel.

Hence, a method for the diagnosis, prediction and/or prognosis of HDP or PE in a subject using a test panel as taught herein may comprise steps: (i) measuring the quantity of the biomarker or biomarkers comprised in said test panel in the sample from the subject and measuring or scoring the parameter or parameters comprised in said test panel in the subject; (ii) comparing the quantity of the biomarker or biomarkers and the measurement or score of the parameter or parameters as measured or scored in (i) with a reference value representing a known diagnosis, prediction and/or prognosis of HDP or PE; (iii) finding a deviation or no deviation of the quantity of the biomarker or biomarkers and/or the measurement or score of the parameter or parameters as measured or scored in (i) from the reference value; and (iv) attributing said finding of deviation or no deviation to a particular diagnosis, prediction and/or prognosis of HDP or PE in the subject. The method may be performed for a subject at two or more successive time points and the respective outcomes at said successive time points may be compared, whereby the presence or absence of a change between the diagnosis, prediction and/or prognosis of HDP or PE at said successive time points is determined. When so applied, the method can monitor a change in the diagnosis, prediction and/or prognosis of HDP or PE in the subject over time.

For example, a deviation of the quantity of the biomarker(s) in a sample from a subject and the measurement or score of parameter(s) in the subject compared to a reference value representing the prediction or diagnosis of no HDP or PE (i.e., healthy state) or representing a good prognosis for HDP or PE can indicate respectively that the subject has or is at risk of having HDP or PE or can indicate a poor prognosis for HDP or PE in the subject (such as, e.g., a prognosis that PE will worsen or progress to HELLP syndrome or eclampsia). In another example, the absence of a deviation from a reference value representing the prediction or diagnosis of no HDP or PE or representing a good prognosis for HDP or PE can indicate respectively that the subject does not have or is not at risk of having HDP or PE or can indicate a good prognosis for HDP or PE in the subject. In yet another example, the absence of a deviation from a reference value representing the prediction or diagnosis of HDP or PE (i.e., disease state) or representing a poor prognosis for HDP or PE can indicate respectively that the subject has or is at risk of having HDP or PE or can indicate a poor prognosis for HDP or PE in the subject.

The quantity of biomarker(s) and the measurement or score of parameter(s) may vary during pregnancy and/or postpartum. To improve the accuracy of the present methods and uses, the quantity of biomarker(s) and the measurement or score of parameter(s) measured or scored at a given age of gestation or postpartum in the subject under examination are preferably compared to a reference value established at the same or substantially the same age of gestation or postpartum, e.g., within +/- about 3 weeks, preferably within +/- about 2 weeks, more preferably within +/- about 1 week, yet more preferably within +/- about 0.5 week.

Preferably, a method for monitoring HDP or PE or for monitoring the probability of developing HDP or PE using a test panel as taught herein comprises the steps of: (i) measuring the quantity of the biomarker or biomarkers comprised in said test panel in the sample from the subject and measuring or scoring the parameter or parameters comprised in said test panel in the subject at two or more successive time points; (ii) comparing the quantity of the biomarker or biomarkers and the measurement or score of the parameter or parameters as measured or scored in (i) between said two or more successive time points; (iii) finding a deviation or no deviation of the quantity of the biomarker or biomarkers and/or the measurement or score of the parameter or parameters as measured or scored in (i) between said two or more successive time points; (iv) attributing said finding of deviation or no deviation to a change in HDP or PE to a change in the probability of developing HDP or PE in the subject between the two or more successive time points.

Also disclosed is a method to determine whether a subject is or is not (such as, e.g., still is, or is no longer) in need of a therapeutic or prophylactic (preventative) treatment of HDP or PE using a test panel as taught herein, comprising: (i) measuring the quantity of the biomarker or biomarkers comprised in said test panel in the sample from the subject and measuring or scoring the parameter or parameters comprised in said test panel in the subject; (ii) comparing the quantity of the biomarker or biomarkers and the measurement or score of the parameter or parameters as measured or scored in (i) with a reference value representing a known diagnosis, prediction and/or prognosis of HDP or PE; (iii) finding a deviation or no deviation of the quantity of the biomarker or biomarkers and/or the measurement or score of the parameter or parameters as measured or scored in (i) from the reference value; (iv) inferring from said finding the presence or absence of a need for a therapeutic or prophylactic treatment of HDP or PE.

A treatment may be particularly indicated where the method allows for a conclusion that the subject has or is at risk of having HDP or PE or has a poor prognosis for HDP or PE. For example, a patient having HDP or PE upon admission to or during stay in a medical care centre may be tested as taught herein for the necessity of continuing the treatment of said HDP or PE, and may be discharged when such treatment is no longer needed or is needed only to a given limited extent.

Illustrative therapeutic and prophylactic treatments of HDP or PE encompass *inter alia* anti-hypertensive treatments (using *inter alia* beta-blockers, calcium channel blockers, vasodilators and/or DOPA decarboxylase inhibitors, such as, e.g., methyldopa, labetalol, acebutolol, metoprolol, pindolol, propranolol, nifedipine, isradipine and/or hydralazine, $MgSO_4$ treatment and/or aspirin (see, e.g., Bujold et al., Obstet Gynecol 2010, vol. 116, 402-14)), abortion, and delivery such as by labour induction or Caesarean section.

The herein disclosed test panels, methods and uses may be particularly useful in subjects known or expected to be at risk of developing HDP or PE, e.g., having one or more risk factors for HDP or PE. Without limitation risk factors associated with HDP and preferably PE include nulliparity, multiple gestation, prolonged interval between pregnancies, history of HDP or PE in a prior pregnancy or family history of HDP or PE, extremes in age (<20 years and >40 years), obesity, chronic hypertension, chronic renal disease, migraine, headaches, (gestational) diabetes mellitus, polycystic ovarian syndrome, autoimmune disorders such as lupus, rheumatoid arthritis, sarcoidosis or MS, vascular or connective tissue diseases, vitamin D insufficiency, antiphospholipid antibody syndrome or inherited thrombophilia, male partner whose previous partner had HDP or PE, hydrops fetalis and unexplained foetal intrauterine growth restriction.

Preferably, the disclosed test panels, methods and uses may be complemented or combined with determination of the presence or absence and/or level of one or more risk factors for HDP or PE in the subject.

In general clinical practice obese subjects (BMI ≥ 30 pre-pregnancy or in 1st trimester) are considered at risk for a number pregnancy complications, including for example gestational diabetes, pre-eclampsia, etc., and therefore already subject to increased antenatal care (NHS National Institute for Health and Clinical Excellence (NICE) clinical guideline 62: Antenatal Care - Routine Care for the Healthy Pregnant woman, March 2008). Therefore, panels are provided and may be used in non-obese subjects (BMI < 30), more particularly in nulliparous non-obese women.

Any one test panel, method or use as taught herein may preferably allow for sensitivity and/or specificity (preferably, sensitivity and specificity) of at least 50%, at least 60%, at least 70% or at least 80%, e.g., ≥ 85% or ≥ 90% or ≥95%, e.g., between about 80% and 100% or between about 85% and 95%.

Reference throughout this specification to "diseases and/or conditions" encompasses any such diseases and conditions as disclosed herein insofar consistent with the context of a particular recitation, more specifically but without limitation including hypertensive disorders of pregnancy (HDP) and preferably preeclampsia (PE).

The disclosed test panels, methods and uses may be applied to subjects who have not yet been diagnosed as having the respective diseases and conditions (for example, preventative screening), or who have been diagnosed as having such, or who are suspected of having such (for example, display one or more characteristic signs and/or symptoms), or who are at risk of developing such (for example, genetic predisposition; presence of one or more developmental, environmental or behavioural risk factors). The test panels, methods and uses may also be used to detect various stages of progression or severity of the diseases and conditions. The test panels, methods and uses may also be used to detect response of the diseases and conditions to prophylactic or therapeutic treatments or other interventions. The test panels, methods and uses can furthermore be used to help the medical practitioner in deciding upon worsening, status-quo, partial recovery, or complete recovery of the subject from the diseases and conditions, resulting in either further treatment or observation or in discharge of the patient from a medical care centre. Also, the test panels, methods and uses as taught herein may be employed for population screening, such as, e.g., screening in a general population or in a population stratified based on one or more criteria, e.g., age, ancestry, occupation, presence or absence of risk factors of the respective diseases and conditions, *etc.*

The disclosed test panels, methods and uses may also benefit from being further complemented or combined with the assessment of one or more other biomarkers and/or clinical parameters relevant for the respective diseases and conditions.

By means of example and not limitation, other biomarkers useful in evaluating HDP or PE include soluble fms-like tyrosine kinase-1 (sFlt-1, sVEGFR-1) (Maynard et al. 2003, J Clin Invest 111(5): 649-58), placental growth factor (PIGF) and vascular endothelial growth factor (VEGF) (Polliotti et al. 2003; Obstet Gynecol 101: 1266-74), and biomarkers disclosed in WO2009/097584A1 to Proteogenix Inc. and WO2009/108073A1 to Auckland Uniservices Ltd..

Additional useful clinical parameters for the pregnant female subject may include without limitation, age, ethnicity, smoking status (esp. at 15 weeks visit), alcohol consumption (esp. 1st trimester), birth weight, occurrence of vaginal bleeding (esp. for (more than) 5 days before 15 weeks visit) (yes/no), etc.

The respective quantities, measurements or scores for the biomarker(s) and parameter(s) in the present test panels may be evaluated separately and individually, i.e., each compared with its corresponding reference value. More advantageously, the quantities, measurements or scores for the biomarker(s) and parameter(s) may be used to establish a biomarker-and-parameter profile, which can be suitably compared with a corresponding multi-parameter reference value. In yet another alternative, the quantities, measurements or scores for the biomarker(s) and parameter(s) may each be modulated by an appropriate weighing factor and added up to yield a single value, which can then be suitably compared with a corresponding reference value obtained accordingly. One shall appreciate that such weighing factors may depend on the methodology used to quantify biomarkers and measure or score parameters, and for each particular experimental setting may be determined and comprised in a model suitable for diagnosis, prediction and/or prognosis of the diseases and conditions as taught herein. Various methods can be used for the purpose of establishing such models, e.g., support vector machine, Bayes classifiers, logistic regression, etc. (Cruz et al. Applications of Machine Learning in Cancer Prediction and Prognosis. Cancer Informatics 2007; 2; 59-77).

Reference values as employed herein may be established according to known procedures previously employed for other test panels comprising biomarkers and/or clinical parameters. Reference values may be established either within (*i.e.*, constituting a step of) or external to (*i.e.*, not constituting a step of) the methods and uses as taught herein. Accordingly, any one of the methods or uses taught herein may comprise a step of establishing a requisite reference value.

Hence, also disclosed is a method for establishing a reference value for a test panel as taught herein, said reference value representing:

(a) a prediction or diagnosis of the absence of the diseases or conditions as taught herein or a good prognosis thereof, or

(b) a prediction or diagnosis of the diseases or conditions as taught herein or a poor prognosis thereof,

comprising:

(i) measuring the quantity of the biomarker or biomarkers comprised in said test panel in a sample from, and measuring or scoring the parameter or parameters comprised in said test panel in:

(i a) one or more subjects not having the respective diseases or conditions or not being at risk of having such or having a good prognosis for such, or

(i b) one or more subjects having the respective diseases or conditions or being at risk of having such or having a poor prognosis for such, and

(ii a) establishing from the quantity of the biomarker or biomarkers and measurement or score of the parameter or parameters as measured in (i a) the reference value representing the prediction or diagnosis of the absence of the respective diseases or conditions or representing the good prognosis therefore, or

(ii b) establishing from the quantity of the biomarker or biomarkers and measurement or score of the parameter or parameters as measured in (i b) the reference value representing the prediction or diagnosis of the respective diseases or conditions or representing the poor prognosis therefore.

Further disclosed is a method for establishing a base-line reference value for a test panel as taught herein in a subject, comprising: (i) measuring the quantity of the biomarker or biomarkers comprised in said test panel in a sample from the subject, and measuring or scoring the parameter or parameters comprised in said test panel in the subject at one or more time points when the subject is not suffering from the diseases or conditions as taught herein, and (ii) establishing from the quantity of the biomarker or biomarkers and measurement or score of the parameter or parameters as measured in (i) a range or mean reference value for the subject, which is the base-line reference value for said subject.

The quantity of biomarker(s) may be measured by any suitable technique such as may be known in the art.

For example, one may employ binding agents capable of specifically binding to the respective biomarkers.

Binding agent may be *inter alia* an antibody, aptamer, photoaptamer, protein, peptide, peptidomimetic or a small molecule. For instance, one may employ an immunoassay technology or a mass spectrometry analysis method or a chromatography method, or a combination of said methods.

Further disclosed is a kit, particularly a kit for the diagnosis, prediction, prognosis and/or monitoring of the diseases or conditions as taught herein in a subject, the kit comprising (i) means for measuring the biomarker or biomarkers comprised in a test panel as taught herein, particularly in a sample from the subject, (ii) optionally means for measuring or scoring the parameter or parameters comprised in the test panel (however, said parameter(s) may be determined independently using devices other than the kit), particularly in the subject, and (iii) optionally and preferably a reference value for the test panel or means for establishing said reference value, wherein said reference value represents a known diagnosis, prediction and/or prognosis of the respective diseases or conditions.

The means for measuring the quantity of the biomarker(s) in the present kits may comprise, respectively, one or more binding agents capable of specifically binding to said biomarker(s). Binding agent may be *inter alia* an antibody, aptamer, photoaptamer, protein, peptide, peptidomimetic or a small molecule. A binding agent may be advantageously immobilised on a solid phase or support. The present kits may employ an immunoassay technology or mass spectrometry analysis technology or chromatography technology, or a combination of said technologies.

Disclosed is thus also a kit, particularly a kit for the diagnosis, prediction, prognosis and/or monitoring the diseases or conditions as taught herein in a subject, the kit comprising: (i) one or more binding agents capable of specifically binding to the biomarker or biomarkers comprised in a test panel as taught herein, particularly in a sample from the subject, (ii) preferably, a known quantity or concentration of said biomarker or biomarkers (e.g., for use as controls, standards and/or calibrators), (iii) optionally means for measuring or scoring the parameter or parameters comprised in the test panel, particularly in the subject (however, said parameter(s) may be determined independently using devices other than the kit), (iv) optionally and preferably a reference value for the test panel or means for establishing said reference value, wherein said reference value represents a known diagnosis, prediction and/or prognosis of the respective diseases or conditions. Said components under (i) and/or (ii) may be suitably labelled as taught elsewhere in this specification.

Further disclosed is the use of any one kit as described herein for the diagnosis, prediction, prognosis and/or monitoring the diseases or conditions as taught herein.

Also disclosed are reagents and tools useful for measuring biomarker(s) comprised in test panels as taught herein. Hence, disclosed is a protein, polypeptide or peptide array or microarray comprising the biomarker or biomarkers comprised in a test panel as taught herein. Also disclosed is a binding agent array or microarray comprising one or more binding agents capable of specifically binding to the biomarker or biomarkers comprised in a test panel as taught herein, preferably a known quantity of, or concentration of said binding agents.

Also disclosed are kits as taught here above configured as portable devices, such as, for example, bed-side devices, for use at home or in clinical settings.

A related aspect thus discloses a portable testing device capable of measuring the quantity of the biomarker or biomarkers comprised in a test panel as taught herein in a sample from a subject comprising: (i) means for obtaining a sample from the subject, (ii) means for measuring the quantity of the biomarker or biomarkers comprised in the test panel in said sample, and (iii) means for visualising the quantity of said biomarker or biomarkers in the sample. The testing device may optionally further comprise (iv) means for measuring or scoring the parameter or parameters comprised in the test panel in the subject (however, said parameter(s) may be determined independently using devices other than the kit), and/or (v) means for visualising the measurement or score of said parameter or parameters in the subject. Preferably, the means of parts (ii) and (iii) may be the same. Preferably, the means of parts (iii) and (v) may be the same.

Preferably, said visualising means is capable of indicating whether the quantity of the biomarker or biomarkers and the measurement or score of the parameter or parameters in the subject deviates from (e.g., is below or above) a certain reference or base-line value as taught herein. Hence, the portable testing device may suitably also comprise said reference or base-line value or means for establishing the same.

## BRIEF DESCRIPTION OF FIGURES

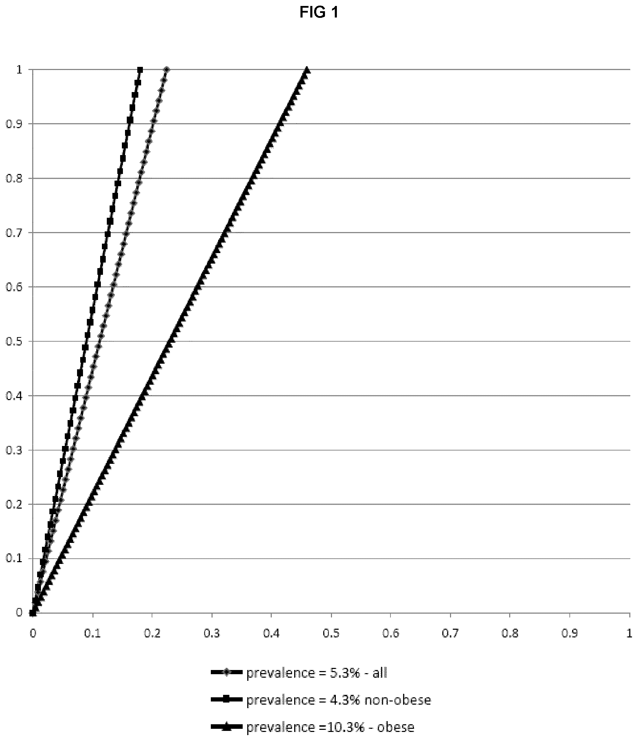

**Figure 1** shows an exemplary plot of PPV-threshold curves calculated for pre-eclampsia in all, non-obese and obese subjects based on previously reported prevalence in these populations, respectively, 5.3%, 4.3% and 10.3% (BMJ 2011, vol. 342, d1875, *supra*).

## DETAILED DESCRIPTION

As used herein, the singular forms "a", "an", and "the" include both singular and plural referents unless the context clearly dictates otherwise.

The terms "comprising", "comprises" and "comprised of" as used herein are synonymous with "including", "includes" or "containing", "contains", and are inclusive or open-ended and do not exclude additional, non-recited members, elements or method steps. The term also encompasses "consisting of" and "consisting essentially of".

The recitation of numerical ranges by endpoints includes all numbers and fractions subsumed within the respective ranges, as well as the recited endpoints.

The term "about" as used herein when referring to a measurable value such as a parameter, an amount, a temporal duration, and the like, is meant to encompass variations of and from the specified value, in particular variations of +/-10% or less, preferably +/-5% or less, more preferably +/-1% or less, and still more preferably +/-0.1 % or less of and from the specified value, insofar such variations are appropriate to perform in the disclosed invention. It is to be understood that the value to which the modifier "about" refers is itself also specifically, and preferably, disclosed.

Whereas the term "one or more", such as one or more members of a group of members, is clear per se, by means of further exemplification, the term encompasses *inter alia* a reference to any one of said members, or to any two or more of said members, such as, e.g., any ≥3, ≥4, ≥5, ≥6 or ≥7 etc. of said members, and up to all said members.

Unless otherwise specified, all terms used in disclosing the invention, including technical and scientific terms, have the meaning as commonly understood by one of ordinary skill in the art to which this invention belongs. By means of further guidance, term definitions may be included to better appreciate the teaching of the present invention.

The inventors identified test panels comprising biomarker(s) and clinical parameter(s) useful in diagnosis, prognosis, prediction and/or monitoring hypertensive disorders of pregnancy (HDP), and more specifically preeclampsia (PE).

The term "panel" or "test panel" as used herein broadly refers to combinations, sets or groupings of biomarkers and/or parameters, particularly where the testing or evaluation of such panels in subjects is predictive and/or informative as regards the subject's status, disease or condition. Without limitation, a panel as intended herein may comprise or consist of between 3 and 10, preferably between 4 and 8, more preferably 5 or 6 biomarkers and parameters.

The term "biomarker" is widespread in the art and may broadly denote a biological molecule and/or a detectable portion thereof whose qualitative and/or quantitative evaluation in a subject is, alone or combined with other data, predictive and/or informative (*e.g.*, predictive, diagnostic and/or prognostic) with respect to one or more aspects of the subject's phenotype and/or genotype, such as, for example, with respect to the status of the subject as to a given disease or condition. Particularly, biomarkers as intended herein may be metabolite-, RNA- (esp. mRNA-), peptide-, polypeptide- or protein-based, preferably peptide-, polypeptide- or protein-based.

The term "parameter" or "clinical parameter" is widespread in the art and may broadly denote information about a subject that is obtained in a clinical setting that may be relevant to a disease or condition of the subject. Particularly, parameters may encompass non-sample and/or non-analyte information. By means of illustration, clinical parameters common in medical practice may including *inter alia* basic subject characteristics such as, e.g., age, gender, weight, height, BMI, body type, ethnicity; biophysical parameters (e.g., diastolic blood pressure, systolic blood pressure, heart rate); imaging information (e.g., MRI); anamnesis information (e.g., medical history of the subject or its relatives); environmental factors etc.

As intended herein, the measurement of blood pressure may refer to any relevant blood pressure parameter, such as without limitation 1st or 2nd measurement, diastolic pressure, systolic pressure and/or mean arterial pressure. Mean arterial pressure at 15 weeks visit calculated from 2nd measurement blood pressures (henceforth "1st_vst_map_2nd") may be preferred in test panels for 15 +/- 2 or 1 weeks. Mean arterial pressure calculated at 20 weeks visit from 1st measurement blood pressures (henceforth "2nd_vst_map_1st") may be preferred in test panels for 20 +/- 2 or 1 weeks. Further, blood pressure measurements may encompass the parameters "*1st_vst_dbp_2nd*", i.e., diastolic blood pressure as obtained from the 2nd measurement at the 15 weeks visit, "*1st_vst_sbp_2nd*", i.e., systolic blood pressure as obtained from the 2nd measurement at the 15 weeks visit and/or "*2nd_vst_map_2nd*", i.e., the mean arterial pressure calculated at 20 weeks visit from 2nd measurement blood pressures. The measurement of any one or more blood pressure parameters 1st_vst_dbp_2*nd*, 1st_vst_sbp_2*nd*, *1st_vst_map_2nd,* and *2nd_vst_map_2nd* may be particularly useful in panels pertaining to those exemplified in Tables 4 to 11 in the experimental section.

Hypertensive disorders of pregnancy (HDP) include a heterogeneous collection of diseases and conditions associated with hypertension during pregnancy and/or post partum (e.g., up to 12 weeks postpartum).

HDP may be conveniently classified as follows:

I. Hypertension induced by pregnancy

    a. without proteinuria or (generalised) oedema
    b. with proteinuria or (generalised) oedema (i.e., preeclampsia)

        i. mild
        ii. severe

c. eclampsia

II. Coincidental hypertension (chronic hypertension)
III. Hypertension worsened by pregnancy (pregnancy aggravated hypertension)

a. superimposed preeclampsia
b. superimposed eclampsia

Recent studies may no longer classify PE as mild or severe, but may instead identify PE groups based on gestation time, preferably: a. early onset (i.e., clinical manifestation <34 weeks of gestation); b. preterm (i.e., clinical manifestation at <37 weeks of gestation such as for example at >34 and <37 weeks of gestation); c. term (i.e., clinical manifestation ≥37 weeks of gestation).

HPD may otherwise be categorised as pre-existing or gestational, optionally adding "with preeclampsia" to either category if maternal or foetal symptoms, signs or test results necessitate this.

Non-proteinuric hypertension of pregnancy may be conveniently defined as blood pressure of systolic BP≥140 mmHg and/or a diastolic BP≥90 mmHg measured on two separate occasions over 4 hours apart, e.g., about 4 hours to about 168 hours apart. When the hypertension was measured before pregnancy or is measured before 20 weeks of gestation, one may commonly denote such as chronic hypertension. When the hypertension is measured in a previously normotensive woman after 20 weeks of gestation, one may denote such as pregnancy-induced hypertension. Typically, pregnancy-induced hypertension will resolve within 12 weeks postpartum. When blood pressure of at least 140/90 mmHg is measured but does not persist for more than 6 hours, one may denote such as transient hypertension.

Proteinuric hypertension of pregnancy may be as defined in the previous paragraph, further accompanied by ≥300 mg of total protein in a 24-hour urine collection.

HDP also encompasses diseases and conditions commonly denoted in the art as gestational hypertension, mild preeclampsia, pregnancy-induced hypertension, specific hypertension of pregnancy, toxaemia of pregnancy, etc.

The terms "gestational age", "age of gestation" and similar are widespread in the art and commonly denote the time as measured in weeks from the 1st day of a female's last menstrual period. A human pregnancy of normal gestation is between about 38 and 42 weeks, preferably about 40 weeks.

"Preeclampsia" (PE or pre-eclampsia) generally denotes a pregnancy-associated disease or condition characterised by hypertension with proteinuria or oedema or both. PE may also be accompanied by glomerular dysfunction, brain oedema, liver oedema, coagulation abnormalities and/or other complications.

PE may be conveniently defined as some combination of the following signs and symptoms:

(1) a systolic blood pressure (BP)≥140 mmHg and/or a diastolic BP≥90 mmHg after 20 weeks gestation (generally measured on two occasions over 4 hours apart, e.g., about 4 to about 168 hours apart),
(2) new onset proteinuria (1+ by dipstick on urinanalysis, ≥300 mg of protein in a 24-hour urine collection, or a single random urine sample having a protein/creatinine ratio ≥0.3) after 20 weeks gestation, and
(3) resolution of hypertension and proteinuria by 12 weeks postpartum,
such as in particular a combination of hypertension and proteinuria.

Severe PE may be conveniently defined as:

(1) a systolic BP ≥160 mmHg or diastolic BP≥110 mmHg (generally measured on two occasions over 4 hours apart, e.g., about 4 to about 168 hours apart) or
(2) proteinuria characterised by a measurement of ≥3.5 g in a 24-hour urine collection or two random urine specimens with at least 3+ protein by dipstick.

In PE, hypertension and proteinuria generally occur within seven days of each other. In severe PE, severe hypertension, severe proteinuria or HELLP syndrome (haemolysis, elevated liver enzymes, low platelets) or eclampsia can occur simultaneously or only one symptom at a time.

Occasionally, severe PE can lead to the development of seizures, i.e., to eclampsia. Eclampsia can also include dysfunction or damage to several organs or tissues such as the liver (e.g., hepatocellular damage, periportal necrosis) and the central nervous system (e.g., cerebral oedema and cerebral haemorrhage).

Hence, HDP also encompasses diseases and conditions commonly denoted in the art as PE, including *inter alia* mild PE, severe PE and PE with further complications, eclampsia and HELLP syndrome.

The term "pre-term pre-eclampsia" in particular denotes pre-eclampsia that warrants for delivery of the child before 37 weeks of gestation (<37 weeks).

The terms "predicting" or "prediction", "diagnosing" or "diagnosis" and "prognosticating" or "prognosis" are commonplace and well-understood in medical and clinical practice. It shall be understood that the phrase "a method for the diagnosis, prediction and/or prognosis" a given disease or condition may also be interchanged with phrases such as "a method for diagnosing, predicting and/or prognosticating" of said disease or condition or "a method for making (or determining or establishing) the diagnosis, prediction and/or prognosis" of said disease or condition, or the like.

By means of further explanation and without limitation, "predicting" or "prediction" generally refer to an advance declaration, indication or foretelling of a disease or condition in a subject not (yet) having said disease or condition. For example, a prediction of a disease or condition in a subject may indicate a probability, chance or risk that the subject will develop said disease or condition, for example within a certain time period or by a certain age. Said probability, chance or risk may be indicated *inter alia* as an absolute value, range or statistics, or may be indicated relative to a suitable control subject or subject population (such as, e.g., relative to a general, normal or healthy subject or subject population). Hence, the probability, chance or risk that a subject will develop a disease or condition may be advantageously indicated as increased or decreased, or as fold-increased or fold-decreased relative to a suitable control subject or subject population. As used herein, the term "prediction" of the conditions or diseases as taught herein in a subject may also particularly mean that the subject has a 'positive' prediction of such, *i.e.*, that the subject is at risk of having such (*e.g.*, the risk is significantly increased vis-à-vis a control subject or subject population). The term "prediction of no" diseases or conditions as taught herein as described herein in a subject may particularly mean that the subject has a 'negative' prediction of such, *i.e.*, that the subject's risk of having such is not significantly increased vis-à-vis a control subject or subject population.

Preferably, prediction of HDP in particular PE as herein disclosed may take form of "rule-in" tests, whereby panels are employed that can adequately predict the HDP preferably PE without identifying too many false positives. The test is thus designed to have maximum sensitivity for ruling patients into a certain treatment regimen or high risk group. Preferably, the panels as used herein can provide for a Positive Predictive Value (PPV) above or equal to 0.2 (i.e., 20%). Such PPV value is deemed clinically in low prevalence diseases, such as HDP particularly PE,

The terms "diagnosing" or "diagnosis" generally refer to the process or act of recognising, deciding on or concluding on a disease or condition in a subject on the basis of symptoms and signs and/or from results of various diagnostic procedures (such as, for example, from knowing the presence, absence and/or quantity of one or more biomarkers characteristic of the diagnosed disease or condition). As used herein, "diagnosis of" the diseases or conditions as taught herein in a subject may particularly mean that the subject has such, hence, is diagnosed as having such. "Diagnosis of no" diseases or conditions as taught herein in a subject may particularly mean that the subject does not have such, hence, is diagnosed as not having such. A subject may be diagnosed as not having such despite displaying one or more conventional symptoms or signs reminiscent of such.

The terms "prognosticating" or "prognosis" generally refer to an anticipation on the progression of a disease or condition and the prospect (*e.g.*, the probability, duration, and/or extent) of recovery. A good prognosis of the diseases or conditions taught herein may generally encompass anticipation of a satisfactory partial or complete recovery from the diseases or conditions, preferably within an acceptable time period. A good prognosis of such may more commonly encompass anticipation of not further worsening or aggravating of such, preferably within a given time period. A poor prognosis of the diseases or conditions as taught herein may generally encompass anticipation of a substandard recovery and/or unsatisfactorily slow recovery, or to substantially no recovery or even further worsening of such.

Hence, prediction or prognosis of a disease or condition can *inter alia* allow to predict or make a prognosis of the occurrence of the disease or condition, or to predict or make a prognosis of the progression, aggravation, alleviation or recurrence of the disease or condition or response to treatment or to other external or internal factors, situations or stressors, *etc.*

Further, monitoring a disease or condition can *inter alia* allow to predict the occurrence of the disease or condition, or to monitor the progression, aggravation, alleviation or recurrence of the disease or condition, or response to treatment or to other external or internal factors, situations or stressors, *etc.* Advantageously, monitoring may be applied in the course of a medical treatment of a subject, preferably medical treatment aimed at alleviating the so-monitored disease or condition. Such monitoring may be comprised, *e.g.,* in decision making whether a patient may be discharged, needs a change in treatment or needs further hospitalisation. As intended herein, a reference to monitoring of a disease or condition also specifically includes monitoring of the probability, risk or chance of a subject to develop the disease or condition, i.e., monitoring change(s) in said probability, risk or chance over time.

The term "subject" or "patient" as used herein typically denotes humans, but may also encompass reference to non-human animals, preferably warm-blooded animals, more preferably viviparous animals, even more preferably mammals, such as, e.g., non-human primates, rodents, canines, felines, equines, ovines, porcines, and the like. Particularly intended are female subjects, more particularly pregnant or postpartum female subjects. The present test panels, methods and uses may be carried out as from any age of gestation (e.g., from about 5 or from about 8 weeks of gestation) and up to about 12 weeks postpartum (e.g., up to about 6 weeks or about 3 weeks post partum), and preferably between about 10 weeks and about 24 weeks of gestation.

The terms "sample" or "biological sample" as used herein include any biological specimen obtained from a subject. Samples may include, without limitation, whole blood, plasma, serum, red blood cells, white blood cells (*e.g.,* peripheral blood mononuclear cells), saliva, urine, stool (*i.e.*, faeces), tears, sweat, sebum, nipple aspirate, ductal lavage, tumour exudates, synovial fluid, cerebrospinal fluid, lymph, fine needle aspirate, amniotic fluid, any other bodily fluid, nail clippings, cell lysates, cellular secretion products, inflammation fluid, vaginal secretions, or biopsies such as preferably placental biopsies. Preferred samples may include ones comprising any one or more biomarkers as taught herein in detectable quantities. Preferably, the sample may be whole blood or a fractional component thereof such as, *e.g.,* plasma, serum, or a cell pellet. Preferably the sample is readily obtainable by minimally invasive methods, allowing to remove or isolate said sample from the subject. Samples may also include tissue samples and biopsies, tissue homogenates and the like.

Preferably, the sample is blood plasma. The term "plasma" generally denotes the substantially colourless watery fluid of the blood that contains no cells, but in which the blood cells (erythrocytes, leukocytes, thrombocytes, etc.) are normally suspended, containing nutrients, sugars, proteins, minerals, enzymes, etc. Also preferably, said sample may be urine.

Preferably, the sample may be a placental biopsy, which can be taken during pregnancy using known techniques that are not or barely posing a risk for the pregnancy, or can in case of abortion or delivery be taken after the pregnancy is aborted or completed, e.g., for pathological or diagnostic purposes or for acquiring information regarding risk of occurrence of HDP such as PE in a future pregnancy of said subject.

A molecule or analyte such as a metabolite, nucleic acid, RNA, DNA or cDNA, protein, polypeptide or peptide, is "measured" in a sample when the presence or absence and/or quantity of said molecule or analyte or of said group of molecules or analytes is detected or determined in the sample, preferably substantially to the exclusion of other molecules and analytes. For example, a biomarker may be measured by measuring the mRNA encoding the same, or by measuring the encoded protein or polypeptide or a peptide thereof. For example, a metabolite (e.g., blood glucose) may be measured by standard laboratory tests. For example, a chemical element or compound (e.g., selenium) may be measured by standard laboratory tests (e.g., as taught in Rayman et al. 2003, Am J Obstet Gynecol 189: 1343).

A parameter is "scored" or "measured" for or in a patient when the presence or absence and/or quantity of said parameter is detected or determined for or in the subject. For example, a biophysical parameter (e.g., blood pressure) can be measured using standard tests and apparatus. For example, anamnesis parameters (e.g., maternal history parameters such as *fh_petxcardio, father_any_ihd,* and *fh_pet*) may be scored by reviewing relevant medical records or preferably by asking the respective question to a subject under examination and obtaining the answer as a "yes" or "no" (or potentially "unknown") statement.

The terms "quantity", "amount" and "level" are synonymous and generally well-understood in the art. With respect to molecules or analytes, the terms may particularly refer to an absolute quantification of the molecule or analyte in a sample, or to a relative quantification of the molecule or analyte in the sample, *i.e.*, relative to another value such as relative to a reference value as taught herein, or to a range of values indicating a base-line expression of the biomarker. These values or ranges can be obtained from a single patient or from a group of patients.

An absolute quantity of a molecule or analyte in a sample may be advantageously expressed as weight or as molar amount, or more commonly as a concentration, e.g. weight per volume or mol per volume.

A relative quantity of a molecule or analyte in a sample may be advantageously expressed as an increase or decrease or as a fold-increase or fold-decrease relative to said another value, such as relative to a reference value as taught herein. Performing a relative comparison between first and second variables (e.g., first and second quantities) may but need not require to first determine the absolute values of said first and second variables. For example, a measurement method can produce quantifiable readouts (such as, e.g., signal intensities) for said first and second variables, wherein said readouts are a function of the value of said variables, and wherein said readouts can be directly compared to produce a relative value for the first variable vs. the second variable , without the actual need to first convert the readouts to absolute values of the respective variables.

As used herein, the reference to any one biomarker, nucleic acid, peptide, polypeptide or protein corresponds to the biomarker, nucleic acid, peptide, polypeptide or protein commonly known under the respective designations in the art. The terms encompass such markers, nucleic acids, proteins and polypeptides of any organism where found, and particularly of animals, preferably warm-blooded animals, more preferably vertebrates, yet more preferably mammals, including humans and non-human mammals, still more preferably of humans. The terms particularly encompass such biomarkers, nucleic acids, proteins and polypeptides with a native sequence, *i.e.*, ones of which the primary sequence is the same as that of the biomarkers, nucleic acids, proteins and polypeptides found in or derived from nature. A skilled person understands that native sequences may differ between different species due to genetic divergence between such species. Moreover, native sequences may differ between or within different individuals of the same species due to normal genetic diversity (variation) within a given species. Also, native sequences may differ between or even within different individuals of the same species due to post-transcriptional or post-translational modifications. Any such variants or isoforms of biomarkers, nucleic acids, proteins and polypeptides are intended herein. Accordingly, all sequences of biomarkers, nucleic acids, proteins and polypeptides found in or derived from nature are considered "native".

The terms encompass the biomarkers, nucleic acids, proteins and polypeptides when forming a part of a living organism, organ, tissue or cell, when forming a part of a biological sample, as well as when at least partly isolated from such sources. The terms also encompass the biomarkers, nucleic acids, proteins and polypeptides when produced by recombinant or synthetic means.

Exemplary human biomarkers, nucleic acids, proteins or polypeptides as taught herein may be as annotated under NCBI Genbank (http://www.ncbi.nlm.nih.gov/) accession numbers given below. A skilled person can also appreciate that in some instances said sequences may be of precursors (e.g., preproteins) of the of biomarkers, nucleic acids, proteins or polypeptides as taught herein and may include parts which are processed away from the mature biomarkers, nucleic acids, proteins or polypeptides. A skilled person can further appreciate that although only one or more isoforms may be listed below, all isoforms are intended. Unless otherwise specified, the entries below are presented in the form: Name (Code; Genbank accession number for one or more representative amino acid sequences (e.g., isoforms), followed by a period and the Genbank sequence version):

Basement membrane-specific heparan sulfate proteoglycan core protein (HSPG2, NP_005520.4)

Cell surface glycoprotein (MUC18 / MCAM, NP_006491.2)

Collagen alpha-3(VI) chain (COL6A3, NP_004360.2, NP_476505.3, NP_476506.3, NP_476507.3, NP_476508.2)

C-reactive protein (CRP; NP_000558, v.2)

Endoglin (ENG, NP_000109.1, NP_001108225.1)

Hepatocyte growth factor-like protein (MST1; NP_066278, v.3.)

Insulin-like growth factor-binding protein complex acid labile subunit (IGFALS; NP_004961.1)

Intercellular adhesion molecule 3 (ICAM3; NP_002153, v.2)

Kunitz-type protease inhibitor 1 (SPINT1, NP_001027539.1, NP_003701.1, NP_857593.1)

Leucyl-cystinyl aminopeptidase (LNPEP, OTASE, NP_005566.2, NP_787116.2)

Lysosomal Pro-X carboxypeptidase (PRCP, NP_005031.1, NP_955450.2)

Peroxiredoxin 1 (PRDX1; NP_002565, v.1; NP_859047, v.1; NP_859048, v.1)

Peroxiredoxin 2 (PRDX2; NP_005800.3),

Phosphatidylinositol-glycan-specific phospholipase D (GPLD1; NP_001494, v.2; NP_803436, v.1)

Probable G-protein coupled receptor 126 (GPR126; NP_001027566, v.1; NP_001027567, v.1; NP_065188, v.4; NP_940971, v.1)

Quiescin Q6 (QSOX1, NP_001004128.1, NP_002817.2)

Selenoprotein P (SEPP1, NP_001078955.1, NP_001087195.1, NP_005401.3)

Tenascin-X (TNXB; NP_061978, v.6; NP_115859, v.2)

Vascular endothelial growth factor receptor 3 (FLT4 or VGFR3; NP_002011, v.2; NP_891555, v.2)

Disintegrin and metalloproteinase domain-containing protein 12 (ADAM12; NP_003465.3)

Phosphatidylcholine-sterol acyltransferase (LCAT, NP_000220.1)

Roundabout homolog 4 (ROBO4, NP_061928.4)

Ectonucleotide pyrophosphatase/phosphodiesterase family member 2 (ENPP2, NP_001035181.1)

Protein S100-A9 (S100A9, NP_002956.1)

The reference herein to any biomarker, nucleic acid, protein or polypeptide may also encompass fragments thereof. Hence, the reference herein to measuring (or measuring the quantity of) any one biomarker, nucleic acid, protein or polypeptide may encompass measuring the biomarker, nucleic acid, protein or polypeptide, such as, e.g., measuring the mature and/or the processed soluble/secreted form (e.g. plasma circulating form) thereof and/or measuring one or more fragments thereof.

For example, any biomarker, nucleic acid, protein or polypeptide and/or one or more fragments thereof may be measured collectively, such that the measured quantity corresponds to the sum amounts of the collectively measured species. In another example, any biomarker, nucleic acid, protein or polypeptide and/or one or more fragments thereof may be measured each individually. Preferably, said fragment may be a plasma circulating (i.e., not cell- or membrane-bound) form. Without being bound by any theory, such circulating forms can be derived from full-length biomarkers, nucleic acids, proteins or polypeptides through natural processing, or can be resulting from known degradation processes occurring in a sample. In certain situations, the circulating form can also be the full-length biomarker, nucleic acid, protein or polypeptide, which is found to be circulating in the plasma. Said "circulating form" can thus be any biomarker, nucleic acid, protein or polypeptide or any processed soluble form thereof or fragments of either one, that is circulating in the sample, i.e. which is not bound to a cell- or membrane fraction of said sample.

Unless otherwise apparent from the context, reference herein to any biomarker, nucleic acid, protein or polypeptide and fragments thereof may generally also encompass modified forms of said biomarker, nucleic acid, protein or polypeptide and fragments such as bearing post-expression modifications including, for example, phosphorylation, glycosylation, lipidation, methylation, cysteinylation, sulphonation, glutathionylation, acetylation, oxidation of methionine to methionine sulphoxide or methionine sulphone, and the like.

Preferably, any biomarker, nucleic acid, protein or polypeptide and fragments thereof may be human, i.e., their primary sequence may be the same as a corresponding primary sequence of or present in a naturally occurring human biomarker, nucleic acid, protein or polypeptide. Hence, the qualifier "human" in this connection relates to the primary sequence of the respective biomarker, nucleic acid, protein or polypeptide, rather than to its origin or source. For example, such biomarker, nucleic acid, protein or polypeptide and fragments may be present in or isolated from samples of human subjects or may be obtained by other means (e.g., by recombinant expression, cell-free translation or non-biological peptide synthesis).

The term "fragment" of a protein, polypeptide or peptide generally refers to N-terminally and/or C-terminally deleted or truncated forms of said protein, polypeptide or peptide. The term encompasses fragments arising by any mechanism, such as, without limitation, by alternative translation, exo- and/or endo-proteolysis and/or degradation of said peptide, polypeptide or protein, such as, for example, in vivo or in vitro, such as, for example, by physical, chemical and/or enzymatic proteolysis. Without limitation, a fragment of a protein, polypeptide or peptide may represent at least about 5%, or at least about 10%, e.g., $\geq 20\%$, $\geq 30\%$ or $\geq 40\%$, such as $\geq 50\%$, e.g., $\geq 60\%$, $\geq 70\%$ or $\geq 80\%$, or even $\geq 90\%$ or $\geq 95\%$ of the amino acid sequence of said protein, polypeptide or peptide.

For example, a fragment may include a sequence of $\geq 5$ consecutive amino acids, or $\geq 10$ consecutive amino acids, or $\geq 20$ consecutive amino acids, or $\geq 30$ consecutive amino acids, e.g., $\geq 40$ consecutive amino acids, such as for example $\geq 50$ consecutive amino acids, e.g., $\geq 60$, $\geq 70$, $\geq 80$, $\geq 90$, $\geq 100$, $\geq 200$, $\geq 300$, $\geq 400$, $\geq 500$ or $\geq 600$ consecutive amino acids of the corresponding full length protein.

Preferably, a fragment may be N-terminally and/or C-terminally truncated by between 1 and about 20 amino acids, such as, e.g., by between 1 and about 15 amino acids, or by between 1 and about 10 amino acids, or by between 1 and about 5 amino acids, compared to the corresponding mature, full-length protein or its soluble or plasma circulating form.

Preferably, fragments of a given protein, polypeptide or peptide may be achieved by in vitro proteolysis of said protein, polypeptide or peptide to obtain advantageously detectable peptide(s) from a sample. For example, such proteolysis may be effected by suitable physical, chemical and/or enzymatic agents, e.g., proteinases, preferably endoproteinases, i.e., protease cleaving internally within a protein, polypeptide or peptide chain. A non-limiting list of suitable endoproteinases includes serine proteinases (EC 3.4.21), threonine proteinases (EC 3.4.25), cysteine proteinases (EC 3.4.22), aspartic acid proteinases (EC 3.4.23), metalloproteinases (EC 3.4.24) and glutamic acid proteinases. Exemplary non-limiting endoproteinases include trypsin, chymotrypsin, elastase, Lysobacter enzymogenes endoproteinase Lys-C, Staphylococcus aureus endoproteinase Glu-C (endopeptidase V8) or Clostridium histolyticum endoproteinase Arg-C (clostripain). Further known or yet to be identified enzymes may be used; a skilled person can choose suitable protease(s) on the basis of their cleavage specificity and frequency to achieve desired peptide forms. Preferably, the proteolysis may be effected by endopeptidases of the trypsin type (EC 3.4.21.4), preferably trypsin, such as, without limitation, preparations of trypsin from bovine pancreas, human pancreas, porcine pancreas, recombinant trypsin, Lys-acetylated trypsin, trypsin in solution, trypsin immobilised to a solid support, *etc.* Trypsin is particularly useful, *inter alia* due to high specificity and efficiency of cleavage. It is further herein disclosed the use of any trypsin-like protease, *i.e.*, with a similar specificity to that of trypsin. Otherwise, chemical reagents may be used for proteolysis. For example, CNBr can cleave at Met; BNPS-skatole can cleave at Trp. The conditions for treatment, e.g., protein concentration, enzyme or chemical reagent concentration, pH, buffer, temperature, time, can be determined by the skilled person depending on the enzyme or chemical reagent employed.

The term "isolated" with reference to a particular component (such as for instance, nucleic acid, protein, polypeptide, peptide or fragment thereof) generally denotes that such component exists in separation from - for example, has been separated from or prepared in separation from - one or more other components of its natural environment. For instance, an isolated human or animal nucleic acid, protein, polypeptide, peptide or fragment exists in separation from a human or animal body where it occurs naturally.

The term "isolated" as used herein may preferably also encompass the qualifier "purified". As used herein, the term "purified" with reference to nucleic acid(s), protein(s), polypeptide(s), peptide(s) and/or fragment(s) thereof does not require absolute purity. Instead, it denotes that such nucleic acid(s), protein(s), polypeptide(s), peptide(s) and/or fragment(s) is (are) in a discrete environment in which their abundance (conveniently expressed in terms of mass or weight or concentration) relative to other proteins is greater than in a biological sample. A discrete environment denotes a single medium, such as for example a single solution, gel, precipitate, lyophilisate, *etc.* Purified nucleic acids, peptides, polypeptides or fragments may be obtained by known methods including, for example, laboratory or recombinant synthesis, chromatography, preparative electrophoresis, centrifugation, precipitation, affinity purification, *etc.*

Purified protein(s), polypeptide(s), peptide(s) and/or fragment(s) may preferably constitute by weight $\geq$ 10%, more preferably $\geq$ 50%, such as $\geq$ 60%, yet more preferably $\geq$ 70%, such as $\geq$ 80%, and still more preferably $\geq$ 90%, such as $\geq$ 95%, $\geq$ 96%, $\geq$ 97%, $\geq$ 98%, $\geq$ 99% or even 100%, of the protein content of the discrete environment. Protein content may be determined, e.g., by the Lowry method (Lowry et al. 1951. J Biol Chem 193: 265), optionally as described by Hartree 1972 (Anal Biochem 48: 422-427). Also, purity of peptides or polypeptides may be determined by SDS-PAGE under reducing or nonreducing conditions using Coomassie blue or, preferably, silver stain.

Preferably, reagents disclosed herein may comprise a detectable label. The term "label" refers to any atom, molecule, moiety or biomolecule that can be used to provide a detectable and preferably quantifiable read-out or property, and that can be attached to or made part of an entity of interest, such as a peptide or polypeptide or a specific-binding agent. Labels may be suitably detectable by mass spectrometric, spectroscopic, optical, colourimetric, magnetic, photochemical, biochemical, immunochemical or chemical means. Labels include without limitation dyes; radiolabels such as $^{32}$P, $^{33}$P, $^{35}$S, $^{125}$I, $^{131}$I; electron-dense reagents; enzymes (e.g. , horse-radish phosphatise or alkaline phosphatise as commonly used in immunoassays); binding moieties such as biotin-streptavidin; haptens such as digoxigenin; luminogenic, phosphorescent or fluorogenic moieties; mass tags; and fluorescent dyes alone or in combination with moieties that can suppress or shift emission spectra by fluorescence resonance energy transfer (FRET).

For example, the label may be a mass-altering label. Preferably, a mass-altering label may involve the presence of a distinct stable isotope in one or more amino acids of the peptide vis-à-vis its corresponding non-labelled peptide. Mass-labelled peptides are particularly useful as positive controls, standards and calibrators in mass spectrometry applications. In particular, peptides including one or more distinct isotopes are chemically alike, separate chromatographically and electrophoretically in the same manner and also ionise and fragment in the same way. However, in a suitable mass analyser such peptides and optionally select fragmentation ions thereof will display distinguishable m/z ratios and can thus be discriminated. Examples of pairs of distinguishable stable isotopes include H and D, $^{12}$C and $^{13}$C, $^{14}$N and $^{15}$N or $^{16}$O and $^{18}$O. Usually, peptides and proteins of biological samples analysed in the present invention may substantially only contain common isotopes having high prevalence in nature, such as for example H, $^{12}$C, $^{14}$N and $^{16}$O. In such case, the mass-labelled peptide may be labelled with one or more uncommon isotopes having low prevalence in nature, such as for instance D, $^{13}$C, $^{15}$N and/or $^{18}$O. It is also conceivable that in cases where the peptides or proteins of a biological sample would include one or more uncommon isotopes, the mass-labelled peptide may comprise the respective common isotope(s).

Isotopically-labelled synthetic peptides may be obtained *inter alia* by synthesising or recombinantly producing such peptides using one or more isotopically-labelled amino acid substrates, or by chemically or enzymatically modifying unlabelled peptides to introduce thereto one or more distinct isotopes. By means of example and not limitation, D-labelled peptides may be synthesised or recombinantly produced in the presence of commercially available deuterated L-methionine $CH_3$-S-$CD_2CD_2$-CH($NH_2$)-COOH or deuterated arginine $H_2$NC(=NH)-NH-$(CD_2)_3$-CD($NH_2$)-COOH. It shall be appreciated that any amino acid of which deuterated or $^{15}$N- or $^{13}$C-containing forms exist may be considered for synthesis or recombinant production of labelled peptides. In another non-limiting example, a peptide may be treated with trypsin in $H_2^{16}$O or $H_2^{18}$O, leading to incorporation of two oxygens ($^{16}$O or $^{18}$O, respectively) at the COOH-termini of said peptide (*e.g.,* US 2006/105415).

Also contemplated is the use of biomarkers, peptides, polypeptides or proteins and fragments thereof as taught herein, optionally comprising a detectable label, as (positive) controls, standards or calibators in qualitative or quantitative detection assays (measurement methods) of said biomarkers, peptides, polypeptides or proteins and fragments thereof, and particularly in such methods for the diagnosis, prediction, prognosis and/or monitoring the diseases or conditions as taught herein in subjects. The biomarkers, proteins, polypeptides or peptides may be supplied in any form, *inter alia* as precipitate, vacuum-dried, lyophilisate, in solution as liquid or frozen, or covalently or non-covalently immobilised on solid phase, such as for example, on solid chromatographic matrix or on glass or plastic or other suitable surfaces (*e.g.,* as a part of peptide arrays and microarrays). The peptides may be readily prepared, for example, isolated from natural sources, or prepared recombinantly or synthetically.

Further disclosed are binding agents capable of specifically binding to biomarkers, peptides, polypeptides or proteins and fragments thereof as taught herein. Binding agents as intended throughout this specification may include *inter alia* an antibody, aptamer, photoaptamer, protein, peptide, peptidomimetic or a small molecule.

The term "specifically bind" as used throughout this specification means that an agent (denoted herein also as "specific-binding agent") binds to one or more desired molecules or analytes substantially to the exclusion of other molecules which are random or unrelated, and optionally substantially to the exclusion of other molecules that are structurally related. The term "specifically bind" does not necessarily require that an agent binds exclusively to its intended target(s). For example, an agent may be said to specifically bind to target(s) of interest if its affinity for such intended target(s) under the conditions of binding is at least about 2-fold greater, preferably at least about 5-fold greater, more preferably at least about 10-fold greater, yet more preferably at least about 25-fold greater, still more preferably at least about 50-fold greater, and even more preferably at least about 100-fold or more greater, than its affinity for a non-target molecule.

Preferably, the agent may bind to its intended target(s) with affinity constant ($K_A$) of such binding $K_A \geq 1 \times 10^6$ $M^{-1}$, more preferably $K_A \geq 1 \times 10^7$ $M^{-1}$, yet more preferably $K_A \geq 1 \times 10^8$ $M^{-1}$, even more preferably $K_A \geq 1 \times 10^9$ $M^{-1}$, and still more preferably $K_A \geq 1 \times 10^{10}$ $M^{-1}$ or $K_A \geq 1 \times 10^{11}$ $M^{-1}$, wherein $K_A = [SBA\_T]/[SBA][T]$, SBA denotes the specific-binding agent, T denotes the intended target. Determination of $K_A$ can be carried out by methods known in the art, such as for example, using equilibrium dialysis and Scatchard plot analysis.

As used herein, the term "antibody" is used in its broadest sense and generally refers to any immunologic binding agent. The term specifically encompasses intact monoclonal antibodies, polyclonal antibodies, multivalent (*e.g.,* 2-, 3- or more-valent) and/or multi-specific antibodies (*e.g.,* bi- or more-specific antibodies) formed from at least two intact antibodies, and antibody fragments insofar they exhibit the desired biological activity (particularly, ability to specifically bind an antigen of interest), as well as multivalent and/or multi-specific composites of such fragments. The term "antibody" is not only inclusive of antibodies generated by methods comprising immunisation, but also includes any polypeptide, e.g., a recombinantly expressed polypeptide, which is made to encompass at least one complementarity-determining region (CDR) capable of specifically binding to an epitope on an antigen of interest. Hence, the term applies to such molecules regardless whether they are produced in vitro or in vivo.

An antibody may be any of IgA, IgD, IgE, IgG and IgM classes, and preferably IgG class antibody. An antibody may be a polyclonal antibody, e.g., an antiserum or immunoglobulins purified there from (e.g., affinity-purified). An antibody may be a monoclonal antibody or a mixture of monoclonal antibodies. Monoclonal antibodies can target a particular antigen or a particular epitope within an antigen with greater selectivity and reproducibility. By means of example and not limitation, monoclonal antibodies may be made by the hybridoma method first described by Kohler et al. 1975 (Nature 256: 495), or may be made by recombinant DNA methods (e.g., as in US 4,816,567). Monoclonal antibodies may also be isolated from phage antibody libraries using techniques as described by Clackson et al. 1991 (Nature 352: 624-628) and Marks et al. 1991 (J Mol Biol 222: 581-597), for example.

Antibody binding agents may be antibody fragments. "Antibody fragments" comprise a portion of an intact antibody, comprising the antigen-binding or variable region thereof. Examples of antibody fragments include Fab, Fab', F(ab')2, Fv and scFv fragments; diabodies; linear antibodies; single-chain antibody molecules; and multivalent and/or multispecific antibodies formed from antibody fragment(s), *e.g.,* dibodies, tribodies, and multibodies. The above designations Fab, Fab', F(ab')2, Fv, scFv *etc.* are intended to have their art-established meaning.

The term antibody includes antibodies originating from or comprising one or more portions derived from any animal species, preferably vertebrate species, including, e.g., birds and mammals. Without limitation, the antibodies may be chicken, turkey, goose, duck, guinea fowl, quail or pheasant. Also without limitation, the antibodies may be human, murine (e.g., mouse, rat, etc.), donkey, rabbit, goat, sheep, guinea pig, camel (e.g., *Camelus bactrianus* and *Camelus dromaderius*), llama (e.g., *Lama paccos, Lama glama* or *Lama vicugna*) or horse.

A skilled person will understand that an antibody can include one or more amino acid deletions, additions and/or substitutions (e.g., conservative substitutions), insofar such alterations preserve its binding of the respective antigen. An antibody may also include one or more native or artificial modifications of its constituent amino acid residues (e.g., glycosylation, etc.).

Methods of producing polyclonal and monoclonal antibodies as well as fragments thereof are well known in the art, as are methods to produce recombinant antibodies or fragments thereof (see for example, Harlow and Lane, "Antibodies: A Laboratory Manual", Cold Spring Harbour Laboratory, New York, 1988; Harlow and Lane, "Using Antibodies: A Laboratory Manual", Cold Spring Harbour Laboratory, New York, 1999, ISBN 0879695447; "Monoclonal Antibodies: A Manual of Techniques", by Zola, ed., CRC Press 1987, ISBN 0849364760; "Monoclonal Antibodies: A Practical Approach", by Dean & Shepherd, eds., Oxford University Press 2000, ISBN 0199637229; Methods in Molecular Biology, vol. 248: "Antibody Engineering: Methods and Protocols", Lo, ed., Humana Press 2004, ISBN 1588290921).

The term "aptamer" refers to single-stranded or double-stranded oligo-DNA, oligo-RNA or oligo-DNA/RNA or any analogue thereof, that can specifically bind to a target molecule such as a peptide. Advantageously, aptamers can display fairly high specificity and affinity (*e.g.,* $K_A$ in the order $1 \times 10^9 \, M^{-1}$) for their targets. Aptamer production is described *inter alia* in US 5,270,163; Ellington & Szostak 1990 (Nature 346: 818-822); Tuerk & Gold 1990 (Science 249: 505-510); or "The Aptamer Handbook: Functional Oligonucleotides and Their Applications", by Klussmann, ed., Wiley-VCH 2006, ISBN 3527310592. The term "photoaptamer" refers to an aptamer that contains one or more photoreactive functional groups that can covalently bind to or crosslink with a target molecule. The term "peptidomimetic" refers to a non-peptide agent that is a topological analogue of a corresponding peptide. Methods of rationally designing peptidomimetics of peptides are known in the art. For example, the rational design of three peptidomimetics based on the sulphated 8-mer peptide CCK26-33, and of two peptidomimetics based on the 11-mer peptide Substance P, and related peptidomimetic design principles, are described in Horwell 1995 (Trends Biotechnol 13: 132-134).

The term "small molecule" refers to compounds, preferably organic compounds, with a size comparable to those organic molecules generally used in pharmaceuticals. The term excludes biological macromolecules (*e.g.,* proteins, nucleic acids, etc.). Preferred small organic molecules range in size up to about 5000 Da, *e.g.,* up to about 4000, preferably up to 3000 Da, more preferably up to 2000 Da, even more preferably up to about 1000 Da, *e.g.,* up to about 900, 800, 700, 600 or up to about 500 Da.

Hence, also disclosed are methods for immunising animals, e.g., non-human animals such as laboratory or farm, animals using (i.e., using as the immunising antigen) any one or more (isolated) markers, peptides, polypeptides or proteins and fragments thereof as taught herein, optionally attached to a presenting carrier. Immunisation and preparation of antibody reagents from immune sera is well-known *per se* and described in documents referred to elsewhere in this specification. The animals to be immunised may include any animal species, preferably warm-blooded species, more preferably vertebrate species, including, *e.g.,* birds, fish, and mammals. Without limitation, the antibodies may be chicken, turkey, goose, duck, guinea fowl, shark, quail or pheasant. Also without limitation, the antibodies may be human, murine (e.g., mouse, rat, etc.), donkey, rabbit, goat, sheep, guinea pig, shark, camel, llama or horse. The term "presenting carrier" or "carrier" generally denotes an immunogenic molecule which, when bound to a second molecule, augments immune responses to the latter, usually through the provision of additional T cell epitopes. The presenting carrier may be a (poly)peptidic structure or a non-peptidic structure, such as *inter alia* glycans, polyethylene glycols, peptide mimetics, synthetic polymers, etc. Exemplary non-limiting carriers include human Hepatitis B virus core protein, multiple C3d domains, tetanus toxin fragment C or yeast Ty particles.

Immune sera obtained or obtainable by immunisation as taught herein may be particularly useful for generating antibody reagents that specifically bind to any one or more biomarkers, peptides, polypeptides or proteins and fragments thereof disclosed herein.

The binding molecule may labelled with a tag that permits detection with another agent (*e.g.* with a probe binding partner). Such tags can be, for example, biotin, streptavidin, histag, myc tag, maltose, maltose binding protein or any other kind of tag known in the art that has a binding partner. Example of associations which can be utilised in the probe:binding partner arrangement may be any, and includes, for example biotin:streptavidin, his-tag:metal ion (*e.g.* $Ni^{2+}$), maltose:maltose binding protein.

The binding molecule conjugate may be associated with or attached to a detection agent to facilitate detection. Examples of lab detection agents include, but are not limited to, luminescent labels; colourimetric labels, such as dyes; fluorescent labels; or chemical labels, such as electroactive agents (*e.g.,* ferrocyanide); enzymes; radioactive labels; or radiofrequency labels. More commonly, the detection agent is a particle. Examples of particles useful in the practice of the invention include, but are not limited to, colloidal gold particles; colloidal sulphur particles; colloidal selenium particles; colloidal barium sulfate particles; colloidal iron sulfate particles; metal iodate particles; silver halide particles; silica particles; colloidal metal (hydrous) oxide particles; colloidal metal sulfide particles; colloidal lead selenide particles; colloidal cadmium selenide particles; colloidal metal phosphate particles; colloidal metal ferrite particles; any of the above-mentioned colloidal particles coated with organic or inorganic layers; protein or peptide molecules; liposomes; or organic polymer latex particles, such as polystyrene latex beads. Preferable particles are colloidal gold particles. Colloidal gold may be made by any conventional means, such as the methods outlined in G. Frens, 1973 Nature Physical Science, 241:20 (1973). Alternative methods may be described in U.S. Pat. Nos. 5,578,577, 5,141,850; 4,775,636; 4,853,335; 4,859,612; 5,079,172; 5,202,267; 5,514,602; 5,616,467; 5,681,775.

Any existing, available or conventional separation, detection and quantification methods can be used herein to measure the presence or absence (*e.g.,* readout being present vs. absent; or detectable amount vs. undetectable amount) and/or quantity (*e.g.,* readout being an absolute or relative quantity, such as, for example, absolute or relative concentration) of biomarkers, peptides, polypeptides, proteins and/or fragments thereof in samples (any molecules or analytes of interest to be so-measured in samples, including any one or more biomarkers, peptides, polypeptides, proteins and fragments thereof as taught herein, may be herein below referred to collectively as biomarkers).

For example, such methods may include biochemical assay methods, immunoassay methods, mass spectrometry analysis methods, or chromatography methods, or combinations thereof.

The term "immunoassay" generally refers to methods known as such for detecting one or more molecules or analytes of interest in a sample, wherein specificity of an immunoassay for the molecule(s) or analyte(s) of interest is conferred by specific binding between a specific-binding agent, commonly an antibody, and the molecule(s) or analyte(s) of interest. Immunoassay technologies include without limitation direct ELISA (enzyme-linked immunosorbent assay), indirect ELISA, sandwich ELISA, competitive ELISA, multiplex ELISA, radioimmunoassay (RIA), ELISPOT technologies, and other similar techniques known in the art. Principles of these immunoassay methods are known in the art, for example John R. Crowther, "The ELISA Guidebook", 1 st ed., Humana Press 2000, ISBN 0896037282.

By means of further explanation and not limitation, direct ELISA employs a labelled primary antibody to bind to and thereby quantify target antigen in a sample immobilised on a solid support such as a microwell plate. Indirect ELISA uses a non-labelled primary antibody which binds to the target antigen and a secondary labelled antibody that recognises and allows to quantify the antigen-bound primary antibody. In sandwich ELISA the target antigen is captured from a sample using an immobilised 'capture' antibody which binds to one antigenic site within the antigen, and subsequent to removal of non-bound analytes the so-captured antigen is detected using a 'detection' antibody which binds to another antigenic site within said antigen, where the detection antibody may be directly labelled or indirectly detectable as above. Competitive ELISA uses a labelled 'competitor' that may either be the primary antibody or the target antigen. In an example, non-labelled immobilised primary antibody is incubated with a sample, this reaction is allowed to reach equilibrium, and then labelled target antigen is added. The latter will bind to the primary antibody wherever its binding sites are not yet occupied by non-labelled target antigen from the sample. Thus, the detected amount of bound labelled antigen inversely correlates with the amount of non-labelled antigen in the sample. Multiplex ELISA allows simultaneous detection of two or more analytes within a single compartment (e.g., microplate well) usually at a plurality of array addresses (see, for example, Nielsen & Geierstanger 2004. J Immunol Methods 290: 107-20 and Ling et al. 2007. Expert Rev Mol Diagn 7: 87-98 for further guidance). As appreciated, labelling in ELISA technologies is usually by enzyme (such as, e.g., horse-radish peroxidase) conjugation and the end-point is typically colourimetric, chemiluminescent or fluorescent, magnetic, piezo electric, pyroelectric and other.

Radioimmunoassay (RIA) is a competition-based technique and involves mixing known quantities of radioactively-labelled (e.g., $^{125}$I- or $^{131}$I-labelled) target antigen with antibody to said antigen, then adding non-labelled or 'cold' antigen from a sample and measuring the amount of labelled antigen displaced (see, e.g., "An Introduction to Radioimmunoassay and Related Techniques", by Chard T, ed., Elsevier Science 1995, ISBN 0444821198 for guidance).

Generally, any mass spectrometric (MS) techniques that can obtain precise information on the mass of peptides, and preferably also on fragmentation and/or (partial) amino acid sequence of selected peptides (*e.g.,* in tandem mass spectrometry, MS/MS; or in post source decay, TOF MS), are useful herein. Suitable peptide MS and MS/MS techniques and systems are well-known *per se* (see, *e.g.,* Methods in Molecular Biology, vol. 146: "Mass Spectrometry of Proteins and Peptides", by Chapman, ed., Humana Press 2000, ISBN 089603609x; Biemann 1990. Methods Enzymol 193: 455-79; or Methods in Enzymology, vol. 402: "Biological Mass Spectrometry", by Burlingame, ed., Academic Press 2005, ISBN 9780121828073) and may be used herein. MS arrangements, instruments and systems suitable for biomarker peptide analysis may include, without limitation, matrix-assisted laser desorption/ionisation time-of-flight (MALDI-TOF) MS; MALDI-TOF post-source-decay (PSD); MALDI-TOF/TOF; surface-enhanced laser desorption/ionization time-of-flight mass spectrometry (SELDI-TOF) MS; electrospray ionization mass spectrometry (ESI-MS); ESI-MS/MS; ESI-MS/(MS)$^n$ (n is an integer greater than zero); ESI 3D or linear (2D) ion trap MS; ESI triple quadrupole MS; ESI quadrupole orthogonal TOF (Q-TOF); ESI Fourier transform MS systems; desorption/ionization on silicon (DIOS); secondary ion mass spectrometry (SIMS); atmospheric pressure chemical ionization mass spectrometry (APCI-MS); APCI-MS/MS; APCI- (MS)$^n$; atmospheric pressure photoionization mass spectrometry (APPI-MS); APPI-MS/MS; and APPI- (MS)$^n$. Peptide ion fragmentation in tandem MS (MS/MS) arrangements may be achieved using manners established in the art, such as, *e.g.,* collision induced dissociation (CID). Detection and quantification of biomarkers by mass spectrometry may involve multiple reaction monitoring (MRM), such as described among others by Kuhn et al. 2004 (Proteomics 4: 1175-86). MS peptide analysis methods may be advantageously combined with upstream peptide or protein separation or fractionation methods, such as for example with the chromatographic and other methods described herein below.

Chromatography can also be used for measuring biomarkers. As used herein, the term "chromatography" encompasses methods for separating chemical substances, referred to as such and vastly available in the art. In a preferred approach, chromatography refers to a process in which a mixture of chemical substances (analytes) carried by a moving stream of liquid or gas ("mobile phase") is separated into components as a result of differential distribution of the analytes, as they flow around or over a stationary liquid or solid phase ("stationary phase"), between said mobile phase and said stationary phase. The stationary phase may be usually a finely divided solid, a sheet of filter material, or a thin film of a liquid on the surface of a solid, or the like. Chromatography is also widely applicable for the separation of chemical compounds of biological origin, such as, e.g., amino acids, proteins, fragments of proteins or peptides, *etc.*

Chromatography as used herein may be preferably columnar (*i.e.*, wherein the stationary phase is deposited or packed in a column), preferably liquid chromatography, and yet more preferably HPLC. While particulars of chromatography are well known in the art, for further guidance see, *e.g.,* Meyer M., 1998, ISBN: 047198373X, and "Practical HPLC Methodology and Applications", Bidlingmeyer, B. A., John Wiley & Sons Inc., 1993. Exemplary types of chromatography include, without limitation, high-performance liquid chromatography (HPLC), normal phase HPLC (NP-HPLC), reversed phase HPLC (RP-HPLC), ion exchange chromatography (IEC), such as cation or anion exchange chromatography, hydrophilic interaction chromatography (HILIC), hydrophobic interaction chromatography (HIC), size exclusion chromatography (SEC) including gel filtration chromatography or gel permeation chromatography, chromatofocusing, affinity chromatography such as immuno-affinity, immobilised metal affinity chromatography, and the like.

Chromatography, including single-, two- or more-dimensional chromatography, may be used as a peptide fractionation method in conjunction with a further peptide analysis method, such as for example, with a downstream mass spectrometry analysis as described elsewhere in this specification.

Further peptide or polypeptide separation, identification or quantification methods may be used, optionally in conjunction with any of the above described analysis methods, for measuring biomarkers in the present disclosure. Such methods include, without limitation, chemical extraction partitioning, isoelectric focusing (IEF) including capillary isoelectric focusing (CIEF), capillary isotachophoresis (CITP), capillary electrochromatography (CEC), and the like, one-dimensional polyacrylamide gel electrophoresis (PAGE), two-dimensional polyacrylamide gel electrophoresis (2D-PAGE), capillary gel electrophoresis (CGE), capillary zone electrophoresis (CZE), micellar electrokinetic chromatography (MEKC), free flow electrophoresis (FFE), etc.

The level of biomarkers at the RNA level may be established using RNA analysis of placental tissue obtained e.g. using transcervical placental biopsy during early pregnancy or similar methods not endangering the pregnancy. This test involves the removal of a small amount of placental tissue between the tenth and twelfth week of pregnancy. Under ultrasound guidance via the vagina, a narrow tube is inserted into the placenta and a small biopsy is taken. Alternatively, the placental biopsy can be obtained from subjects with natural abortion of the pregnancy in order to establish the cause of said premature abortion. This information is an important predictive tool in view of future pregnancies.

The RNA level can be detected using standard quantitative RNA measurement tools known in the art. Non-limiting examples include hybridization-based analysis, microarray expression analysis, digital gene expression (DGE), RNA-in-situ hybridization (RISH), Northern-blot analysis and the like; PCR, RT-PCR, RT-qPCR, end-point PCR, digital PCR or the like; supported oligonucleotide detection, pyrosequencing, polony cyclic sequencing by synthesis, simultaneous bi-directional sequencing, single-molecule sequencing, single molecule real time sequencing, true single molecule sequencing, hybridization-assisted nanopore sequencing and sequencing by synthesis.

Biomarker presence can also be detected on placental biopsies obtained as indicated above using standard immunohistochemistry techniques, wherein the presence, absence, or quantity of biomarker proteins is detected directly in the placental tissue. The bioptic tissue can be fixed following routine procedures well known in the art.

The various aspects taught herein may further rely on comparing the quantity of biomarkers measured in samples and the measurement or score of parameters in patients with reference values, wherein said reference values represent known predictions, diagnoses and/or prognoses of diseases or conditions as taught herein.

For example, distinct reference values may represent the prediction of a risk (*e.g.,* an abnormally elevated risk) of having a given disease or condition as taught herein vs. the prediction of no or normal risk of having said disease or condition. In another example, distinct reference values may represent predictions of differing degrees of risk of having such disease or condition.

In a further example, distinct reference values can represent the diagnosis of a given disease or condition as taught herein vs. the diagnosis of no such disease or condition (such as, e.g., the diagnosis of healthy, or recovered from said disease or condition, *etc.*). In another example, distinct reference values may represent the diagnosis of such disease or condition of varying severity.

In yet another example, distinct reference values may represent a good prognosis for a given disease or condition as taught herein vs. a poor prognosis for said disease or condition. In a further example, distinct reference values may represent varyingly favourable or unfavourable prognoses for such disease or condition.

Such comparison may generally include any means to determine the presence or absence of at least one difference and optionally of the size of such difference between values being compared. A comparison may include a visual inspection, an arithmetical or statistical comparison of measurements. Such statistical comparisons include, but are not limited to, applying a rule.

Reference values may be established according to known procedures previously employed for other biomarkers and parameters. For example, a reference value may be established in an individual or a population of individuals characterised by a particular diagnosis, prediction and/or prognosis of said disease or condition (*i.e.*, for whom said diagnosis, prediction and/or prognosis of the disease or condition holds true). Such population may comprise without limitation ≥ 2, ≥ 10, ≥ 100, or even several hundreds or more individuals.

A "deviation" of a first value from a second value may generally encompass any direction (*e.g.,* increase: first value > second value; or decrease: first value < second value) and any extent of alteration.

For example, a deviation may encompass a decrease in a first value by, without limitation, at least about 10% (about 0.9-fold or less), or by at least about 20% (about 0.8-fold or less), or by at least about 30% (about 0.7-fold or less), or by at least about 40% (about 0.6-fold or less), or by at least about 50% (about 0.5-fold or less), or by at least about 60% (about 0.4-fold or less), or by at least about 70% (about 0.3-fold or less), or by at least about 80% (about 0.2-fold or less), or by at least about 90% (about 0.1-fold or less), relative to a second value with which a comparison is being made.

For example, a deviation may encompass an increase of a first value by, without limitation, at least about 10% (about 1.1-fold or more), or by at least about 20% (about 1.2-fold or more), or by at least about 30% (about 1.3-fold or more), or by at least about 40% (about 1.4-fold or more), or by at least about 50% (about 1.5-fold or more), or by at least about 60% (about 1.6-fold or more), or by at least about 70% (about 1.7-fold or more), or by at least about 80% (about 1.8-fold or more), or by at least about 90% (about 1.9-fold or more), or by at least about 100% (about 2-fold or more), or by at least about 150% (about 2.5-fold or more), or by at least about 200% (about 3-fold or more), or by at least about 500% (about 6-fold or more), or by at least about 700% (about 8-fold or more), or like, relative to a second value with which a comparison is being made.

Preferably, a deviation may refer to a statistically significant observed alteration. For example, a deviation may refer to an observed alteration which falls outside of error margins of reference values in a given population (as expressed, for example, by standard deviation or standard error, or by a predetermined multiple thereof, e.g., $\pm1\times SD$ or $\pm2\times SD$, or $\pm1\times SE$ or $\pm2\times SE$). Deviation may also refer to a value falling outside of a reference range defined by values in a given population (for example, outside of a range which comprises ≥40%, ≥50%, ≥60%, ≥70%, ≥75% or ≥80% or ≥85% or ≥90% or ≥95% or even ≥100% of values in said population).

Preferably, a deviation may be concluded if an observed alteration is beyond a given threshold or cut-off. Such threshold or cut-off may be selected as generally known in the art to provide for a chosen sensitivity and/or specificity of the diagnosis, prediction and/or prognosis methods, *e.g.,* sensitivity and/or specificity of at least 50%, or at least 60%, or at least 70%, or at least 80%, or at least 85%, or at least 90%, or at least 95%.

Also disclosed are kits or devices as set forth above for the diagnosis, prediction, prognosis and/or monitoring of any one disease or condition as taught herein comprising means for detecting the level of biomarker(s) comprised in test panels as taught herein in a sample of the patient. Preferably, such a kit or kits can be used in clinical settings or at home. The kit can be used for diagnosing said disease or condition, for monitoring the effectiveness of treatment of a subject suffering from said disease or condition with an agent, or for preventive screening of subjects for the occurrence of said disease or condition in said subject.

In a clinical setting, the kit or device can be in the form of a bed-side device or in an emergency team setting, e.g. as part of the equipment of an ambulance or other moving emergency vehicle or team equipment or as part of a first-aid kit. The diagnostic kit or device can assist a medical practitioner, a first aid helper, or nurse to decide whether the patient under observation is developing a disease or condition as taught herein, after which appropriate action or treatment can be performed.

A home-test kit gives the patient a readout which she can communicate to a medicinal practitioner, a first aid helper or to the emergency department of a hospital, after which appropriate action can be taken. Such a home-test device is of particular interest for people having either a history of, or are at risk of suffering from any one disease or condition as taught herein.

Non-limiting examples are: systems comprising specific binding molecules for the requisite biomarker(s) attached to a solid phase, e.g. lateral flow strips or dipstick devices and the like well known in the art. One non-limiting example to perform a biochemical assay is to use a test-strip and labelled antibodies which combination does not require any washing of the membrane. The test strip is well known, for example, in the field of pregnancy testing kits where an anti-hCG antibody is present on the support, and is carried complexed with hCG by the flow of urine onto an immobilised second antibody that permits visualisation. Other non-limiting examples of such home test devices, systems or kits can be found for example in the following U.S. patents: 6,107,045, 6,974,706, 5,108,889, 6,027,944, 6,482,156, 6,511,814, 5,824,268, 5,726,010, 6,001,658 or U.S. patent applications: 2008/0090305 or 2003/0109067. Preferably, it is disclosed a lateral flow device or dipstick. Such dipstick comprises a test strip allowing migration of a sample by capillary flow from one end of the strip where the sample is applied to the other end of such strip where presence of an analyte in said sample is measured. Preferably, it is disclosed a device comprising a reagent strip. Such reagent strip comprises one or more test pads which when wetted with the sample, provide a colour change in the presence of an analyte and/or indicate the concentration of the protein in said sample.

In order to obtain a semi-quantitative test strip in which only a signal is formed once the level of the requisite biomarker(s) in the sample is higher than a certain predetermined threshold level or value, a predetermined amount of fixed capture antibodies for the biomarker(s) can be present on the test strip. This enables the capture of a certain amount of the biomarker(s) present in the sample, corresponding to the threshold level or value as predetermined. The remaining amount of biomarker(s) (if any) bound by e.g. a conjugated or labelled binding molecules can then be allowed to migrate to a detection zone which subsequently only produces a signal if the level of the biomarker(s) in the sample is higher than the predetermined threshold level or value.

Another possibility to determine whether the amount of any the requisite biomarker(s) in the sample is below or above a certain threshold level or value, is to use a primary capturing antibody capturing all said biomarker(s) present in the sample, in combination with a labelled secondary antibody, developing a certain signal or colour when bound to the solid phase. The intensity of the colour or signal can then either be compared to a reference colour or signal chart indicating that when the intensity of the signal is above a certain threshold signal, the test is positive. Alternatively, the amount or intensity of the colour or signal can be measured with an electronic device comprising e.g. a light absorbance sensor or light emission meter, resulting in a numerical value of signal intensity or colour absorbance formed, which can then be displayed to the subject in the form of a negative result if said numerical value is below the threshold value or a positive result if said numerical value is above the threshold value. It is of particular relevance in monitoring the level of said biomarker(s) in a patient over a period of time.

The reference value or range can e.g. be determined using the home device in a period wherein the subject is free of a given disease or condition, giving the patient an indication of her base-line level of the biomarker(s). Regularly using the home test device will thus enable the subject to notice a sudden change in levels of said biomarker(s) as compared to the base-line level, which can enable her to contact a medical practitioner.

Alternatively, the reference value can be determined in the subject suffering from a given disease or condition as taught herein, which then indicates her personal "risk level" for the biomarker(s), i.e. the level of the biomarker(s) which indicates she is or will soon be exposed to said disease or condition. This risk level is interesting for monitoring the disease progression or for evaluating the effect of the treatment.

Furthermore, the reference value or level can be established through combined measurement results in subjects with highly similar disease states or phenotypes (e.g. all having no disease or condition as taught herein or having said disease or condition).

Non-limiting examples of semi-quantitative tests known in the art, the principle of which could be used for the home test device as disclosed are the HIV/AIDS test or Prostate Cancer tests sold by Sanitoets. The home prostate test is a rapid test intended as an initial semi-quantitative test to detect PSA blood levels higher than 4 ng/ml in whole blood. The typical home self-test kit comprises the following components: a test device to which the blood sample is to be administered and which results in a signal when the protein level is above a certain threshold level, an amount of diluent e.g. in dropper pipette to help the transfer of the analytes (i.e. the protein of interest) from the sample application zone to the signal detection zone, optionally an empty pipette for blood specimen collection, a finger pricking device, optionally a sterile swab to clean the area of pricking and instructions of use of the kit.

Similar tests are also known for e.g. breast cancer detection and CRP-protein level detection in view of cardiac risk home tests. The latter test encompasses the sending of the test result to a laboratory, where the result is interpreted by a technical or medical expert. Such telephone or internet based diagnosis of the patient's condition is of course possible and advisable with most of the kits, since interpretation of the test result is often more important than conducting the test. When using an electronic device as mentioned above which gives a numerical value of the level of protein present in the sample, this value can of course easily be communicated through telephone, mobile telephone, satellite phone, E-mail, internet or other communication means, warning a hospital, a medicinal practitioner or a first aid team that a person is, or may be at risk of, suffering from the disease or condition as taught herein. A non-limiting example of such a system is disclosed in U.S. patent 6,482,156.

The presence and/or concentration of biomarker(s) in a sample can be measured by surface plasmon resonance (SPR) using a chip having binding molecule for said biomarker(s) immobilized thereon, fluorescence resonance energy transfer (FRET), bioluminescence resonance energy transfer (BRET), fluorescence quenching, fluorescence polarization measurement or other means known in the art. Any of the binding assays described can be used to determine the presence and/or concentration of any biomarker(s) in a sample. To do so, binding molecules for the biomarker(s) are reacted with a sample, and the concentration of the biomarker(s) is measured as appropriate for the binding assay being used. To validate and calibrate an assay, control reactions using different concentrations of standard biomarker(s) and/or binding molecule therefore can be performed. Where solid phase assays are employed, after incubation, a washing step is performed to remove unbound markers. Bound biomarker is measured as appropriate for the given label (*e.g.,* scintillation counting, fluorescence, antibody-dye etc.). If a qualitative result is desired, controls and different concentrations may not be necessary. Of course, the roles of said biomarker(s) and binding molecule may be switched; the skilled person may adapt the method so binding molecule is applied to sample, at various concentrations of sample.

The above aspects are further supported by the following non-limiting examples.

**EXAMPLES**

**Example 1: Test panels for HDP /PE prediction**

Prospective clinical samples were collected from pregnant women with a singleton pregnancy at 15+/-1 and 20 +/-1 weeks' gestation and which were either diagnosed with pre-eclampsia (cases) or not diagnosed with pre-eclampsia (controls) in the further course of their pregnancy. All samples were obtained from participants in the SCOPE study (SCreening fOr Pregnancy Endpoints), Australian Clinical Trials Registry ACTRN12607000551493, a prospective screening study of nulliparous women. Written consent was obtained from each participant. The inclusion criteria applied for the study were nulliparity, singleton pregnancy, gestation age between 14 weeks 0 days and 16 weeks 6 days gestation and informed consent to participate. The exclusion criteria applied were: Unsure of last menstrual period (LMP) and unwilling to have ultrasound scan at <= 20 weeks, >=3 miscarriages, >=3 terminations, major fetal anomaly/abnormal karyotype, essential hypertension treated pre-pregnancy, moderate-severe hypertension at booking >=160/100 mmHg, diabetes, renal disease, systemic lupus erythematosus, anti-phospholipid syndrome, sickle cell disease, HIV positive, major uterine anomaly, cervical suture, knife cone biopsy, ruptured membranes now, long term steroids, treatment low-dose aspirin, treatment calcium (>1g/24h), treatment eicosopentanoic acid (fish oil), treatment vitamin C >=1000mg & Vit E >=400iu, treatment heparin/low molecular weight heparin.

Preeclampsia defined as gestational hypertension (systolic blood pressure (BP) >= 140 mmHg and/or diastolic BP >= 90mmHg (Korotkoff V) on at least 2 occasions 4 hours apart after 20 weeks gestation but before the onset of labour) or postpartum systolic BP >= 140 mmHg and/or diastolic BP >= 90mmHg postpartum on at least 2 occasions 4 hours apart with proteinuria >= 300 mg/24h or spot urine protein: creatinine ratio >=30 mg/mmol creatinine or urine dipstick protein >= 2 or any multi-system complication of preeclampsia. Multisystem complications include any of the following: 1. Acute renal insufficiency defined as a new increase in serum creatinine >=100 umol/L antepartum or >130 umol/L postpartum 2. Liver disease defined as raised aspartate transaminase and/or alanine transaminase >45 IU/L and/or severe right upper quadrant or epigastric pain or liver rupture 3. Neurological problems defined as eclampsia or imminent eclampsia (severe headache with hyperreflexia and persistent visual disturbance) or cerebral haemorrhage 4. Haematological including thrombocytopenia (platelets <100 x $10^9$/L), disseminated intravascular coagulation or haemolysis, diagnosed by features on blood film (e.g., fragmented cells, helmet cells) and reduced haptoglobin. Preeclampsia could be diagnosed at any stage during pregnancy after recruitment until delivery or in the first 2 weeks after delivery.

Spontaneous preterm birth is defined as spontaneous preterm labour or preterm premature rupture of the membranes (PPROM) resulting in preterm birth at <37.0 weeks.

Preterm preeclampsia is defined as preeclampsia resulting in delivery at <37.0 weeks.

Early onset preeclampsia is defined as preeclampsia resulting in delivery at <34.0 weeks.

Small for Gestational Age is defined as a birthweight <10th% using customized centiles, adjusted for maternal weight, height, parity, ethnicity and infant sex. The weight is determined within 24 hours after the baby's birth.

Clinical data on known risk factors for pre-eclampsia (Zhong et al, Prenatal Diagnosis, 30, p. 293-308, 2010; Sibai et al, 365, p. 785-799, 2005) was collected at 15+/-1 and 20 +/-1 weeks' gestation by interview and examination of the women. Ultrasound data were obtained at 20 weeks on fetal measurements, anatomy, uterine and umbilical artery Doppler and cervical length. Fetal growth, uterine and umbilical Dopplers are measured at 24 weeks. Pregnancy outcome was tracked and the woman seen within 48 hours of delivery. Baby measurements are obtained within 48 hours of delivery.

In **Table 1** an overview of baseline characteristics of the cases (n= 50) and controls (n=100) is given together with some clinical parameters as obtained at the 15 and 20 weeks interviews and examinations. Blood pressure measurements were performed twice. The mean arterial pressure is calculated as follows: (1/3 * systolic blood pressure + 2/3 * diastolic blood pressure).

**Table 1**

| Parameter | Code | Controls (100) | Cases (50) |
|---|---|---|---|
| Age mother | | 30.40 (5.38) | 30.58 (4.60) |
| Ethnicity | | Asian = 3<br>Caucasian = 86<br>Indian = 5<br>Maori = 3 | African Ancestry = 1<br>Asian = 4<br>Caucasian = 38<br>Indian = 3 |

(continued)

| Parameter | Code | Controls (100) | Cases (50) |
|---|---|---|---|
| | | Pacific Islander = 2 Other (including African) = 1 | Maori = 2 Pacific Islander = 2 |
| Mother of patient had preeclampsia (yes/no) | | yes = 1 no = 95 Unknown = 4 | yes =6 no = 43 Unknown = 1 |
| Any sister of patient had preeclampsia (yes/no) | | yes = 2 no = 98 | yes = 3 no =47 |
| Father of patient has ischemic heart disease (yes/no) | father_any_ihd | yes = 9 no = 91 | yes = 15 no = 35 |
| Mother or sister of patient had preeclampsia (yes/no) | fh_pet | yes = 3 no = 97 | yes = 9 no = 41 |
| Mother or sister of patient had preeclampsia and/or father of patient has ischemic heart disease (yes/no) | fh_petxcardio | yes = 12 no = 88 | yes = 23 no = 27 |
| BMI at 15 weeks | 1st_vst_bmi | 25.38 (5.00) | 26.60 (4.39) |
| diastolic blood pressure at 15 weeks visit - 1st measurement (mm Hg) | 1st_vst_1st_dbp | 65.29 (8.10) | 71.40 (8.42) |
| Systolic blood pressure at 15 weeks visit - 1st measurement (mm Hg) | 1st_vst_1st_sbp | 107.92 (10.68) | 114.28 (10.92) |
| diastolic blood pressure at 15 weeks visit - 2nd measurement (mm Hg) | 1st_vst_2nd_dbp (also denoted herein 1st_vst_dbp_2nd) | 64.68 (7.80) | 71.32 (8.72) |
| Systolic blood pressure at 15 weeks visit - 2nd measurement (mm Hg) | 1st_vst_2nd_sbp (also denoted herein 1st_vst_sbp_2nd) | 106.14 (9.77) | 113.64 (11.32) |
| Mean arterial pressure calculated at 15 weeks visit from 1st measurement blood pressures | 1st_vst_map_1st | 79.50 (8.13) | 85.69 (8.43) |
| Mean arterial pressure at 15 weeks visit calculated from 2nd measurement blood pressures | 1st_vst_map_2nd | 78.50 (7.62) | 85.43 (8.81) |
| Random blood glucose level (mmol/L) at 15 weeks visit | 1st_vst_random_gluc ose | 5.16 (0.90) | 5.33 (1.03) |
| High Density Lipoprotein levels | bb_hdl | 1.83 (0.40) | 1.70 (0.31) |
| Ratio of total cholesterol to high density lipoprotein | bb_total_hdl_ratio | 3.12 (0.69) | 3.36 (0.76) |
| triglycerides levels | bb_trig | 1.54 (0.67) | 1.68 (0.76) |
| Metabolic syndrome | Metabolic_syndrome | yes = 9 No = 91 | yes = 13 No = 37 |
| diastolic blood pressure at 20 weeks visit - 1st measurement (mm Hg) | 2nd_vst_1st_dbp | 64.30 (7.29) | 69.22 (7.80) |

(continued)

| Parameter | Code | Controls (100) | Cases (50) |
|---|---|---|---|
| Systolic blood pressure at 20 weeks visit - 1st measurement (mm Hg) | 2nd_vst_1st_sbp | 109.20 (10.65) | 114.62 (9.32) |
| diastolic blood pressure at 20 weeks visit - 2nd measurement (mm Hg) | 2nd_vst_2nd_dbp | 64.64 (7.20) | 69.04 (8.10) |
| Systolic blood pressure at 20 weeks visit - 2nd measurement (mm Hg) | 2nd_vst_2nd_sbp | 108.35 (10.57) | 113.56 (9.89) |
| Mean arterial pressure calculated at 20 weeks visit from 1st measurement blood pressures | 2nd_vst_map_1st | 79.27 (7.24) | 84.35 (7.08) |
| Mean arterial pressure at 20 weeks visit calculated from 2nd measurement blood pressures | 2nd_vst_map_2nd | 79.21 (6.96) | 83.88 (7.54) |
| Random blood glucose level (mmol/L) at 20 weeks visit | 2nd_vst_random_glu cose | 5.02 (0.79) | 5.69 (1.11) |
| birth weight of newborn (g) | | 3561 (478) | 2933 (775) |
| Highest diastolic blood pressure measured during pregnancy | highest_dbp | 75.00 (9.94) | 104.34 (9.88) |
| Highest systolic blood pressure measured during pregnancy | highest_sbp | 121.28 (13.29) | 164.06 (19.01) |
| Maximal read out for dipstick proteinurea (number of patients) | | dipstick = 1: 93 dipstick =2: 6 No data: 1 | dipstick = 1: 5 disptick = 2: 8 dipstick = 3: 23 |
| Newborn is Small for Gestational Age (number of patients) | | 5 | 11 |
| Preeclampsia (number of patients) | | - | 50 Early onset preeclampsia: 6 Preterm preeclampsia: 18 Multisystem complications: 16 |

Further evaluation of the subjects allowed to refine the information "Mother of patient had preeclampsia (yes/no)" as yes = 1, no = 99, which is the information included in Table 3 and particularly relied on in Examples 5 and 6. Further evaluation allowed to more accurately determine the ethnicity of the subjects in Table 1 as Asian = 4, Caucasian = 38, Indian = 3, Maori = 1, Pacific Islander = 3, and Other (including African) =1, which is the information included in Table 3.

The case-control study was further expanded with another 50 cases and 100 controls obtained from another participating centre to the SCOPE study located in Australia. In **Table 2** an overview of baseline characteristics of the cases (n= 50) and controls (n=100) is given together with some clinical parameters as obtained at the 15 and 20 weeks interviews and examinations. Blood pressure measurements were performed twice. The mean arterial pressure is calculated as follows: (1/3 * systolic blood pressure + 2/3 * diastolic blood pressure).

**Table 2**

| Parameter | Code | Controls (100) | Cases (50) |
|---|---|---|---|
| Age mother | | 23.26 (5.17) | 22.54 (4.25) |
| | | Asian = 3 Caucasian = 93 | Asian = 3 Caucasian = 46 |

(continued)

| Parameter | Code | Controls (100) | Cases (50) |
|---|---|---|---|
| Ethnicity | | Indian = 0<br>Maori = 1<br>Pacific Islander = 0<br>Other (including African)=3 | Indian = 0<br>Maori = 0<br>Pacific Islander = 0<br>Other (including African)=1 |
| Mother of patient had preeclampsia (yes/no) | | yes =16<br>no = 84 | yes = 9<br>no = 41 |
| Any sister of patient had preeclampsia (yes/no) | | yes = 5<br>no = 95 | yes = 3<br>no =47 |
| Father of patient has ischemic heart disease (yes/no) | father_any_ihd | yes = 7<br>no = 93 | yes = 6<br>no = 44 |
| Mother or sister of patient had preeclampsia (yes/no) | fh_pet | yes = 18<br>no = 82 | yes = 11<br>no = 39 |
| Mother or sister of patient had preeclampsia and/or father of patient has ischemic heart disease (yes/no) | fh_petxcardio | yes = 23<br>no = 77 | yes = 16<br>no = 34 |
| BMI at 15 weeks | 1st_vst_bmi | 26.28 (6.44) | 29.96 (9.29) |
| diastolic blood pressure at 15 weeks visit - 1st measurement (mm Hg) | 1st_vst_1st_dbp | 64.14 (7.81) | 66.54 (7.55) |
| Systolic blood pressure at 15 weeks visit - 1st measurement (mm Hg) | 1st_vst_1st_sbp | 109.57 (10.15) | 114.34 (11.99) |
| diastolic blood pressure at 15 weeks visit - 2nd measurement (mm Hg) | 1st_vst_2nd_dbp (also denoted herein 1st_vst_dbp_2nd) | 64.11 (7.71) | 65.88 (7.73) |
| Systolic blood pressure at 15 weeks visit - 2nd measurement (mm Hg) | 1st_vst_2nd_sbp (also denoted herein 1st_vst_sbp_2nd) | 108.44 (9.60) | 113.38 (10.39) |
| Mean arterial pressure calculated at 15 weeks visit from 1st measurement blood pressures | 1st_vst_map_1st | 79.28 (7.75) | 82.47 (8.17) |
| Mean arterial pressure at 15 weeks visit calculated from 2nd measurement blood pressures | 1st_vst_map_2nd | 78.89 (7.40) | 81.71 (7.80) |
| Random blood glucose level (mmol/L) at 15 weeks visit | 1 st_vst_random_gluc ose | 5.53 (0.75) | 5.62 (0.93) |
| High Density Lipoprotein levels | bb_hdl | 1.74 (0.38) | 1.60 (0.36) |
| Ratio of total cholesterol to high density lipoprotein | bb_total_hdl_ratio | 3.19 (0.79) | 3.62 (0.98) |
| triglycerides levels | bb_trig | 1.43 (0.53) | 1.71 (0.69) |
| Metabolic syndrome | Metabolic_syndrome | yes = 13<br>No = 87 | yes = 18<br>No = 32 |
| diastolic blood pressure at 20 weeks visit - 1st measurement (mm Hg) | 2nd_vst_1st_dbp | 65.36 (8.08) | 68.42 (9.67) |
| Systolic blood pressure at 20 weeks visit - 1st measurement (mm Hg) | 2nd_vst_1st_sbp | 111.90 (10.50) | 116.00 (10.30) |

(continued)

| Parameter | Code | Controls (100) | Cases (50) |
|---|---|---|---|
| diastolic blood pressure at 20 weeks visit - 2nd measurement (mm Hg) | 2nd_vst_2nd_dbp | 64.95 (7.75) | 67.92 (8.96) |
| Systolic blood pressure at 20 weeks visit - 2nd measurement (mm Hg) | 2nd_vst_2nd_sbp | 111.23 (10.20) | 115.4 (9.60) |
| Mean arterial pressure calculated at 20 weeks visit from 1st measurement blood pressures | 2nd_vst_map_1st | 80.87 (7.51) | 84.28 (8.62) |
| Mean arterial pressure at 20 weeks visit calculated from 2nd measurement blood pressures | 2nd_vst_map_2nd | 80.38 (7.29) | 83.75 (8.17) |
| Random blood glucose level (mmol/L) at 20 weeks visit | 2nd_vst_random_glucose | 5.77 (0.94) | 5.57 (1.18) |
| birth weight of newborn (g) | | 3410 (645) | 3098 (789) |
| Highest diastolic blood pressure measured during pregnancy | highest_dbp | 73.12 (9.19) | 101.7 (9.15) |
| Highest systolic blood pressure measured during pregnancy | highest_sbp | 124.32 (12.43) | 162.80 (17.39) |
| Maximal read out for dipstick proteinurea (number of patients) | | dipstick = 1: 1 dipstick = 2: 1 No data: 98 | dipstick = 1: 6 dipstick = 2: 4 dipstick = 3: 10 dipstick = 4: 14 no data: 16 |
| Newborn is Small for Gestational Age (number of patients) | | 11 | 11 |
| Preeclampsia (number of patients) | | - | 50 Early onset preeclampsia: 4 Preterm preeclampsia: 12 Multisystem complications: 18 |

A comparison of the baseline characteristics in the two study populations represented in Tables 1 and 2 indicates that the populations appear rather distinct. **Table 3** represents the baseline characteristics and clinical parameters for the cases and controls as captured in Tables 1 and 2 as a single population of cases (n=100) and controls (n=200).

**Table 3**

| Parameter | Code | Controls (200) | Cases (100) |
|---|---|---|---|
| Age mother | | 26.83 (6.36) | 26.56 (5.98) |
| Ethnicity | | Asian = 6 Caucasian = 179 Indian = 5 Maori = 4 | Asian = 7 Caucasian = 84 Indian = 3 Maori = 1 |

(continued)

| Parameter | Code | Controls (200) | Cases (100) |
|---|---|---|---|
| | | Pacific Islander = 2 Other (including African) = 4 | Pacific Islander = 3 Other (including African) = 2 |
| Mother of patient had preeclampsia (yes/no) | | yes = 17 no = 183 | yes =15 no = 85 |
| Any sister of patient had preeclampsia (yes/no) | | yes = 7 no = 193 | yes = 6 no = 21 |
| Father of patient has ischemic heart disease (yes/no) | father_any_ihd | yes = 16 no = 184 | yes = 21 no = 79 |
| Mother or sister of patient had preeclampsia (yes/no) | fh_pet | yes = 21 no = 179 | yes = 20 no = 80 |
| Mother or sister of patient had preeclampsia and/or father of patient has ischemic heart disease (yes/no) | fh_petxcardio | yes = 35 no = 165 | yes = 39 no = 61 |
| BMI at 15 weeks | 1st_vst_bmi | 25.83 (5.77) | 28.28 (7.39) |
| diastolic blood pressure at 15 weeks visit - 1st measurement (mm Hg) | 1st_vst_1st_dbp | 64.72 (7.95) | 68.97 (8.32) |
| Systolic blood pressure at 15 weeks visit - 1st measurement (mm Hg) | 1st_vst_1st_sbp | 108.75 (10.42) | 114.31 (11.41) |
| diastolic blood pressure at 15 weeks visit - 2nd measurement (mm Hg) | 1st_vst_2nd_dbp (also denoted herein 1st_vst_dbp_2nd) | 64.40 (7.69) | 68.60 (10.39) |
| Systolic blood pressure at 15 weeks visit - 2nd measurement (mm Hg) | 1st_vst_2nd_sbp (also denoted herein 1st_vst_sbp_2nd) | 107.29 (9.73) | 113.51 (10.79) |
| Mean arterial pressure calculated at 15 weeks visit from 1st measurement blood pressures | 1st_vst_map_1st | 79.39 (7.92) | 84.08 (8.42) |
| Mean arterial pressure at 15 weeks visit calculated from 2nd measurement blood pressures | 1 st_vst_map_2nd | 78.69 (7.49) | 83.57 (8.49) |
| Random blood glucose level (mmol/L) at 15 weeks visit | 1 st_vst_random_glucose | 5.43 (0.87) | 5.47 (0.99) |
| High Density Lipoprotein levels | bb_hdl | 1.79 (0.39) | 1.65 (0.34) |
| Ratio of total cholesterol to high density lipoprotein | bb_total_hdl_ratio | 3.16 (0.74) | 3.49 (0.88) |
| triglycerides levels | bb_trig | 1.48 (0.60) | 1.69 (0.72) |
| Metabolic syndrome | Metabolic_syndrome | yes = 22 no = 178 | yes = 31 no = 69 |

(continued)

| Parameter | Code | Controls (200) | Cases (100) |
|---|---|---|---|
| diastolic blood pressure at 20 weeks visit - 1st measurement (mm Hg) | 2nd_vst_1st_dbp | 64.83 (7.69) | 68.82 (8.75) |
| Systolic blood pressure at 20 weeks visit - 1st measurement (mm Hg) | 2nd_vst_1st_sbp | 110.55 (10.64) | 115.31 (9.80) |
| diastolic blood pressure at 20 weeks visit - 2nd measurement (mm Hg) | 2nd_vst_2nd_dbp | 64.80 (7.46) | 68.48 (8.52) |
| Systolic blood pressure at 20 weeks visit - 2nd measurement (mm Hg) | 2nd_vst_2nd_sbp | 109.79 (10.46) | 114.48 (9.74) |
| Mean arterial pressure calculated at 20 weeks visit from 1st measurement blood pressures | 2nd_vst_map_1st | 80.07 (7.40) | 84.32 (7.85) |
| Mean arterial pressure at 20 weeks visit calculated from 2nd measurement blood pressures | 2nd_vst_map_2nd | 79.79 (7.14) | 83.81 (7.82) |
| Random blood glucose level (mmol/L) at 20 weeks visit | 2nd_vst_random_glucose | 5.40 (0.94) | 5.63 (1.11) |
| birth weight of newborn (g) | | 3561 (478) | 3016 (782) |
| Highest diastolic blood pressure measured during pregnancy | highest_dbp | 74.06 (9.59) | 103.02 (9.57) |
| Highest systolic blood pressure measured during pregnancy | highest_sbp | 122.80 (12.93) | 163.43 (18.13) |
| Maximal read out for dipstick proteinurea (number of patients) | | dipstick = 1: 94 dipstick =2: 7 No data: 99 | dipstick = 1: 11 dipstick = 2: 12 dipstick = 3: 33 dipstick = 4: 28 no data: 16 |
| Newborn is Small for Gestational Age (number of patients) | | 16 | 22 |
| Preeclampsia (number of patients) | | - | 100 Early onset preeclampsia: 10 Preterm preeclampsia: 30 Multisystem complications: 34 |

Caption Tables 1, 2 and 3: Maternal characteristics including information about family history of disease, clinical parameters obtained during visits at 15 weeks and 20 weeks of gestation and some maternal and fetal characteristics as collected at pregnancy outcome. Results are N, number of patients, or mean (Standard deviation).

**Example 2: MASSterclass® targeted protein quantification**

The following describes one exemplary and preferred way of targeted protein quantification in samples, particularly as also used in and throughout the present examples.

*MASSTERCLASS experimental setup*

MASSterclass® assays use targeted tandem mass spectrometry with stable isotope dilution as an end-stage peptide quantitation system (also called Multiple Reaction Monitoring (MRM) and Single Reaction Monitoring (SRM)). The targeted peptide is specific (*i.e.*, proteotypic) for the specific protein of interest. *i.e.*, the amount of peptide measured is directly related to the amount of protein in the original sample. To reach the specificity and sensitivity needed for biomarker quantitation in complex samples, peptide fractionations precede the end-stage quantitation step.

For the proteins cited, the panel building was based on the relative readouts of proteotypic peptides listed below as quantified in MASSterclass. For PRDX1 two different peptides are taken into account. Additionally, it is noted below whether or not the extra peptide fractionation step was applied to generate the readouts as used within the test panels:

| Protein | Proteotypic peptide sequence | Additional peptide fractionation (yes/no) |
|---|---|---|
| ENG | LPDTPQGLLGEAR | yes |
| FLT4 | GPILEATAGDELVK | yes |
| GPR126 | VILPQTSDAYQVSVAK | no |
| LNPEP | YISIGSEAEK | yes |
| ICAM3 | IALETSLSK | no |
| PRDX1 | ATAVVDGAFK | no |
| PRDX1 | ADEGISFR | yes |
| PRDX2 | EGGLGPLNIPLLADVTR | no |
| TNXB | TVTVEDLEPGK | yes |
| CRP | GYSIFSYATK | no |
| MST1 | SPLNDFQVLR | no |
| PRCP | YYGESLPFGDNSFK | yes |
| COL6A3 | SLDEISQPAQELK | yes |
| SPINT1 | YTSGFDELQR | yes |
| HSPG2 | GSIQVDGEELVSGR | yes |
| SEPP1 | LPTDSELAPR | no |
| QSOX1 | LAGAPSEDPQFPK | yes |
| IGFALS | LAELPADALGPLQR | yes |
| MCAM | GATLALTQVTPQDER | yes |
| GPLD1 | IADVTSGLIGGEDGR | yes |

For the proteins read outs as obtained for the combined data set of Table 3, the following peptides were quantified by means of MASSterclass. In this case, the extra fractionation step was always applied, For ADAM12, ECM1, LCAT, SPINT1, and IGFALS more than one peptides were measured.

| Protein | Proteotypic peptide sequence |
|---|---|
| ADAM12 | DLETSLEK |
| ADAM12 | ELIINLER |
| ECM1 | ELLALIQLER |
| ECM1 | NVALVSGDTENAK |
| ECM1 | EVGPPLPQEAVPLQK |
| ENG | LPDTPQGLLGEAR |
| FLT4 | GPILEATAGDELVK |
| LCAT | TYSVEYLDSSK |
| LCAT | LEPGQQEEYYR |

(continued)

| Protein | Proteotypic peptide sequence |
|---------|------------------------------|
| PCYOX1 | SDFYDIVLVATPLNR |
| ANGPTL6 | LAAADGAVAGEVR |
| PRCP | YYGESLPFGDNSFK |
| SPINT1 | YTSGFDELQR |
| SPINT1 | DPNQVELWGLK |
| XPNPEP2 | GTVDEFSGAEIVDK |
| HSPG2 | GSIQVDGEELVSGR |
| SEPP1 | LPTDSELAPR |
| IGFALS | LAELPADALGPLQR |
| IGFALS | VAGLLEDTFPGLLGLR |
| MUC18 | GATLALTQVTPQDER |
| ROBO4 | EDFQIQPR |
| ENPP2 | DIEHLTSLDFFR |
| S100A9 | VIEHIMEDLDTNADK |

A suitable MASSterclass® assay may include the following steps:

- Preparation of plasma or serum sample

- Depletion of human albumin and IgG (complexity reduction on protein level) using affinity capture with anti-albumin and anti-IgG antibodies using ProteoPrep spin columns (Sigma Aldrich)

- Spiking of known amounts of isotopically labelled peptides. This peptide has the same amino acid sequence as the proteotypic peptide of interest, typically with one isotopically labelled amino acid built in to generate a mass difference. During the entire process, the labelled peptide has identical chemical and chromatographic behaviour as the endogenous peptide, except during the end-stage quantitation step which is based on molecular mass.

- Tryptic digest. The proteins in the depleted serum/plasma sample are digested into peptides using trypsin. This enzyme cleaves proteins C-terminally from lysine and arginine, except when a proline is present C-terminally of the lysine or arginine. Before digestion, proteins are denatured by boiling, which renders the protein molecule more accessible for the trypsin activity during the 16h incubation at 37°C.

- Peptide-based fractionation: dPC™ Fractionator (CellBiosciences, now ProteinSimple) enables to rapidly and reproducibly fractionate complex peptide samples with great precision based on their isoelectric points using parallel isoelectric focusing. Peptides are trapped into pH-controlled gel plugs, present in a Digital ProteomeChip™ (Westburg, Leusden, The Netherlands), that are exposed to high electric fields. The chip separates the sample running chamber into anode and cathode electrode chambers. The anode buffer is more acidic, the cathode buffer more basic than any pi traps of the chip used. An applied electric field maintains circulation of the peptides through the plugs. Peptides get trapped when passing a pH plug that is at or very near its pl.

- LC-MS/MS based quantitation, including further separation on reversed phase (C18) nanoLC (PepMap C18; Dionex) and MS/MS: tandem mass spectrometry using MRM (4000 QTRAP; ABI)/SRM (Vantage TSQ; Thermo Scientific) mode. The LC column is connected to an electrospray needle connected to the source head of the mass spectrometer. As material elutes from the column, molecules are ionized and enter the mass spectrometer in the gas phase. The peptide that is monitored is specifically selected to pass the first quadrupole (Q1), based on its mass to charge ratio (m/z). The selected peptide is then fragmented in a second quadrupole (Q2) which is used as a collision cell. The resulting fragments then enter the third quadrupole (Q3). Depending on the instrument settings (determined during the assay development phase) only a specific peptide fragment or specific peptide fragments (or so called transitions) are selected for detection.

- The combination of the m/z of the monitored peptide and the m/z of the monitored fragment of this peptide is called a transition. This process can be performed for multiple transitions during one experiment. Both the endogenous peptide (analyte) and its corresponding isotopically labelled synthetic peptide (internal standard) elute at the same retention time, and are measured in the same LC-MS/MS experiment.

- The MASSterclass® readout is defined by the ratio between the area under the peak specific for the analyte and the area under the peak specific for the synthetic isotopically labelled analogue (internal standard). MASSterclass® readouts are directly related to the original concentration of the protein in the sample. MASSterclass® readouts can therefore be compared between different samples and groups of samples.

A suitable MASSterclass® protocol followed in the present study is given here below:

- 25$\mu$L of plasma is subjected to a depletion of human albumin and IgG (ProteoPrep spin columns; Sigma Aldrich) according to the manufacturer's protocol, except that 20mM $NH_4HCO_3$ was used as the binding/equilibration buffer.

- The depleted sample (225$\mu$L) is denatured for 15min at 95°C and immediately cooled on ice

- 2000 fmol of the isotopically labelled peptide (custom made 'Heavy AQUA' peptide; Thermo Scientific) is spiked in the sample

- 20$\mu$g trypsin is added to the sample and digestion is allowed for 16h at 37°C. Final volume of each sample at this stage is 270 $\mu$L

- For LC-MS/MS analysis on the unfractionated peptides: 10$\mu$L of the digested sample was first diluted 1/15 in solvent A (0.1% formic acid) and then 1/10 in the same solvent containing 250 amol/$\mu$L of all isotopically labelled peptides (custom made 'Heavy AQUA' peptide; Thermo Scientific) of interest.

- For LC-MS/MS analysis on the fractionated peptides: 120$\mu$L of the digested sample is fractionated using the dPC™ Fractionator with a Digital ProteomeChip spanning a pH range of 3.5-4.5. Buffers and chips were supplied by CellBiosciences, as well as the separation protocol used. After separation, gel plugs are harvested and soaked in 200$\mu$L 0.2% formic acid in 50% acetonitrile for 1h at 37°C to recover the peptides.

Plugs are removed and the solvent dried under vacuum. Peptides are redissolved in 100$\mu$L 0.1% formic acid.

- 20$\mu$L of the final dilution (unfractionated or fractionated peptide pool) was separated using reverse-phase NanoLC with on-line MS/MS in MRM/SRM mode:

  - Column: PepMap C18, 75$\mu$m I.D. x 25cm L, 100 Å pore diameter, 5$\mu$m particle size

  - Solvent A: 0.1% formic acid

  - Solvent B: 80% acetonitrile, 0.1% formic acid

  - Gradient: 30 min; 2%-55% Solvent B

  - MS/MS in MRM mode: method contains the transitions for the analyte as well as for the synthetic, labelled peptide.

  - The used transitions were experimentally determined and selected during protein assay development

  - Each of the transitions of interest was measured for a period starting 3 minutes before and ending 3 minutes after the determined retention time of the peptide of interest, making sure that each peak had at least 15 datapoints.

- The raw data was analysed and quantified using the LCQuan software (Thermo Scientific): the area under the analyte peak and under the internal standard (the labelled, synthetic peptides) peak at the same C18 retention time was determined by automatic peak detection. These were checked manually. The MASSterclass® readout was defined by the ratio of the analyte peak area and the internal standard peak area

**Example 3: Statistical analysis**

Logistic regression was used to define multivariate classifier models (test panels) that predict the outcome (pre-eclampsia / no pre-eclampsia) [Royston et al. 2009, Prognosis and prognostic research: Developing a prognostic model, BMJ 2009: 338:b604].

The predictors (biomarkers and parameters) were normalised. The binary variables were coded 0/1, the analyte concentrations and relative concentrations (MasterClass measurements) were log-transformed. For feature selection, all parameters were normalised (Z-normalisation).

Feature selection was performed using the shrinkage and selection method Lasso (Tibshirani 1996, Regression shrinkage and selection via the lasso, J. Royal. Statist. Soc B. 58(1): 267-288). The performance of the models (test panels) was estimated using the apparent area under the receiver-operating curve (AUC). The prediction error for the classifiers was estimated using cross-validation. The classifiers were ranked based on their performance and prediction error.

Where indicated, the test panels were also evaluated for their performance for "rule-in" tests (i.e., using PPV criterion) . To this aim, the panels were assessed for their ability to adequately predict pre-eclampsia without identifying too many false positives. Within the context of a low prevalence disease, such as PE, a Positive Predictive Value (PPV) above or equal to 0.2 (i.e., 20%) is found clinically desirable. PPV = # True Positives / (#True Positives + False Positives).

To enable a quantitative assessment of the above PPV criterion, PPV-values are calculated for a population of 1000 pregnancies, taking into account the prevalence as relevant to the population studied. For all pre-eclampsia, prevalence of 5.3% has been previously reported in literature (BMJ 2011, vol. 342, d1875, doi: 10.1136/bmj.d1875) and may be used for this calculation. The PPV data are then transformed to sensitivity and specificity values to allow plotting of the PPV threshold on the receiving-operating curve (ROC). Exemplary calculations for prevalence 5.3% are shown in **Figure 1.**

In clinical reality women that are obese (BMI $\geq$ 30 pre-pregnancy or in 1st trimester) are considered at risk for a number pregnancy complications, including for example gestational diabetes, pre-eclampsia, etc., and therefore already subject to increased antenatal care (NHS National Institute for Health and Clinical Excellence (NICE) clinical guideline 62: Antenatal Care - Routine Care for the Healthy Pregnant woman, March 2008). Therefore, pre-eclampsia prediction panels that focus on subjects considered to be at low risk for PE, namely non-obese women, more particularly nulliparous non-obese women, are considered particularly clinically relevant. Panels predicting pre-eclampsia in this sub-population were thus also developed. Again "rule-in" tests were also investigated; as outlined above, taking into account the prevalence for non-obese subjects. For pre-eclampsia in non-obese subjects, prevalence of 4.3% has been previously reported in literature (BMJ 2011, vol. 342, d1875, *supra*). Exemplary calculations of the PPV threshold on the ROC curve for prevalence 4.3% are also shown in **Figure 1.**

**Figure 1** shows an exemplary plot of PPV-threshold curves calculated for pre-eclampsia in all, non-obese and obese subjects based on previously reported prevalence in these populations, respectively, 5.3%, 4.3% and 10.3% (BMJ 2011, vol. 342, d1875, *supra*).

Further, because accurate prediction of pre-term pre-eclampsia, in particular pre-eclampsia that warrants for delivery of the child before 37 weeks of gestation (<37 weeks), is considered of high clinical relevance due to the extra risks to the neonate, e.g., prematurity, and frequent serious complications to the mother. Therefore the pre-eclampsia prediction panels for all subjects (with and without PPV criterion) and for the non-obese subjects (with and without PPV criterion) were also checked for their ability to predict pre-term pre-eclampsia.

**Example 4: Results using case-control set of Table 1**

The data and analyses in this example have been obtained using the case-control set as captured in Table 1.

Unexpectedly powerful results were realised for the preferred test panels E, F, G and H as described herein. The respective biomarkers and parameters of these panels were used to develop a model that estimates the probability of contracting pre-eclampsia using logistic regression. Note that the model is specific to the quantitation platform (herein, relative quantitation using MASSterClass™). Indeed, different quantification methods may yield different estimates of the same protein concentration due to their respective sensitivity and bias. However, the performance of a model trained on quantitations obtained from different platforms will be similar if the platforms have similarly low technical variability.

In particular, an embodiment of panel F as evaluated in this experiment at 20 +/- 1 weeks consisted of the following biomarkers and parameters: measurement of IGFALS level, a score for *fh_petxcardio*, measurement of blood pressure (specifically *2nd_vst_map_1st*), measurement of blood glucose level (specifically *2nd_vst_random_glucose*), measurement of SEPP1 level, and measurement of ENG level. The model has the form:

$$risk\ score = 0.0618 * 2nd\_vst\_map\_1st + 0.736 * 2nd\_vst\_random\_glucose + 2.41 *$$
$$fh\_petxcardio + 13.1 * \log_{10}(ENG) - 20.8 * \log_{10}(SEPP1) + 14.5 * \log_{10}(IGFALS) - 11.4$$

where $\log_{10}$ is the base 10 logarithm and ENG, SEPP1 and IGFALS are the relative plasma concentrations of the proteins as measured with MASSterClass™.

The predicted probability of developing pre-eclampsia is:

$$p = 1 / ( 1 + e^{-risk\_score})$$

The model shows an AUC of 0.91 (95% confidence interval: 0.86-0.96) for the discrimination of women that develop pre-eclampsia and the women that do not develop the condition.

For comparison, the apparent areas under the receiving-operating characteristic curves of the individual constituents of the model, i.e., their individual performances, were also calculated (MedCalc package, MedCalc Software bvba, Belgium):

| Biomarker / Parameter | AUC | 95% CI |
|---|---|---|
| 2nd_vst_map_1st | 0.69 | 0.61 to 0.77 |
| 2nd_vst_random_glucose | 0.67 | 0.59 to 0.75 |
| fh_petxcardio | 0.67 | 0.59 to 0.75 |
| ENG | 0.65 | 0.57 to 0.73 |
| SEPP1 | 0.62 | 0.54 to 0.70 |
| IGFALS | 0.75 | 0.68 to 0.82 |

Further, an embodiment of panel E as evaluated in this experiment at 20 +/- 1 weeks consisted of the following biomarkers and parameters: measurement of IGFALS level, a score for *fh_petxcardio*, measurement of blood pressure (specifically 2nd_vst_map_1st), measurement of blood glucose level (specifically 2nd_vst_random_glucose), and measurement of SEPP1 level. The model has the form:

$$\text{risk score} = 0.0435 * \text{2nd\_vst\_map\_1st} + 0.716 * \text{2nd\_vst\_random\_glucose} + 1.88 *$$
$$\text{fh\_petxcardio} - 15.9 * \log_{10}(\text{SEPP1}) + 15.4 * \log_{10}(\text{IGFALS}) - 24.8$$

This model has an AUC of 0.87 (95% confidence interval: 0.81-0.92).

For comparison, the apparent areas under the receiving-operating characteristic curves of the individual constituents of the model, i.e., their individual performances, were also calculated (MedCalc package, MedCalc Software bvba, Belgium):

| Biomarker / Parameter | AUC | 95% CI |
|---|---|---|
| 2nd_vst_map_1st | 0.69 | 0.61 to 0.77 |
| 2nd_vst_random_glucose | 0.67 | 0.59 to 0.75 |
| fh_petxcardio | 0.67 | 0.59 to 0.75 |
| SEPP1 | 0.62 | 0.54 to 0.70 |
| IGFALS | 0.75 | 0.68 to 0.82 |

Further, an embodiment of panel G as evaluated in this experiment at 15 +/- 1 weeks consisted of the following biomarkers and parameters: measurement of IGFALS level, a score for *fh_petxcardio*, measurement of blood pressure (specifically 1st_vst_map_2nd), measurement of SEPP1 level, measurement of PRDX2 level, and measurement of QSOX1 level. The model has the form:

$$\text{risk score} = 0.0745 * \textit{1st\_vst\_map\_2nd} + 1.61 * \textit{fh\_petxcardio} - 1.83 * \log_{10}(\text{PRDX2}) -$$
$$6.97 * \log_{10}(\text{SEPP1}) - 4.97 * \log_{10}(\text{QSOX1}) + 10.9 * \log_{10}(\text{IGFALS}) - 22.8$$

This model has an AUC of 0.84 (95% confidence interval: 0.77-0.90).

For comparison, the apparent areas under the receiving-operating characteristic curves of the individual constituents of the model, i.e., their individual performances, were also calculated (MedCalc package, MedCalc Software bvba, Belgium):

| Biomarker / Parameter | AUC | 95% CI |
|---|---|---|
| 1 st_vst_map_2nd | 0.73 | 0.65 to 0.8 |
| fh_petxcardio | 0.67 | 0.59 to 0.75 |
| PRDX2 | 0.59 | 0.51 to 0.67 |
| SEPP1 | 0.56 | 0.48 to 0.64 |
| QSOX1 | 0.60 | 0.51 to 0.68 |
| IGFALS | 0.69 | 0.61 to 0.77 |

An embodiment of panel H as evaluated in this experiment at 15 +/- 1 weeks consisted of the following biomarkers and parameters: measurement of IGFALS level, a score for *fh_petxcardio*, measurement of blood pressure (specifically 1st_vst_map_2nd), measurement of ENG level, and measurement of QSOX1 level. The model has the form:

$$\text{risk score} = 0.0887 * \text{1st\_vst\_map\_2nd} + 1.89 * \text{fh\_petxcardio} + 8.74 * \log_{10}(\text{ENG}) - 9.12 * \log_{10}(\text{QSOX1}) + 6.62 * \log_{10}(\text{IGFALS}) - 6.71$$

This model has an AUC of 0.85 (95% confidence interval: 0.78-0.92).

For comparison, the apparent areas under the receiving-operating characteristic curves of the individual constituents of the model, i.e., their individual performances, were also calculated:

| Biomarker / Parameter | AUC | 95% CI |
|---|---|---|
| 1 st_vst_map_2nd | 0.73 | 0.65 to 0.8 |
| fh_petxcardio | 0.67 | 0.59 to 0.75 |
| ENG | 0.61 | 0.53 to 0.69 |
| QSOX1 | 0.60 | 0.51 to 0.68 |
| IGFALS | 0.69 | 0.61 to 0.77 |

Data also indicates that the measurement of any blood pressure parameter (e.g., 1st or 2nd measurement, diastolic pressure, systolic pressure or mean arterial pressure) can be included in the test panels as taught in the above examples and more generally throughout the application. 1st_vst_map_2nd (i.e., mean arterial pressure at 15 weeks visit calculated from 2nd measurement blood pressures) may be preferred in test panels for 15 +/- 2 or 1 weeks, and 2nd_vst_map_1st (mean arterial pressure calculated at 20 weeks visit from 1 st measurement blood pressures) may be preferred in test panels for 20 +/- 2 or 1 weeks.

**Example 5: Results using case-control set of Table 3**

As noted, comparison of the baseline characteristics in the two study populations represented in Tables 1 and 2 indicates that these populations appear distinct in some respects. Because it may be preferred to use pre-eclampsia prediction panels that remain comparably relevant across several or even many distinct populations, panels were shown to adequately predict pre-eclampsia in the combined population as captured in Table 3. The data and analyses in this example have thus been obtained using the combined case-control population as captured in Table 3. This example further particularly focuses on prediction of pre-eclampsia at about 20 weeks of gestation.

In this example, the following non-limiting criteria were applied to consider or classify pre-eclampsia prediction models as successful:

1. For generic panels:

- AUC $\geq$ 0.75, panels consisting of 3, 4, 5 or 6 members (biomarkers, clinical parameters), all members have to add significantly to the panel

2. For "rule-in" panels:

a. AUC $\geq$ 0.75, panels consisting of 3, 4, 5 or 6 members (biomarkers, clinical parameters), all members have to add significantly to the panel; and
b. PPV criterion: the ROC curve needs to have points in the PPV $\geq$0.2 zone with a sensitivity $\geq$ 0.5 ($\geq$50 % detection rate)

i. Panels were ranked for maximum PPV at 0.5 sensitivity

ii. Panels ranked for maximum sensitivity at 0.20 PPV.

3. For preterm pre-eclampsia prediction:

- the panels as selected under item 1 and 2 above are applied to predict subgroup preterm pre-eclampsia (i.e., to classify preterm pre-eclampsia vs. all non-pre-eclampsia controls) without modification of the weighing factors for the members of the panels (biomarkers, parameters). Panels with significantly better AUC values for preterm pre-eclampsia prediction than for all pre-eclampsia prediction (p-value AUC difference $\leq$ 0.05) were considered successful.

4. For all subjects and for non-obese subjects:

- the criteria as explained under items 1, 2 or 3 were applied to pre-eclampsia prediction both in all subjects and in non-obese subjects.

The resulting data is summarised in tables 4 to 11 below, in which:

- the field "subclass" indicates the subjects for which the prediction was made, i.e., for all subjects ("all") or for non-obese subjects ("non-obese");

- the field "PPV filter" indicates whether the panels were evaluated for their suitability as "rule-in" panels ("on") or not ("off");

- the field "applied to preterm" indicates whether the panels were applied to prediction of pre-term pre-eclampsia ("yes") or to all pre-eclampsia ("no");

- the field "#constituents" denotes the number of constituents or members (biomarkers, clinical parameters) of which a panel consists;

- the field "number of panels" specifies the number of panels considered as successful according to the aforementioned criteria upon application of the parameters defined in the above-explained fields, as specified in each table;

- the subsequent fields of each table list members (markers, clinical parameters) appearing in the panels, and specify the number of successful panels in which each respective marker or parameter was present; the higher the number of panels in which a given marker or parameter was present, the more preferred such marker or parameter may be for inclusion into successful panels embodying the principles herein disclosed;

Following each table, several exemplary but non-limiting panels which had the best performance in the tests according to that table are specified.

All panels in this example comprise the measurement of the level of insulin-like growth factor-binding protein complex acid labile subunit (IGFALS), a score for the maternal history parameter 'father with (i.e., father of the subject has/had) ischemic heart disease' (*father_any_ihd*), and measurement of blood pressure.

**Table 4. Pre-eclampsia prediction in all subjects; no PPV criterion**

| subclass | all | | | |
|---|---|---|---|---|
| PPV filter | off | | | |
| applied to preterm | no | | | |
| | | | | |
| #constituents | n=3 | n=4 | n=5 | n=6 |
| | | | | |
| number of panels | 4 | 29 | 115 | 103 |
| | | | | |
| **bb_hdl** | 0 | 6 | 24 | 24 |
| **bb_total_hdl_ratio** | 0 | 0 | 13 | 4 |
| **bmi** | 0 | 0 | 15 | 51 |
| **metabolic_syndrome** | 0 | 6 | 24 | 24 |
| **father_any_ihd** | 4 | 29 | 115 | 103 |
| **fh_pet** | 0 | 0 | 0 | 0 |
| **1st_vst_dbp_2nd** | 1 | 6 | 29 | 23 |
| **1st_vst_sbp_2nd** | 1 | 10 | 30 | 34 |
| **1st_vst_map_2nd** | 1 | 8 | 32 | 33 |
| **2nd_vst_map_2nd** | 1 | 5 | 24 | 13 |
| **ADAM12** | 0 | 11 | 78 | 50 |
| **s-ENG** | 0 | 5 | 37 | 78 |
| **LCAT** | 0 | 0 | 0 | 2 |
| **SPINT1** | 0 | 0 | 5 | 10 |
| **HSPG2** | 0 | 0 | 0 | 8 |
| **SEPP1** | 0 | 0 | 11 | 10 |
| **IGFALS** | 4 | 29 | 115 | 103 |
| **MUC18** | 0 | 1 | 23 | 32 |
| **ROBO4** | 0 | 0 | 0 | 11 |
| **ENPP2** | 0 | 0 | 0 | 3 |
| **S10A9** | 0 | 0 | 0 | 2 |

Representative but non-limiting particularly successful panels of those identified in Table 4 include:

T4.1: a 3-member panel consisting of: IGFALS level, a score for father_any_ihd and a value for 1st_vst_map_2nd. AUC = 0.75.

An exemplary risk score for panel T4.1 was calculated as: risk score = 1.2 * father_any_ihd + 3.1 * 1st_vst_map_2nd + 2.8 * $\log_{10}$MC004 - 22. The MC004 stands for the MASSterclass readout for the LAELPADALGPLQR peptide of IGFALS.

As mentioned previously, weighing factors used in such risk scores may depend on the methodology used to quantify biomarkers and measure or score parameters, and can be determined for each particular experimental setting and data set by a skilled person when the latter is provided with information on the composition of a desired panel of marker(s) and parameter(s). Consequently, in the following the particular formulas to calculate the risk scores need not be and are not listed.

T4.2: a 4-member panel consisting of: IGFALS level, ADAM12 level, a score for father_any_ihd, value for 1st_vst_map_2nd. AUC = 0.77.

T4.3: a 5-member panel consisting of: IGFALS level, s-ENG level, MUC18 level, score for father_any_ihd, value for 1st_vst_map_2nd. AUC = 0.80

T4.4: a 6-member panel consisting of: IGFALS level, s-ENG level, MUC18 level, score for father_any_ihd, score for metabolic_syndrome, value for 1st_vst_map_2nd. AUC = 0.81.

**Table 5. Pre-eclampsia prediction in all subjects; with PPV criterion.**

| subclass | all | | | |
|---|---|---|---|---|
| PPV filter | on | | | |
| applied to preterm | no | | | |
| | | | | |
| #constituents | n=3 | n=4 | n=5 | n=6 |
| | | | | |
| number of panels | 1 | 3 | 28 | 52 |
| | | | | |
| bb_hdl | 0 | 0 | 0 | 7 |
| bmi | 0 | 0 | 5 | 19 |
| metabolic_syndrome | 0 | 0 | 9 | 20 |
| father_any_ihd | 1 | 3 | 28 | 52 |
| fh_pet | 0 | 0 | 0 | 0 |
| 1st_vst_dbp_2nd | 0 | 0 | 3 | 9 |
| 1st_vst_sbp_2nd | 1 | 1 | 9 | 19 |
| 1st_vst_map_2nd | 0 | 2 | 13 | 17 |
| 2nd_vst_map_2nd | 0 | 0 | 3 | 7 |
| ADAM12 | 0 | 2 | 16 | 24 |
| s-ENG | 0 | 1 | 12 | 44 |
| SPINT1 | 0 | 0 | 0 | 4 |
| HSPG2 | 0 | 0 | 0 | 3 |
| SEPP1 | 0 | 0 | 5 | 7 |
| IGFALS | 1 | 3 | 28 | 52 |
| MUC18 | 0 | 0 | 9 | 22 |
| ROBO4 | 0 | 0 | 0 | 5 |
| ENPP2 | 0 | 0 | 0 | 1 |
| S10A9 | 0 | 0 | 0 | 0 |

Representative but non-limiting particularly successful panels of those identified in Table 5 include:

T5.1: a 3-member panel consisting of: IGFALS level, score for father_any_ihd, value for 1st_vst_sbp_2nd. AUC = 0.75, PPV at 50% sensitivity = 23%, sensitivity at 20% PPV = 50%.

T5.2: a 4-member panel consisting of: IGFALS level, ADAM12 level, score of father_any_ihd, value of 1st_vst_map_2nd. AUC = 0.77, PPV at 50% sensitivity = 23%.

T5.3: a 4-member panel consisting of measurement of: IGFALS level, ADAM12 level, score for father_any_ihd, value for 1st_vst_map_2nd. Sensitivity at 20% PPV = 56%.

T5.4: a 5-member panel consisting of: IGFALS level, s-ENG level, MUC18 level, score for father_any_ihd, value for 1st_vst_map_2nd. AUC = 0.80.

T5.5: a 5-member panel consisting of: IGFALS level, s-ENG level, score for father_any_ihd, score for metabolic_syndrome, value for 1st_vst_dbp_2nd. PPV at 50% sensitivity = 26%.

T5.6: a 5-member panel consisting of: IGFALS level, s-ENG level, a score for father_any_ihd, a score for metabolic_syndrome, value for 1st_vst_dbp_2nd. Sensitivity at 20% PPV = 66%.

T5.7: a 6-member panel consisting of: IGFALS level, s-ENG level, MUC18 level, a score for father_any_ihd, a score for metabolic_syndrome, value for 1st_vst_map_2nd. AUC = 0.81.

T5.8: a 6-member panel consisting of: IGFALS level, a s-ENG level, a MUC18 level a score for father_any_ihd, a score for metabolic-syndrome, value for 1st_vst_sbp_2nd. PPV at 50% sensitivity = 28%.

T5.9: a 6-member panel consisting of: IGFALS level, s-ENG level, ADAM12 level, a score for father_any_ihd, a score for metabolic syndrome, value for 1st_vst_sbp_2nd. Sensitivity at 20% PPV = 70%.

**Table 6. Pre-eclampsia prediction in all subjects; no PPV criterion; application to preterm pre-eclampsia.**

| subclass | all | | | |
|---|---|---|---|---|
| PPV filter | off | | | |
| applied to preterm | yes | | | |
| #constituents | n=3 | n=4 | n=5 | n=6 |
| | | | | |
| number of panels | 0 | 0 | 15 | 16 |
| | | | | |
| **bb_hdl** | | | 0 | 0 |
| **bb_total_hdl_ratio** | | | 1 | 0 |
| **metabolic_syndrome** | | | 9 | 8 |
| **father_any_ihd** | | | 15 | 16 |
| **fh_pet** | | | 0 | 0 |
| **1st_vst_dbp_2nd** | | | 4 | 1 |
| **1st_vst_sbp_2nd** | | | 1 | 6 |
| **1st_vst_map_2nd** | | | 3 | 2 |
| **2nd_vst_map_2nd** | | | 7 | 7 |
| **ADAM12** | | | 7 | 13 |
| **s-ENG** | | | 8 | 16 |
| **SPINT1** | | | 3 | 8 |
| **HSPG2** | | | 0 | 0 |
| **SEPP1** | | | 1 | 1 |
| **IGFALS** | | | 15 | 16 |
| **MUC18** | | | 1 | 1 |
| **ROBO4** | | | 0 | 1 |

Representative but non-limiting particularly successful panels of those identified in Table 6 include:

T6.1: a 5-member panel consisting of: IGFALS level, s-ENG level a score for father_any_ihd, a score for metabolic_syndrome, value for 1st_vst_sbp_2nd. AUC pre-term PE = 0.89, AUC all PE = 0.79.

T6.2: a 6-member panel consisting of: IGFALS level, s-ENG level, ADAM12 level, a score for father_any_ihd, a score for metabolic_syndrome, a value for 1st_vst_sbp_2nd. AUC pre-term PE = 0.90, AUC all PE = 0.80.

**Table 7. Pre-eclampsia prediction in all subjects; with PPV criterion; application to preterm pre-eclampsia.**

| subclass | all | | | |
|---|---|---|---|---|
| PPV filter | on | | | |
| applied to preterm | yes | | | |
| | | | | |
| #constituents | n=3 | n=4 | n=5 | n=6 |
| | | | | |
| number of panels | 0 | 0 | 4 | 10 |
| | | | | |
| **metabolic_syndrome** | | | 4 | 6 |
| **father_any_ihd** | | | 4 | 10 |
| **fh_pet** | | | 0 | 0 |
| **1st_vst_dbp_2nd** | | | 0 | 0 |
| **1st_vst_sbp_2nd** | | | 1 | 6 |
| **1st_vst_map_2nd** | | | 2 | 1 |
| **2nd_vst_map_2nd** | | | 1 | 3 |
| **ADAM12** | | | 2 | 7 |
| **s-ENG** | | | 2 | 10 |
| **SPINT1** | | | 0 | 4 |
| **SEPP1** | | | 0 | 1 |
| **IGFALS** | | | 4 | 10 |
| **MUC18** | | | 0 | 1 |
| **ROBO4** | | | 0 | 1 |

Representative but non-limiting particularly successful panels of those identified in Table 7 include:

T7.1: a 5-member panel consisting of: IGFALS level, s-ENG level, a score for father_any_ihd, a score for metabolic_syndrome, value for 1st_vst_sbp_2nd. AUC pre-term PE = 0.89, AUC all PE = 0.79, PPV at 50% sensitivity = 25%, sensitivity at 20% PPV = 66%.

T7.2: a 6-member panel consisting of: IGFALS level, s-ENG level, ADAM12 level, a score for father_any_ihd, a score for metabolic_syndrome, value for 1st_vst_map_2nd. AUC pre-term PE = 0.90, AUC all PE = 0.81.

T7.3: a 6-member panel consisting of: IGFALS level, s-ENG level, SEPP1 level, a score for father_any_ihd, a score for metabolic syndrome, value for 2nd_vst_map_2nd. PPV at 50% sensitivity = 24%.

T7.4: a 6-member panel consisting of: IGFALS level, s-ENG level, ADAM12 level, a score for father_any_ihd, a score for metabolic_syndrome, value for 1st_vst_sbp_2nd. Sensitivity at 20% PPV = 70%.

**Table 8. Pre-eclampsia prediction in non-obese; no PPV criterion**

| subclass | non-obese | | | |
|---|---|---|---|---|
| PPV filter | off | | | |
| applied to preterm | no | | | |
| | | | | |
| #constituents | n=3 | n=4 | n=5 | n=6 |
| | | | | |
| number of panels | 6 | 24 | 37 | 50 |

(continued)

| | | | | |
|---|---|---|---|---|
| **metabolic_syndrome** | 0 | 0 | 2 | 8 |
| **father_any_ihd** | 6 | 24 | 37 | 50 |
| **fh_pet** | 0 | 0 | 0 | 0 |
| **1st_vst_dbp_2nd** | 1 | 6 | 10 | 16 |
| **1st_vst_sbp_2nd** | 2 | 6 | 10 | 8 |
| **1st_vst_map_2nd** | 2 | 6 | 9 | 10 |
| **2nd_vst_map_2nd** | 1 | 6 | 8 | 16 |
| **ADAM12** | 0 | 15 | 15 | 41 |
| **s-ENG** | 0 | 8 | 30 | 50 |
| **HSPG2** | 0 | 0 | 0 | 1 |
| **SEPP1** | 0 | 0 | 11 | 20 |
| **IGFALS** | 6 | 24 | 37 | 50 |
| **MUC18** | 0 | 0 | 8 | 14 |
| **ROBO4** | 0 | 1 | 8 | 16 |

Representative but non-limiting particularly successful panels of those identified in Table 8 include:
T8.1: a 3-member panel consisting of: IGFALS level, a score for father_any_ihd, value for 1st_vst_sbp_2nd. AUC = 0.77.
T8.2: a 4-member panel consisting of: IGFALS level, ADAM12 level a score for father_any_ihd, value for 1st_vst_sbp_2nd. AUC = 0.82.
T8.3: a 5-member panel consisting of: IGFALS level, SEPP1 level, ADAM12 level, a score for father_any_ihd, value for 1st_vst_sbp_2nd. AUC = 0.84.
T8.4: a 6-member panel consisting of: IGFALS level, MUC18 level, s-ENG level, ADAM12 level, a score for father_any_ihd, value for 1st_vst_map_2nd. AUC = 0.91.

**Table 9. Pre-eclampsia prediction in non-obese; with PPV criterion**

| subclass | non-obese | | | |
|---|---|---|---|---|
| PPV filter | on | | | |
| applied to preterm | no | | | |
| | | | | |
| #constituents | n=3 | n=4 | n=5 | n=6 |
| | | | | |
| number of panels | 1 | 15 | 33 | 48 |
| | | | | |
| **metabolic_syndrome** | 0 | 0 | 1 | 8 |
| **father_any_ihd** | 1 | 15 | 33 | 48 |
| **fh_pet** | 0 | 0 | 0 | 0 |
| **1st_vst_dbp_2nd** | 0 | 6 | 9 | 16 |
| **1st_vst_sbp_2nd** | 1 | 4 | 9 | 8 |
| **1st_vst_map_2nd** | 0 | 5 | 9 | 10 |
| **2nd_vst_map_2nd** | 0 | 0 | 6 | 14 |
| **ADAM12** | 0 | 11 | 13 | 39 |

(continued)

| s-ENG | 0 | 4 | 27 | 48 |
|---|---|---|---|---|
| HSPG2 | 0 | 0 | 0 | 1 |
| SEPP1 | 0 | 0 | 11 | 20 |
| IGFALS | 1 | 15 | 33 | 48 |
| MUC18 | 0 | 0 | 7 | 13 |
| ROBO4 | 0 | 0 | 7 | 15 |

Representative but non-limiting particularly successful panels of those identified in Table 9 include:

T9.1: a 3-member panel consisting of: IGFALS level, a score for father_any_ihd, value for 1st_vst_sbp_2nd. AUC = 0.77, PPV at 50% sensitivity = 21%, sensitivity at 20% PPV = 52%.

T9.2: a 4-member panel consisting of: IGFALS level, ADAM12 level, a score for father_any_ihd, value for 1st_vst_map_2nd. AUC = 0.82.

T9.3: a 4-member panel consisting of: IGFALS level, ADAM12 level, a score for father_any_ihd, value for 1st_vst_map_2nd. PPV at 50% sensitivity = 32%.

T9.4: a 4-member panel consisting of: IGFALS level, s-ENG level, a score for father_any_ihd, value for 1st_vst_sbp_2nd. Sensitivity at 20% PPV = 61%.

T9.5: a 5-member panel consisting of: IGFALS level, SEPP1 level, ADAM12 level, a score for father_any_ihd, value for 1st_vst_sbp_2nd. AUC = 0.84.

T9.6: a 5-member panel consisting of: IGFALS level, s-ENG level, ADAM12 level, a score for father_any_ihd , value for 1st_vst_sbp_2nd. PPV at 50% sensitivity = 34%.

T9.7: a 5-member panel consisting of: IGFALS level, SEPP1 level, ADAM12 level, a score for father_any_ihd, value for 1st_vst_sbp_2nd. Sensitivity at 20% PPV = 64%.

T9.8: a 6-member panel consisting of: IGFALS level, MUC18 level, s-ENG level, ADAM12 level a score for father_any_ihd, value for 1st_vst_map_2nd. AUC = 0.86.

T9.9: a 6-member panel consisting of: IGFALS level, SEPP1 level, s-ENG level, ADAM12 level, a score for father_any_ihd, value for 1st_vst_sbp_2nd. PPV at 50% sensitivity = 48%.

T9.10: a 6-member panel consisting of: IGFALS level, MUC18 level, s-ENG level, ADAM12 level a score for father_any_ihd, value for 1st_vst_map_2nd. Sensitivity at 20% PPV = 69%.

**Table 10. Pre-eclampsia prediction in non-obese; no PPV criterion; application to preterm pre-eclampsia**

| subclass | non-obese | | | |
|---|---|---|---|---|
| PPV filter | off | | | |
| applied to preterm | yes | | | |
| | | | | |
| #constituents | n=3 | n=4 | n=5 | n=6 |
| number of panels | 1 | 12 | 25 | 26 |
| | | | | |
| metabolic_syndrome | 0 | 0 | 2 | 8 |
| father_any_ihd | 1 | 12 | 25 | 26 |
| fh_pet | 0 | 0 | 0 | 0 |
| 1st_vst_dbp_2nd | 0 | 3 | 8 | 10 |
| 1st_vst_sbp_2nd | 1 | 4 | 8 | 6 |
| 1st_vst_map_2nd | 0 | 3 | 6 | 5 |
| 2nd_vst_map_2nd | 0 | 2 | 3 | 5 |
| ADAM12 | 0 | 4 | 9 | 22 |
| s-ENG | 0 | 8 | 24 | 26 |

(continued)

| | | | | |
|---|---|---|---|---|
| **SEPP1** | 0 | 0 | 1 | 3 |
| **IGFALS** | 1 | 12 | 25 | 26 |
| **MUC18** | 0 | 0 | 7 | 11 |
| **ROBO4** | 0 | 0 | 7 | 8 |

Representative but non-limiting particularly successful panels of those identified in Table 10 include:
T10.1: a 3-member panel consisting of: IGFALS level, a score for father_any_ihd, value for 1st_vst_sbp_2nd. AUC preterm PE = 0.86, AUC all PE = 0.76.
T10.2: a 4-member panel consisting of: IGFALS level, s-ENG level, a score for father_any_ihd, value for 1st_vst_sbp_2nd. AUC pre-term PE = 0.92, AUC all PE = 0.80.
T10.3: a 5-member panel consisting of: IGFALS level, MUC18 level, s-ENG level a score for father_any_ihd, value for 1st_vst_sbp_2nd. AUC pre-term PE = 0.95, AUC all PE = 0.84.
T10.4: a 6-member panel consisting of: IGFALS level, MUC18 level, s-ENG level, ADAM12 level, a score for father_any_ihd, value for 1st_vst_map_2nd. AUC pre-term PE = 0.96, AUC all PE = 0.86.

**Table 11. Pre-eclampsia prediction in non-obese; with PPV criterion; application to preterm pre-eclampsia.**

| subclass | non-obese | | | |
|---|---|---|---|---|
| PPV filter | on | | | |
| applied to preterm | yes | | | |
| | | | | |
| #constituents | n=3 | n=4 | n=5 | n=6 |
| | | | | |
| number of panels | | 8 | 21 | 25 |
| | | | | |
| **metabolic_syndrome** | | 0 | 1 | 8 |
| **father_any_ihd** | | 8 | 21 | 25 |
| **fh_pet** | | 0 | 0 | 0 |
| **1st_vst_dbp_2nd** | | 3 | 7 | 10 |
| **1st_vst_sbp_2nd** | | 3 | 7 | 6 |
| **1st_vst_map_2nd** | | 2 | 6 | 5 |
| **2nd_vst_map_2nd** | | 0 | 1 | 4 |
| **ADAM12** | | 4 | 7 | 21 |
| **s-ENG** | | 4 | 21 | 25 |
| **SEPP1** | | 0 | 1 | 3 |
| **IGFALS** | | 8 | 21 | 25 |
| **MUC18** | | 0 | 6 | 10 |
| **ROBO4** | | 0 | 6 | 8 |

Representative but non-limiting particularly successful panels of those identified in Table 11 include:

T11.1: a 4-member panel consisting of: IGFALS level, s-ENG level, a score for father_any_ihd, value for 1st_vst_sbp_2nd. AUC pre-term PE = 0.92, AUC all PE = 0.80, sensitivity at 20% PPV = 61%.

T11.2: a 4-member panel consisting of: IGFALS level, ADAM12 level, a score for father_any_ihd, value for 1st_vst_map_2nd. PPV at 50% sensitivity = 32%.

T11.3: a 5-member panel consisting of: IGFALS level, MUC18 level, s-ENG level, a score for father_any_ihd, value for 1st_vst_sbp_2nd. AUC pre-term PE = 0.95, AUC all PE = 0.84.

T11.4: a 5-member panel consisting of: IGFALS level, s-ENG level, ADAM12 level, a score for father_any_ihd,, value for 1st_vst_sbp_2nd. PPV at 50% sensitivity = 34%.

T11.5: a 5-member panel consisting of: IGFALS level, s-ENG level, ROBO4 level, a score for father_any_ihd , value for 1st_vst_map_2nd. Sensitivity at 20% PPV = 63%.

T11.6: a 6-member panel consisting of: IGFALS level, MUC18 level, s-ENG level, ADAM12 level, a score for father_any_ihd , value for 1st_vst_map_2nd. AUC pre-term PE = 0.96, AUC all PE = 0.86, sensitivity at 20% PPV = 69%.

T11.7: a 6-member panel consisting of: IGFALS level, SEPP1 level, s-ENG level, ADAM12 level a score for father_any_ihd, value for 1st_vst_sbp_2nd. PPV at 50% sensitivity = 48%.

## Example 6: Further results using case-control set of Table 3

The data and analyses in this example have also been obtained using the combined case-control population as captured in Table 3. This example further particularly focuses on prediction of pre-eclampsia at about 20 weeks of gestation.

In this example, the following non-limiting criteria were applied to consider or classify pre-eclampsia prediction models as successful:

1. For generic panels:

- AUC $\geq$ 0.75, panels consisting of 3, 4, 5 or 6 members (biomarkers, clinical parameters), all members have to add significantly to the panel

2. For "rule-in" panels:

a. AUC $\geq$ 0.75, panels consisting of 3, 4, 5 or 6 members (biomarkers, clinical parameters), all members have to add significantly to the panel; and

b. PPV criterion: the ROC curve needs to have points in the PPV $\geq$0.2 zone with a sensitivity $\geq$ 0.5 ($\geq$50 % detection rate)

i. Panels were ranked for maximum PPV at 0.5 sensitivity

ii. Panels ranked for maximum sensitivity at 0.20 PPV.

3. For preterm pre-eclampsia prediction:

- the panels as selected under item 1 and 2 above are applied to predict subgroup preterm pre-eclampsia (i.e., to classify preterm pre-eclampsia vs. all non-pre-eclampsia controls) without modification of the weighing factors for the members of the panels (biomarkers, parameters). Panels with significantly better AUC values for preterm pre-eclampsia prediction than for all pre-eclampsia prediction (p-value AUC difference $\leq$ 0.05) were considered successful.

4. For all subjects and for non-obese subjects:

- the criteria as explained under items 1, 2 or 3 were applied to pre-eclampsia prediction both in all subjects and in non-obese subjects.

The resulting data is summarised in tables 12 to 19 below, in which:

- the field "subclass" indicates the subjects for which the prediction was made, i.e., for all subjects ("all") or for non-obese subjects ("non-obese");

- the field "PPV filter" indicates whether the panels were evaluated for their suitability as "rule-in" panels ("on") or not ("off");

- the field "applied to preterm" indicates whether the panels were applied to prediction of pre-term pre-eclampsia ("yes") or to all pre-eclampsia ("no");

- the field "#constituents" denotes the number of constituents or members (biomarkers, clinical parameters) of which a panel consists;

- the field "number of panels" specifies the number of panels considered as successful according to the aforementioned criteria upon application of the parameters defined in the above-explained fields, as specified in each table;

- the subsequent fields of each table list members (markers, clinical parameters) appearing in the panels, and specify the number of successful panels in which each respective marker or parameter was present; the higher the number of panels in which a given marker or parameter was present, the more preferred such marker or parameter may be for inclusion into successful panels embodying the principles herein disclosed;

Following each table, several exemplary but non-limiting panels which had the best performance in the tests according to that table are specified.

Compared with the experiments using the case-control population of Table 1, the relative importance of the maternal history parameter 'mother or sister with previous PE and/or father with ischemic heart disease' (*fh_petxcardio*) has been somewhat reduced in the population of Table 3, allowing to define panels not containing the score for this parameter.

All panels in this example thus comprise the measurement of the level of insulin-like growth factor-binding protein complex acid labile subunit (IGFALS) and measurement of blood pressure.

**Table 12. Pre-eclampsia prediction in all subjects; no PPV criterion**

| subclass | all | | | |
|---|---|---|---|---|
| PPV filter | off | | | |
| applied to preterm | no | | | |
| #constituents | n=3 | n=4 | n=5 | n=6 |
| number of panels | 5 | 119 | 170 | 264 |
| **bb_hdl** | 1 | 19 | 35 | 58 |
| **bb_total_hdl_ratio** | 0 | 12 | 13 | 35 |
| **bb_trig** | 0 | 0 | 0 | 0 |
| **bmi** | 0 | 18 | 74 | 118 |
| **metabolic_syndrome** | 2 | 25 | 29 | 71 |
| **father_any_ihd** | 0 | 0 | 0 | 0 |
| **fh_pet** | 0 | 0 | 0 | 0 |
| **1st_vst_dbp_2nd** | 1 | 30 | 54 | 74 |
| **1st_vst_sbp_2nd** | 0 | 35 | 43 | 72 |

(continued)

| | | | | |
|---|---|---|---|---|
| **1st_vst_map_2nd** | 4 | 35 | 47 | 82 |
| **2nd_vst_map_2nd** | 0 | 19 | 26 | 36 |
| **ADAM12** | 1 | 75 | 91 | 217 |
| **s-ENG** | 0 | 43 | 111 | 239 |
| **LCAT** | 0 | 0 | 1 | 1 |
| **SPINT1** | 0 | 10 | 20 | 85 |
| **HSPG2** | 0 | 0 | 16 | 28 |
| **SEPP1** | 1 | 12 | 29 | 44 |
| **IGFALS** | 5 | 119 | 170 | 264 |
| **MUC18** | 0 | 20 | 41 | 80 |
| **ROBO4** | 0 | 4 | 32 | 53 |
| **ENPP2** | 0 | 0 | 18 | 27 |

Representative but non-limiting particularly successful panels of those identified in Table 12 include:
T12.1: a 3-member panel consisting of: IGFALS level, a score for metabolic_syndrome, value for 1st_vst_sbp_2nd. AUC = 0.75.
T12.2: a 4-member panel consisting of: IGFALS level, MUC18 level, s-ENG level, value for 1st_vst_map_2nd. AUC = 0.78.
T12.3: a 5-member panel consisting of: IGFALS level, MUC18 level, s-ENG level, a score for metabolic_syndrome, value for 1st_vst_map_2nd. AUC = 0.80.
T12.4: a 6-member panel consisting of: IGFALS level, MUC18 level, SEPP1 level, s-ENG level, a score for metabolic_syndrome, value for 1st_vst_map_2nd. AUC = 0.81.

**Table 13. Pre-eclampsia prediction in all subjects; with PPV criterion**

| | | | | |
|---|---|---|---|---|
| subclass | all | | | |
| PPV filter | on | | | |
| applied to preterm | no | | | |
| #constituents | n=3 | n=4 | n=5 | n=6 |
| number of panels | n=0 | n=6 | n=21 | n=77 |
| **bb_hdl** | | 0 | 3 | 15 |
| **bb_total_hdl_ratio** | | 0 | 0 | 7 |
| **bb_trig** | | 0 | 0 | 0 |
| **bmi** | | 1 | 5 | 39 |
| **metabolic_syndrome** | | 3 | 12 | 32 |
| **father_any_ihd** | | 0 | 0 | 0 |
| **fh_pet** | | 0 | 0 | 0 |
| **1st_vst_dbp_2nd** | | 1 | 5 | 15 |
| **1st_vst_sbp_2nd** | | 4 | 7 | 30 |
| **1st_vst_map_2nd** | | 1 | 7 | 29 |
| **2nd_vst_map_2nd** | | 0 | 2 | 3 |
| **ADAM12** | | 1 | 5 | 56 |
| **s-ENG** | | 5 | 17 | 75 |
| **SPINT1** | | 1 | 1 | 17 |
| **HSPG2** | | 0 | 4 | 12 |
| **SEPP1** | | 0 | 4 | 4 |
| **IGFALS** | | 6 | 21 | 77 |
| **MUC18** | | 1 | 7 | 35 |
| **ROBO4** | | 0 | 5 | 14 |
| **ENPP2** | | 0 | 0 | 2 |

Representative but non-limiting particularly successful panels of those identified in Table 13 include:
T13.1: a 4-member panel consisting of: IGFALS level, s-ENG level, a score for metabolic_syndrome, value for 1st_vst_map_2nd. AUC = 0.77, PPV at 50% sensitivity = 23%.
T13.2: a 4-member panel consisting of: IGFALS level, s-ENG level, a score for metabolic_syndrome, value for 1st_vst_dbp_2nd. Sensitivity at 20% PPV = 61%.
T13.3: a 5-member panel consisting of: IGFALS level, MUC18 level, s-ENG level, a score for metabolic syndrome, value for 1st_vst_map_2nd. AUC = 0.80.
T13.4: a 5-member panel consisting of: IGFALS level, ROBO4 level, S-ENG level, a score for metabolic_syndrome, value for 1st_vst_map_2nd. PPV at 50% sensitivity = 24%.
T13.5: a 5-member panel consisting of: IGFALS level, ROBO4 level, s-ENG level, a score for metabolic_syndrome, value for 1st_vst_map_2nd. Sensitivity at 20% PPV = 61%.
T13.6: a 6-member panel consisting of: IGFALS level, MUC18 level, SEPP1 level, s-ENG level, a score for metabolic_syndrome, value for 1st_vst_map_2nd. AUC = 0.81.
T13.7: a 6-member panel consisting of: IGFALS level, MUC18 level, ADAM12 level, s-ENG level, a score for metabolic_syndrome, value for 1st_vst_map_2nd. PPV at 50% sensitivity = 27%.
T13.8: a 6-member panel consisting of: IGFALS level, MUC18 level, s-ENG level, ADAM12 level a score form metabolic_syndrome, value for 1st_vst_map_2nd. Sensitivity at 20% PPV = 63%.

**Table 14. Pre-eclampsia prediction in all subjects; no PPV criterion; application to preterm pre-eclampsia**

| subclass | all | | | |
|---|---|---|---|---|
| PPV filter | off | | | |
| applied to preterm | yes | | | |
| | | | | |
| #constituents | n=3 | n=4 | n=5 | n=6 |
| | | | | |
| number of panels | n=0 | n=24 | n=35 | n=80 |
| | | | | |
| **bb_hdl** | | 0 | 0 | 16 |
| **bb_total_hdl_ratio** | | 1 | 0 | 15 |
| **bb_trig** | | 0 | 0 | 0 |
| **bmi** | | 0 | 0 | 1 |
| **metabolic_syndrome** | | 12 | 13 | 23 |
| **father_any_ihd** | | 0 | 0 | 0 |
| **fh_pet** | | 0 | 0 | 0 |
| **1st_vst_dbp_2nd** | | 5 | 7 | 21 |
| **1st_vst_sbp_2nd** | | 5 | 9 | 19 |
| **1st_vst_map_2nd** | | 6 | 8 | 21 |
| **2nd_vst_map_2nd** | | 8 | 11 | 19 |
| **ADAM12** | | 8 | 23 | 79 |
| **s-ENG** | | 16 | 31 | 80 |
| **SPINT1** | | 6 | 19 | 71 |
| **SEPP1** | | 4 | 9 | 11 |
| **IGFALS** | | 24 | 35 | 80 |
| **MUC18** | | 1 | 4 | 17 |
| **ROBO4** | | 0 | 6 | 6 |
| **ENPP2** | | 0 | 0 | 1 |

Representative but non-limiting particularly successful panels of those identified in Table 14 include:
T14.1: a 4-member panel consisting of: IGFALS level, s-ENG level, a score for metabolic-syndrome, value for 1st_vst_map_2nd. AUC pre-term PE = 0.88, AUC all PE = 0.77.
T14.2: a 5-member panel consisting of: IGFALS levels, SPINT1 level, s-ENG level, ADAM12 level, value for 2nd_vst_map_2nd. AUC pre-term PE = 0.89, AUC all PE = 0.75.
T14.3: a 6-member panel consisting of: IGFALS level, SPINT1 level, s-ENG level, ADAM12 level, a score for metabolic_syndrome, value for 1st_vst_map_2nd . AUC pre-term PE = 0.91, AUC all PE = 0.80.

**Table 15. Pre-eclampsia prediction in all subjects; with PPV criterion; application to preterm pre-eclampsia**

| subclass | all | | | |
|---|---|---|---|---|
| PPV filter | on | | | |
| applied to preterm | yes | | | |
| | | | | |
| #constituents | n=3 | n=4 | n=5 | n=6 |
| number of panels | n=0 | n=4 | n=8 | n=17 |
| | | | | |
| bb_hdl | | 0 | 0 | 0 |
| bb_total_hdl_ratio | | 0 | 0 | 1 |
| bb_trig | | 0 | 0 | 0 |
| bmi | | 0 | 0 | 1 |
| metabolic_syndrome | | 3 | 6 | 9 |
| father_any_ihd | | 0 | 0 | 0 |
| fh_pet | | 0 | 0 | 0 |
| 1st_vst_dbp_2nd | | 1 | 1 | 1 |
| 1st_vst_sbp_2nd | | 2 | 3 | 8 |
| 1st_vst_map_2nd | | 1 | 2 | 5 |
| 2nd_vst_map_2nd | | 0 | 2 | 3 |
| ADAM12 | | 0 | 1 | 17 |
| s-ENG | | 4 | 7 | 17 |
| SPINT1 | | 1 | 1 | 15 |
| SEPP1 | | 0 | 4 | 2 |
| IGFALS | | 4 | 8 | 17 |
| MUC18 | | 0 | 1 | 5 |
| ROBO4 | | 0 | 4 | 1 |

Representative but non-limiting particularly successful panels of those identified in Table 15 include:

T15.1: a 4-member panel consisting of: IGFALS level, s-ENG level, a score for metabolic_syndrome, value for 1st_vst_map_2nd. AUC pre-term PE = 0.88, AUC all PE = 0.77, PPV at 50% sensitivity = 23%, sensitivity at 20% PPV = 66%.

T15.2: a 4-member panel consisting of: IGFALS level, s-ENG level, a score for metabolic_syndrome, value for 1st_vst_sbp_2nd . Sensitivity at 20% PPV = 61%.

T15.3: a 5-member panel consisting of: IGFALS level, ROBO4 level, s-ENG level, a score for metabolic syndrome, value for 1st_vst_map_2nd . AUC pre-term PE = 0.89, AUC all PE =0.79, sensitivity at 20% PPV = 61%.

T15.4: a 5-member panel consisting of: IGFALS level, ROBO4 level, s-ENG level, a score for metabolic_syndrome, value for 1st_vst_map_2nd. PPV at 50% sensitivity = 24%.

T15.5: a 6-member panel consisting of: IGFALS level, SPINT1 level, s-ENG level, ADAM12 level, a score for metabolic_syndrome, value for 1st_vst_map_2nd. AUC pre-term PE = 0.91, AUC all PE = 0.80.

T15.6: a 6-member panel consisting of: IGFALS level, SPINT1 level, s-ENG level, ADAM12 level, a score for metabolic_syndrome, value for 1st_vst_sbp_2nd. PPV at 50% sensitivity = 25%.

T15.7: a 6-member panel consisting of: IGFALS level, SPINT1 level, s_ENG level, ADAM12 level, a score for metabolic_syndrome, value for 1st_vst_map_2nd. Sensitivity at 20% PPV = 60%.

**Table 16. Pre-eclampsia prediction in non-obese; no PPV criterion**

| subclass | non-obese | | | |
|---|---|---|---|---|
| PPV filter | off | | | |
| applied to preterm | no | | | |
| #constituents | n=3 | n=4 | n=5 | n=6 |
| number of panels | 24 | 69 | 76 | 43 |
| bb_hdl | 0 | 4 | 0 | 0 |
| bb_total_hdl_ratio | 0 | 0 | 0 | 0 |

(continued)

| | | | | |
|---|---|---|---|---|
| bb_trig | 0 | 0 | 0 | 0 |
| bmi | 0 | 0 | 0 | 0 |
| metabolic_syndrome | 0 | 3 | 10 | 9 |
| father_any_ihd | 0 | 0 | 0 | 0 |
| fh_pet | 0 | 0 | 0 | 0 |
| 1st_vst_dbp_2nd | 6 | 19 | 23 | 19 |
| 1st_vst_sbp_2nd | 6 | 18 | 16 | 6 |
| 1st_vst_map_2nd | 6 | 15 | 18 | 8 |
| 2nd_vst_map_2nd | 6 | 17 | 19 | 10 |
| ADAM12 | 16 | 42 | 65 | 43 |
| s-ENG | 8 | 43 | 72 | 42 |
| SPINT1 | 0 | 0 | 12 | 12 |
| SEPP1 | 0 | 16 | 25 | 30 |
| IGFALS | 12 | 36 | 32 | 13 |
| MUC18 | 0 | 8 | 16 | 11 |
| ROBO4 | 0 | 22 | 28 | 24 |
| ENPP2 | 0 | 0 | 0 | 1 |

Representative but non-limiting particularly successful panels of those identified in Table 16 include:
T16.1: a 3-member panel consisting of: IGFALS level, ADAM12 level, value for 1st_vst_map_2nd. AUC = 0.79.
T16.2: a 4-member panel consisting of: IGFALS level, MUC18 level, s-ENG level, value for 1st_vst_map_2nd. AUC = 0.81.
T16.3: a 5-member panel consisting of: IGFALS level, MUC18 level, s-ENG level, ADAM 12 level, value for 1st_vst_map_2nd. AUC = 0.83.
T16.4: a 6-member panel consisting of: IGFALS level, MUC18 level, SEPP1 level, s_ENG level, ADAM12 level, value for 1st_vst_map_2nd. AUC = 0.85.

**Table 17. Pre-eclampsia prediction in non-obese; with PPV criterion**

| subclass | non-obese | | | |
|---|---|---|---|---|
| PPV filter | on | | | |
| applied to preterm | no | | | |
| #constituents | n=3 | n=4 | n=5 | n=6 |
| number of panels | | 11 | 40 | 33 |
| bb_hdl | | 1 | 0 | 0 |
| bb_total_hdl_ratio | | 0 | 0 | 0 |
| bb_trig | | 0 | 0 | 0 |
| bmi | | 0 | 0 | 0 |
| metabolic_syndrome | | 0 | 2 | 4 |
| father_any_ihd | | 0 | 0 | 0 |
| fh_pet | | 0 | 0 | 0 |
| 1st_vst_dbp_2nd | | 3 | 12 | 14 |
| 1st_vst_sbp_2nd | | 2 | 10 | 6 |
| 1st_vst_map_2nd | | 6 | 13 | 8 |
| 2nd_vst_map_2nd | | 0 | 5 | 5 |
| ADAM12 | | 7 | 36 | 33 |
| s-ENG | | 11 | 40 | 33 |
| SPINT1 | | 0 | 11 | 10 |
| SEPP1 | | 1 | 10 | 24 |
| IGFALS | | 11 | 40 | 33 |
| MUC18 | | 0 | 12 | 10 |
| ROBO4 | | 2 | 9 | 18 |

Representative but non-limiting particularly successful panels of those identified in Table 17 include:

T17.1: a 4-member panel consisting of: IGFALS level, s-ENG level, ADAM12 level, value for 1st_vst_sbp_2nd. AUC = 0.81.

T17.2: a 4-member panel consisting of: IGFALS level, ROBO4 level, s-ENG level, value for 1st_vst_map_2nd. PPV at 50% sensitivity = 29%, sensitivity at 20% PPV = 58%.

T17.3: a 5-member panel consisting of: IGFALS level, MUC18 level, s_ENG level, ADAM 12 level, value for 1st_vst_map_2nd. AUC = 0.84.

T17.4: a 5-member panel consisting of: IGFALS level, SPINT1 level, s-ENG level, ADAM 12 level, value for 1st_vst_map_2nd. PPV at 50% sensitivity = 32%.

T17.5: a 5-member panel consisting of: IGFALS level, SPINT1 level, s-ENG level, ADAM12 level, value for 1st_vst_map_2nd. Sensitivity at 20% PPV = 63%.

T17.6: a 6-member panel consisting of: IGFALS level, MUC18 level, SEPP1 level, ADAM12 level, s-ENG level, value for 1st_vst_map_2nd. AUC = 0.85.

T17.7: a 6-member panel consisting of: IGFALS level, ROBO4 level, SEPP1 level, s-ENG level, ADAM12 level, value for 1st_vst_map_2nd. PPV at 50% sensitivity = 38%.

T17.8: a 6-member panel consisting of: IGFALS level, MUC18 level, SEPP1 level, s-ENG level, ADAM12 level, value for 1st_vst_map_dbp_2nd. Sensitivity at 20% PPV = 66%.

**Table 18. Pre-eclampsia prediction in non-obese; no PPV criterion; application to preterm pre-eclampsia**

| subclass | non-obese | | | |
|---|---|---|---|---|
| PPV filter | off | | | |
| applied to preterm | yes | | | |
| | | | | |
| #constituents | n=3 | n=4 | n=5 | n=6 |
| number of panels | 9 | 41 | 56 | 30 |
| | | | | |
| **bb_hdl** | 0 | 4 | 0 | 0 |
| **bb_total_hdl_ratio** | 0 | 0 | 0 | 0 |
| **bb_trig** | 0 | 0 | 0 | 0 |
| **bmi** | 0 | 0 | 0 | 0 |
| **metabolic_syndrome** | 0 | 3 | 10 | 9 |
| **father_any_ihd** | 0 | 0 | 0 | 0 |
| **fh_pet** | 0 | 0 | 0 | 0 |
| **1st_vst_dbp_2nd** | 2 | 13 | 19 | 14 |
| **1st_vst_sbp_2nd** | 3 | 11 | 15 | 6 |
| **1st_vst_map_2nd** | 2 | 10 | 16 | 4 |
| **2nd_vst_map_2nd** | 2 | 7 | 6 | 6 |
| **ADAM12** | 1 | 16 | 50 | 30 |
| **s-ENG** | 8 | 39 | 54 | 30 |
| **SPINT1** | 0 | 0 | 12 | 12 |
| **SEPP1** | 0 | 5 | 11 | 17 |
| **IGFALS** | 9 | 41 | 56 | 30 |
| **MUC18** | 0 | 8 | 12 | 11 |
| **ROBO4** | 0 | 7 | 19 | 11 |

Representative but non-limiting particularly successful panels of those identified in Table 18 include:

T18.1: a 3-member panel consisting of: IGFALS level, s-ENG level, value for 1st_vst_sbp_2nd. AUC pre-term PE = 0.90, AUC all PE = 0.78.

T18.2: a 4-member panel consisting of: IGFALS level, MUC18 level, s-ENG level, value for 1st_vst_map_2nd. AUC pre-term PE = 0.93, AUC all PE = 0.81.

T18.3: a 5-member panel consisting of: IGFALS level, MUC18 level, s-ENG level, ADAM12 level, value for 1st_vst_map_2nd. AUC pre-term PE = 0.95, AUC all PE = 0.84.

T18.4: a 6-member panel consisting of: IGFALS level, MUC18 level, SPINT1 level, ENG level, ADAM12 level, value for 1st_vst_dbp_2nd. AUC pre-term PE = 0.96, AUC all PE = 0.84.

**Table 19. Pre-eclampsia prediction in non-obese; with PPV criterion; application to preterm pre-eclampsia.**

| subclass | non-obese | | | |
|---|---|---|---|---|
| PPV filter | on | | | |
| applied to preterm | yes | | | |
| #constituents | n=3 | n=4 | n=5 | n=6 |
| number of panels | | 11 | 34 | 23 |
| **bb_hdl** | | 1 | 0 | 0 |
| **bb_total_hdl_ratio** | | 0 | 0 | 0 |
| **bb_trig** | | 0 | 0 | 0 |
| **bmi** | | 0 | 0 | 0 |
| **metabolic_syndrome** | | 0 | 2 | 4 |
| **father_any_ihd** | | 0 | 0 | 0 |
| **fh_pet** | | 0 | 0 | 0 |
| **1st_vst_dbp_2nd** | | 3 | 10 | 10 |
| **1st_vst_sbp_2nd** | | 2 | 10 | 6 |
| **1st_vst_map_2nd** | | 6 | 12 | 4 |
| **2nd_vst_map_2nd** | | 0 | 2 | 3 |
| **ADAM12** | | 7 | 31 | 23 |
| **s-ENG** | | 11 | 34 | 23 |
| **SPINT1** | | 0 | 11 | 10 |
| **SEPP1** | | 1 | 6 | 14 |
| **IGFALS** | | 11 | 34 | 23 |
| **MUC18** | | 0 | 10 | 10 |
| **ROBO4** | | 2 | 8 | 8 |

Representative but non-limiting particularly successful panels of those identified in Table 19 include:

T19.1: a 4-member panel consisting of: IGFALS level, s-ENG level, ADAM12 level, value for 1st_vst_map_2nd. AUC pre-term PE = 0.91, AUC all PE = 0.80.

T19.2: a 4-member panel consisting of: IGFALS level, ROBO4 level, s_ENG level, value for 1st_vst_map_2nd. PPV at 50% sensitivity = 29%, sensitivity at 20% PPV = 58%.

T19.3: a 5-member panel consisting of: IGFALS level, MUC18 level, s-ENG level, ADAM12 level, value for 1st_vst_map_2nd. AUC pre-term PE = 0.95, AUC all PE = 0.84.

T19.4: a 5-member panel consisting of: IGFALS level, SPINT level, s-ENG, ADAM12 level, value for 1st_vst_map_2nd. PPV at 50% sensitivity = 32%.

T19.5: a 5-member panel consisting of: IGFALS level, SPINT level, s-ENG level, ADAM12 level, value for 1st_vst_map_2nd. Sensitivity at 20% PPV = 63%.

T19.6: a 6-member panel consisting of: IGFALS level, MUC18 level, SPINT1 level, s_ENG level, ADAM12 level, value for 1st_vst_sbp_2nd. AUC pre-term PE = 0.96, AUC all PE = 0.84, PPV at 50% sensitivity = 34%.

T19.7: a 6-member panel consisting of: IGFALS level, MUC18 level, SEPP1 level, s-ENG level, ADAM12 level, value for 1st_vst_dbp_2nd. Sensitivity at 20% PPV = 66%.

**Claims**

1. A method for the prediction of preeclampsia (PE) in a subject comprising testing and evaluating in a sample that has been obtained from said subject a test panel, wherein the sample is whole blood, plasma or serum, the test panel comprising:

   - measurement of the level of insulin-like growth factor-binding protein complex acid labile subunit (IGFALS),
   - measurement of blood pressure,
   - measurement of the level of Kunitz-type protease inhibitor 1 (SPINT1), and
   - measurement of the level of either i) disintegrin and metalloproteinase domain containing protein 12 (ADAM12) or ii) s-Endoglin (ENG or s-ENG),

   wherein the measurements of the biomarkers and parameter of the test panel is used to establish a biomarker-and-parameter profile, which is compared with a corresponding multiparameter reference value for prediction of PE.

2. The method according to claim 1, wherein the test panel comprises measurement of the level of s-Endoglin and measurement of the level of ADAM12.

3. The method according to claim 1 or 2, wherein the test panel further comprises at least one, more preferably at least two or even at least three of measurement of the level of selenoprotein P (SEPP1), measurement of the level of quiescin Q6 (QSOX1), measurement of the level of peroxiredoxin-2 (PRDX2), measurement of blood glucose level, measurement of body mass index (BMI), a score for the maternal history parameter 'mother or sister of subject has/had preeclampsia' ("fh pet"), a value for the parameter 'high density lipoprotein level' ("bb hdr), a value for the parameter 'ratio of total cholesterol to high density lipoprotein' ("bb total hdt ratio"), a score for the parameter metabolic syndrome, a value for the parameter triglycerides level ("bb trip"), measurement of the level of vascular endothelial growth factor receptor 3 (FLT4), measurement of the level of lysosomal Pro-X carboxypeptidase (PRCP), measurement of the level of peroxiredoxin-1 (PRDX1), measurement of the level of leucyl-cystinyl aminopeptidase (LNPEP), measurement of the level of tenascin-X (TNXB), measurement of the level of basement membrane-specific heparan sulfate proteoglycan core protein (HSPG2), measurement of the level of cell surface glycoprotein (CD146, MUC18, MCAM), measurement of the level of phosphatidylinositol-glycan-specific phospholipase D (GPLD1), measurement of the level of collagen alpha-3(VI) chain (COL6A3), measurement of the level of hepatocyte growth factor-like protein (MST1), measurement of the level of probable G-protein coupled receptor 126 (GPR126), measurement of the level of intercellular adhesion molecule 3 (ICAM3), measurement of the level of C-reactive protein (CRP), measurement of the level of phosphatidylcholine-sterol acyltransferase (LCAT), measurement of the level of roundabout homolog 4 (ROBO4), measurement of the level of ectonucleotide pyrophosphatase/phosphodiesterase family member 2 (ENPP2), and measurement of the level of protein S100-A9 (S100A9).

4. The method according to claim 3, wherein the measurement of the level of SEPP1 is supplemented or substituted by the measurement of the level of selenium.

**Patentansprüche**

1. Verfahren zur Vorhersage von Präeklampsie (PE) bei einem Patienten, umfassend Testen und Beurteilen eines Testfeldes in einer Probe dieses Patienten, wobei es sich bei dieser Probe um Vollblut, Plasma oder Serum handeln kann, wobei das Testfeld umfasst:

   - Messung des Spiegels der säurelabilen Untergruppe des insulin-ähnlichen Wachstumsfaktor-bindenden Proteinkomplexes (IGFALS),
   - Messung des Blutdrucks,
   - Messung des Kunitz-Typ Proteaseinhibitors 1 (SPINT1)-Spiegels und
   - Messung des Spiegels von entweder i) der Protein 12 enthaltenden Disintegrin- und Metalloproteinase-Domäne (ADAM12) oder ii) s-Endoglin (ENG oder s-ENG),

   wobei die Messungen der Biomarker und Parameter des Testfeldes verwendet werden, um ein Biomarker- und Parameter- Profil zu erstellen, das mit einem entsprechenden Multiparameter- Referenzwert zur Vorhersage von PE verglichen wird.

**2.** Verfahren gemäß Anspruch 1, wobei das Testfeld Messungen des s-Endoglin - Spiegels und Messungen des ADAM12-Spiegels umfasst.

**3.** Verfahren nach Anspruch 1 oder 2, wobei das Testfeld weiterhin: mindestens eine, besser noch zwei oder sogar drei Messungen des Selenoprotein P (SEPP1) - Spiegels, Messung des Quiescin Q6 (QSOX1)-Spiegels, Messung des Peroxiredoxin-2 (PRDX2)-Spiegels, Messungen des Blutzuckerspiegels, Messung des Body-Mass-Indexes (BMI), eine Punktzahl für den Parameter Vorgeschichte der weiblichen Verwandten 'Mutter oder Schwester der Patientien hat/ hatte Präeklampsie' ("fh pet"), einen Wert für den Parameter 'High-Density-Lipoprotein-Spiegel' ("bb hdr), einen Wert für den Parameter 'Verhältnis Gesamtcholesterin zu High-Density-Lipoprotein' ("bb total hdt ratio"), eine Punktzahl für den Parameter metabolisches Syndrom, einen Wert für den Parameter Triglycerid-Spiegel ("bb trip"), Messung des Vascular Endothelial Growth Factor Rezeptor 3 (FLT4)-Spiegels, Messung des lysosomalen Pro-X Carboxypeptidase (PROP)-Spiegels, Messung des Peroxiredoxin-1 (PRDX1)-Spiegels, Messung des Leucyl-Cystinyl Aminopeptidase (LNPEP) -Spiegels, Messung des Tenascin-X (TNXB)-Spiegels, Messung des Basalmembran-spezifischen Heparansulfat-Proteoglycan-Kernprotein (HSPG2)-Spiegels, Messung des Zelloberflächen-Glykoprotein (CD146, MUC18, MCAM)-Spiegels, Messung des Phosphatidylinositol-glykanspezifischen Phospholipase D (GPLD1)-Spiegels, Messung des Kollagen Typ IV, alpha 3 (COL6A3)-Spiegels, Messung des Hepatozyten-Wachstumsfaktor-ähnlichen Protein (MST1)-Spiegels, Messung des voraussichtlichen G-Protein gekoppelten Rezeptor 126 (GPR126)-Spiegels, Messung des interzellulären Adhäsionsmolekül 3 (ICAM3)-Spiegels, Messung des C-reaktiven Protein (CRP)-Spiegels, Messung des Phosphatidylcholin-Sterol-Acyltransferase (LCAT)-Spiegels, Messung des Roundabout-Homolog 4 (ROBO4)-Spiegels, Messung des Ectonucleotid-Pyrophosphatase/Phosphodiesterase Familienmitglieds 2 (ENPP2)-Spiegels und Messung des Protein S100-A9 (S100A9)-Spiegels umfasst.

**4.** Verfahren gemäß Anspruch 3, wobei die Messung des SEPP1-Spiegels durch die Messung des Selenium-Spiegels ergänzt oder ersetzt wird.

## Revendications

**1.** Méthode pour le pronostic de la pré-éclampsie (PE) chez un sujet comprenant le test et l'évaluation, dans un échantillon obtenu à partir dudit sujet, d'un panel de tests, dans lequel l'échantillon est du sang total, du plasma ou du sérum, le panel de tests comprenant :

- la mesure du niveau de la sous-unité labile aux acides d'un complexe protéique de liaison du facteur de croissance analogue à l'insuline (IGFALS),
- la mesure de la pression sanguine,
- la mesure du niveau d'inhibiteur de la protéase de type Kunitz 1 (SPINT1), et
- la mesure du niveau i) le domaine désintégrine et métalloprotéinase de la protéine 12 (ADAM12) ou ii) de l'Endogline-s (s-ENG ou ENG),

dans laquelle les mesures des biomarqueurs et des paramètres du panel de tests sont utilisées pour établir un profil biomarqueur-et-paramètre, lequel est comparé à une valeur de référence multiparamétrique pour le pronostic de la PE.

**2.** Méthode selon la revendication 1, dans laquelle le panel de tests comprend la mesure du niveau d'Endogline-s et la mesure du niveau d'ADAM12.

**3.** Méthode selon la revendication 1 ou 2, dans laquelle le panel de tests comprend en outre au moins une, de préférence au moins deux ou même au moins trois mesures parmi la mesure du niveau de sélénoprotéine P (SEPP1), la mesure du niveau de quiescine Q6 (QSOX1), la mesure du niveau de péroxirédoxine-2 (PRDX2), la mesure de niveau de glucose sanguin, la mesure de l'index de masse corporelle (IMC), une valeur pour le paramètre historique maternel « mère ou soeur du sujet a/ avait une pré-éclampsie » (fh pet »), une valeur pour le paramètre « niveau de lipoprotéines de haute densité » (« bb hdr »), une valeur pour le paramètre « rapport du cholestérol total sur lipoprotéines de haute densité » (« bb total hdt ratio »), une valeur pour le paramètre syndrome métabolique, une valeur pour le paramètre niveau de triglycérides (« bb trip »), la mesure du niveau du récepteur de type 3 du facteur de croissance endothéliale vasculaire (FLT4), la mesure du niveau de carboxypeptidase lysosomale Pro-X (PRCP), la mesure du niveau de péroxirédoxine-1 (PRDX1), la mesure du niveau de leucyl-cystinyl aminopeptidase (LNPEP), la mesure du niveau de ténascine-X (TNXB), la mesure du niveau de protéine nucléocapsidique de l'héparane sulfate protéoglycane spécifique à la membrane basale (HSPG2), la mesure du niveau de glycoprotéine de surface cellulaire (CD146, MUC18, MCAM), la mesure du niveau de phospholipase D spécifique du phosphatidylinositol-glycane (GPLD1), la mesure du niveau de chaîne α3 du collagène de type VI (COL3A6), la mesure du niveau de protéine de type facteur de croissance des hépatocytes (MST1), la mesure du niveau probable de récepteur 126 couplé à une protéine G (GPR126), la mesure du niveau de molécule d'adhésion intercellulaire 3 (ICAM3), la mesure du niveau de protéine C réactive (CRP), la mesure du niveau de phosphatidilcholine-stérol acyltransférase (LCAT), la mesure du niveau d'homologue roudabout 4 (ROBO4), la mesure du niveau du membre de la famille ectonucléotide pyrophosphatase / phosphodiestérase 2 (ENPP2), et la mesure du niveau de la protéine S100-A9 (S100A9).

4. Méthode selon la revendication 3, dans laquelle la mesure du niveau de SEPP1 est complétée ou substituée par la mesure du niveau de sélénium.

## FIG 1

prevalence = 5.3% - all
prevalence = 4.3% non-obese
prevalence =10.3% - obese

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2009094665 A
- WO 2009097584 A1
- WO 2009108073 A1
- US 2006105415 A
- US 4816567 A
- US 5270163 A
- US 5578577 A
- US 5141850 A
- US 4775636 A
- US 4853335 A
- US 4859612 A
- US 5079172 A
- US 5202267 A
- US 5514602 A

- US 5616467 A
- US 5681775 A
- US 6107045 A
- US 6974706 B
- US 5108889 A
- US 6027944 A
- US 6482156 B
- US 6511814 B
- US 5824268 A
- US 5726010 A
- US 6001658 A
- US 20080090305 A
- US 20030109067 A

**Non-patent literature cited in the description**

- **SOLOMON ; SEELY.** *Endocrinol Metab Clin North Am,* 2006, vol. 35 (1), 157-71
- **SIBAI ; BARTON.** *Am J Obstet Gynecol,* 2007, vol. 196 (6), 514
- **ROBERTS et al.** *Hypertension,* 2003, vol. 41 (3), 37-45
- **CONDE-AGUDELO et al.** *Obstet Gynecol,* 2004, vol. 104, 1367-91
- **KAAJAR R.** *Minerva Ginecologica,* 2008, vol. 60 (5), 475-480
- **LEWITT et al.** *Journal of Endocrinology,* 1998, vol. 159, 265
- **MISTRY et al.** *Hypertension,* 2008, vol. 52, 881
- **RAYMAN et al.** *Am J Obstet Gynecol,* 2003, vol. 189, 1343
- *BMJ,* 2011, vol. 342, d1875
- **ALBERTI et al.** *Diabetic Medicine,* 2006, vol. 23, 469-480
- **BUJOLD et al.** *Obstet Gynecol,* 2010, vol. 116, 402-14
- clinical guideline 62. Antenatal Care - Routine Care for the Healthy Pregnant woman. NHS National Institute for Health and Clinical Excellence (NICE), March 2008
- **MAYNARD et al.** *J Clin Invest,* 2003, vol. 111 (5), 649-58
- **POLLIOTTI et al.** *Obstet Gynecol,* 2003, vol. 101, 1266-74

- **CRUZ et al.** Applications of Machine Learning in Cancer Prediction and Prognosis. *Cancer Informatics,* 2007, vol. 2, 59-77
- **LOWRY et al.** *J Biol Chem,* 1951, vol. 193, 265
- **HARTREE.** *Anal Biochem,* 1972, vol. 48, 422-427
- **KOHLER et al.** *Nature,* 1975, vol. 256, 495
- **CLACKSON et al.** *Nature,* 1991, vol. 352, 624-628
- **MARKS et al.** *J Mol Biol,* 1991, vol. 222, 581-597
- **HARLOW ; LANE.** Antibodies: A Laboratory Manual. Cold Spring Harbour Laboratory, 1988
- **HARLOW ; LANE.** Using Antibodies: A Laboratory Manual. Cold Spring Harbour Laboratory, 1999
- Monoclonal Antibodies: A Manual of Techniques. CRC Press, 1987
- Monoclonal Antibodies: A Practical Approach. Oxford University Press, 2000
- Antibody Engineering: Methods and Protocols. Methods in Molecular Biology. Humana Press, 2004, vol. 248
- **ELLINGTON ; SZOSTAK.** *Nature,* 1990, vol. 346, 818-822
- **TUERK ; GOLD.** *Science,* 1990, vol. 249, 505-510
- The Aptamer Handbook: Functional Oligonucleotides and Their Applications. Wiley-VCH, 2006

- **HORWELL.** *Trends Biotechnol,* 1995, vol. 13, 132-134
- **G. FRENS.** *Nature Physical Science,* 1973, vol. 241, 20
- **JOHN R. CROWTHER.** The ELISA Guidebook. Humana Press, 2000
- **NIELSEN ; GEIERSTANGER.** *J Immunol Methods,* 2004, vol. 290, 107-20
- **LING et al.** *Expert Rev Mol Diagn,* 2007, vol. 7, 87-98
- An Introduction to Radioimmunoassay and Related Techniques. Elsevier Science, 1995
- Mass Spectrometry of Proteins and Peptides. Methods in Molecular Biology. Humana Press, 2000, vol. 146

- **BIEMANN.** *Methods Enzymol,* 1990, vol. 193, 455-79
- Biological Mass Spectrometry. Methods in Enzymology. Academic Press, 2005, vol. 402
- **KUHN et al.** *Proteomics,* 2004, vol. 4, 1175-86
- **BIDLINGMEYER, B. A.** Practical HPLC Methodology and Applications. John Wiley & Sons Inc, 1993
- **ZHONG et al.** *Prenatal Diagnosis,* 2010, vol. 30, 293-308
- **ROYSTON et al.** Prognosis and prognostic research: Developing a prognostic model. *BMJ 2009,* 2009, vol. 338, b604
- **TIBSHIRANI.** Regression shrinkage and selection via the lasso. *J. Royal. Statist. Soc,* 1996, vol. B. 58 (1), 267-288